# EUROPÄISCHE PATENTSCHRIFT

(11) **EP 2 428 565 B1**
(45) Veröffentlichungstag und Bekanntmachung des Hinweises auf die Patenterteilung: **25.09.2013**
(21) Anmeldenummer: 11192294.4
(22) Anmeldetag: 02.11.2007
(51) Int. Cl.: C12N 9/16

(54) **Polypeptid mit Phytaseaktivität und erhöhter Temperaturstabilität der Enzymaktivität sowie dieses codierende Nukleotidsequenz**
Polypeptide having phytase activity and increased temperature resistance of the enzyme activity, and nucleotide sequence coding said polypeptide
Polypeptide à activité phytase et à stabilité thermique accrue de l'activité enzymatique et séquence nucléotidique codant ce polypeptide

(30) Priorität: 10.11.2006 DE 102006053059
(43) Veröffentlichungstag der Anmeldung: 14.03.2012
(62) Teilanmeldung aus: 07819547.6
(73) Patentinhaber: AB Enzymes GmbH, 64293 Darmstadt (DE)
(72) Erfinder: Nguyen, Khanh Q., 64385 Reichelsheim (DE); Winter, Bruno, 70190 Stuttgart (DE)
(74) Vertreter: Hiebl, Inge Elisabeth

(56) Entgegenhaltungen:
- WO-A-01/36607
- WO-A-01/90333
- WO-A-03/066847
- WO-A-03/102174
- WO-A-2006/037327
- GARRETT J B ET AL: "Enhancing the thermal tolerance and gastric performance of a microbial phytase for use as a phosphate-mobilizing monogastric-feed supplement", APPLIED AND ENVIRONMENTAL MICROBIOLOGY 200405 US, Bd. 70, Nr. 5, Mai 2004 (2004-05), Seiten 3041-3046, XP002477018, ISSN: 0099-2240
- RODRIGUEZ E ET AL: "Site-directed mutagenesis improves catalytic efficiency and thermostability of Escherichia coli pH 2.5 acid phosphatase/phytase expressed in Pichia pastoris", ARCHIVES OF BIOCHEMISTRY AND BIOPHYSICS, NEW YORK, US, US, Bd. 382, Nr. 1, 1. Oktober 2000 (2000-10-01), Seiten 105-112, XP002980498, ISSN: 0003-9861

### SEQUENCE LISTING

<110> AB Enzymes GmbH
<120> Polypeptid mit Phytaseaktivität und erhöhter Temperaturstabilität der
   Enzymaktivität sowie dieses codierende Nukleotidsequenz
<130> 16936EP1
<140>
<140> 2007-11-02
<150> DE 10 2006 053 059.4
   <151> 2006-11-10
<160> 22
<170> PatentIn version 3.1
<210> 1
   <211> 33
   <212> DNA
   <213> Artificial Sequence
<220>
   <223> Primer
<400> 1
   ccaactggat aacgcccggg tgaccgacgc cat 33
<210> 2
   <211> 33
   <212> DNA
   <213> Artificial Sequence
<220>
   <223> Primer
<400> 2
   atggcgtcgg tcacccgggc gttatccagt tgg 33
<210> 3
   <211> 27
   <212> DNA
   <213> Artificial Sequence
<220>
   <223> Primer
<400> 3
   gccaacgtga ccgaggccat cctcagc 27
<210> 4
   <211> 27
   <212> DNA
   <213> Artificial Sequence
<220>
   <223> Primer
<400> 4
   gctgaggatg gcctcggtca cgttggc 27
<210> 5
   <211> 29
   <212> DNA
   <213> Artificial Sequence
<220>
   <223> Primer
<400> 5
   cggactcctg gctgacaagg gatgcccgc 29
<210> 6
   <211> 29
   <212> DNA
   <213> Artificial Sequence
<220>
   <223> Primer
<400> 6
   gcgggcatcc cttgtcagcc aggagtccg 29
<210> 7
   <211> 40
   <212> DNA
   <213> Artificial Sequence
<220>
   <223> Primer
<400> 7
   aggcgcgccc tagtcatcat tggataggca gattactcag 40
<210> 8
   <211> 39
   <212> DNA
   <213> Artificial Sequence
<220>
   <223> Primer
<400> 8
   ggaattctcg cgaaaggcaa caaccagctc acccctgag 39
<210> 9
   <211> 1230
   <212> DNA
   <213> Artificial Sequence
<220>
   <223> DNA PhyM2
<220>
   <221> CDS
   <222> (1)..(1230)
   <223>
<400> 9
   cag agc gag ccc gag ctg aag ctg gag tcg gtc gtg atc gtc agc cgc      48
   Gln Ser Glu Pro Glu Leu Lys Leu Glu Ser Val Val Ile Val Ser Arg
   1               5                   10                  15
   cac ggc gtg cgt gct cct acc aag gcc acg cag ctg atg cag gac gtc      96
   His Gly Val Arg Ala Pro Thr Lys Ala Thr Gln Leu Met Gln Asp Val
               20                  25                  30
   acc cct gac gcc tgg ccc acc tgg ccc gtc aag ctt ggc tgg ctg act     144
   Thr Pro Asp Ala Trp Pro Thr Trp Pro Val Lys Leu Gly Trp Leu Thr
           35                  40                  45
   cct cgc ggc ggt gag ctc atc gcc tac ctc gga cac tac caa cgc cag     192
   Pro Arg Gly Gly Glu Leu Ile Ala Tyr Leu Gly His Tyr Gln Arg Gln
       50                  55                  60
   cgt ctg gtt gcc gac gga ctc ctg gct aag aag gga tgc ccg cag tct     240
   Arg Leu Val Ala Asp Gly Leu Leu Ala Lys Lys Gly Cys Pro Gln Ser
   65                  70                  75                  80
   ggc cag gtc gcg att atc gcc gat gtc gac gag cgt acc cgt aag acc     288
   Gly Gln Val Ala Ile Ile Ala Asp Val Asp Glu Arg Thr Arg Lys Thr
                   85                  90                  95
   ggc gaa gcc ttc gct gcc ggc ctc gct cct gac tgt gcc atc acg gtc     336
   Gly Glu Ala Phe Ala Ala Gly Leu Ala Pro Asp Cys Ala Ile Thr Val
               100                 105                 110
   cac acc cag gca gac acg tcc agc ccc gat ccg ctg ttt aac cct ctc     384
   His Thr Gln Ala Asp Thr Ser Ser Pro Asp Pro Leu Phe Asn Pro Leu
           115                 120                 125
   aag act ggc gtc tgc caa ctg gat aac gcc cgg gtg acc gag gcc atc     432
   Lys Thr Gly Val Cys Gln Leu Asp Asn Ala Arg Val Thr Glu Ala Ile
       130                 135                 140
   ctc agc agg gct gga ggt tcc atc gcc gac ttc acc ggc cat cgg cag     480
   Leu Ser Arg Ala Gly Gly Ser Ile Ala Asp Phe Thr Gly His Arg Gln
   145                 150                 155                 160
   acg gcg ttc cgc gag ctg gag cgg gtc ctt aat ttt ccc cag tcg aac     528
   Thr Ala Phe Arg Glu Leu Glu Arg Val Leu Asn Phe Pro Gln Ser Asn
                   165                 170                 175
   ctg tgc ctc aag cgt gag aag cag gac gag agc tgt tcc ctg acc cag     576
   Leu Cys Leu Lys Arg Glu Lys Gln Asp Glu Ser Cys Ser Leu Thr Gln
               180                 185                 190
   gca ctc ccg tcg gaa ctc aag tac agc gcc gac aac gtc tcc ctt acc     624
   Ala Leu Pro Ser Glu Leu Lys Tyr Ser Ala Asp Asn Val Ser Leu Thr
           195                 200                 205
   ggt gcc gtt agc ctc gct tcc atg ctg acg gag atc ttc ctc ctg cag     672
   Gly Ala Val Ser Leu Ala Ser Met Leu Thr Glu Ile Phe Leu Leu Gln
       210                 215                 220
   caa gcg cag gga atg ccc gag cct ggg tgg ggc cgc att acc gat tct     720
   Gln Ala Gln Gly Met Pro Glu Pro Gly Trp Gly Arg Ile Thr Asp Ser
   225                 230                 235                 240
   cac cag tgg aac acc ctg ctc tcg ctt cac aac gcc cag ttc tat ctg     768
   His Gln Trp Asn Thr Leu Leu Ser Leu His Asn Ala Gln Phe Tyr Leu
                   245                 250                 255
   ctc caa cgc acg ccc gag gtt gcc cgc agc cgc gcc acc ccg ctg ctc     816
   Leu Gln Arg Thr Pro Glu Val Ala Arg Ser Arg Ala Thr Pro Leu Leu
               260                 265                 270
   gac ctc atc aag act gcg ctg acg ccc cac cct ccg cag aag cag gct      864
   Asp Leu Ile Lys Thr Ala Leu Thr Pro His Pro Pro Gln Lys Gln Ala
           275                 280                 285
   tac ggt gtc acc ctc ccc act tcc gtc ctg ttt atc gcc ggt cac gac      912
   Tyr Gly Val Thr Leu Pro Thr Ser Val Leu Phe Ile Ala Gly His Asp
       290                 295                 300
   acc aac ctg gcc aat ctc ggc ggc gct ctg gag ctc aac tgg acg ctt      960
   Thr Asn Leu Ala Asn Leu Gly Gly Ala Leu Glu Leu Asn Trp Thr Leu
   305                 310                 315                 320
   ccc gga cag ccg gat aac act ccc cct ggc ggt gag ctg gtg ttc gaa     1008
   Pro Gly Gln Pro Asp Asn Thr Pro Pro Gly Gly Glu Leu Val Phe Glu
                   325                 330                 335
   cgc tgg cgt cgg ctc agc gac aac tcc cag tgg att cag gtt tcg ctg     1056
   Arg Trp Arg Arg Leu Ser Asp Asn Ser Gln Trp Ile Gln Val Ser Leu
               340                 345                 350
   gtc ttc cag acc ctg cag cag atg cgc gac aaa acg ccc ctg tcc ctc     1104
   Val Phe Gln Thr Leu Gln Gln Met Arg Asp Lys Thr Pro Leu Ser Leu
           355                 360                 365
   aat acc cct ccc ggc gag gtc aag ctg acc ctg gca ggc tgt gaa gag     1152
   Asn Thr Pro Pro Gly Glu Val Lys Leu Thr Leu Ala Gly Cys Glu Glu
       370                 375                 380
   cgc aac gcc cag ggc atg tgc tct ctc gct ggc ttt acg caa atc gtg     1200
   Arg Asn Ala Gln Gly Met Cys Ser Leu Ala Gly Phe Thr Gln Ile Val
   385                 390                 395                 400
   aac gag gcc cgc atc ccc gct tgc tct ctg                             1230
   Asn Glu Ala Arg Ile Pro Ala Cys Ser Leu
                   405                 410
<210> 10
   <211> 410
   <212> PRT
   <213> Artificial Sequence
<220>
   <223> DNA PhyM2
<400> 10
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Trp Leu Thr
           35                  40                  45
   Pro Arg Gly Gly Glu Leu Ile Ala Tyr Leu Gly His Tyr Gln Arg Gln
       50                  55                  60
   Arg Leu Val Ala Asp Gly Leu Leu Ala Lys Lys Gly Cys Pro Gln Ser
   65 70 75 80
   Gly Gln Val Ala Ile Ile Ala Asp Val Asp Glu Arg Thr Arg Lys Thr
   85 90 95
   Gly Glu Ala Phe Ala Ala Gly Leu Ala Pro Asp Cys Ala Ile Thr Val
   100 105 110
   His Thr Gln Ala Asp Thr Ser Ser Pro Asp Pro Leu Phe Asn Pro Leu
   115 120 125
   Lys Thr Gly Val Cys Gln Leu Asp Asn Ala Arg Val Thr Glu Ala Ile
   130 135 140
   Leu Ser Arg Ala Gly Gly Ser Ile Ala Asp Phe Thr Gly His Arg Gln
   145 150 155 160
   Thr Ala Phe Arg Glu Leu Glu Arg Val Leu Asn Phe Pro Gln Ser Asn
   165 170 175
   Leu Cys Leu Lys Arg Glu Lys Gln Asp Glu Ser Cys Ser Leu Thr Gln
   180 185 190
   Ala Leu Pro Ser Glu Leu Lys Tyr Ser Ala Asp Asn Val Ser Leu Thr
   195 200 205
   Gly Ala Val Ser Leu Ala Ser Met Leu Thr Glu Ile Phe Leu Leu Gln
   210 215 220
   Gln Ala Gln Gly Met Pro Glu Pro Gly Trp Gly Arg Ile Thr Asp Ser
   225 230 235 240
   His Gln Trp Asn Thr Leu Leu Ser Leu His Asn Ala Gln Phe Tyr Leu
   245 250 255
   Leu Gln Arg Thr Pro Glu Val Ala Arg Ser Arg Ala Thr Pro Leu Leu
   260 265 270
   Asp Leu Ile Lys Thr Ala Leu Thr Pro His Pro Pro Gln Lys Gln Ala
   275 280 285
   Tyr Gly Val Thr Leu Pro Thr Ser Val Leu Phe Ile Ala Gly His Asp
   290 295 300
   Thr Asn Leu Ala Asn Leu Gly Gly Ala Leu Glu Leu Asn Trp Thr Leu
   305 310 315 320
   Pro Gly Gln Pro Asp Asn Thr Pro Pro Gly Gly Glu Leu Val Phe Glu
   325 330 335
   Arg Trp Arg Arg Leu Ser Asp Asn Ser Gln Trp Ile Gln Val Ser Leu
   340 345 350
   Val Phe Gln Thr Leu Gln Gln Met Arg Asp Lys Thr Pro Leu Ser Leu
   355 360 365
   Asn Thr Pro Pro Gly Glu Val Lys Leu Thr Leu Ala Gly Cys Glu Glu
   370 375 380
   Arg Asn Ala Gln Gly Met Cys Ser Leu Ala Gly Phe Thr Gln Ile Val
   385 390 395 400
   Asn Glu Ala Arg Ile Pro Ala Cys Ser Leu
   405 410
<210> 11
   <211> 1230
   <212> DNA
   <213> Artificial Sequence
<220>
   <223> DNA PhyM3
<220>
   <221> CDS
   <222> (1)..(1230)
   <223>
<400> 11
   cag agc gag ccc gag ctg aag ctg gag tcg gtc gtg atc gtc agc cgc      48
   Gln Ser Glu Pro Glu Leu Lys Leu Glu Ser Val Val Ile Val Ser Arg
   1               5                   10                  15
   cac ggc gtg cgt gct cct acc aag gcc acg cag ctg atg cag gac gtc      96
   His Gly Val Arg Ala Pro Thr Lys Ala Thr Gln Leu Met Gln Asp Val
               20                  25                  30
   acc cct gac gcc tgg ccc acc tgg ccc gtc aag ctt ggc tgg ctg act     144
   Thr Pro Asp Ala Trp Pro Thr Trp Pro Val Lys Leu Gly Trp Leu Thr
           35                  40                  45
   cct cgc ggc ggt gag ctc atc gcc tac ctc gga cac tac caa cgc cag     192
   Pro Arg Gly Gly Glu Leu Ile Ala Tyr Leu Gly His Tyr Gln Arg Gln
       50                  55                  60
   cgt ctg gtt gcc gac gga ctc ctg gct aag aag gga tgc ccg cag tct     240
   Arg Leu Val Ala Asp Gly Leu Leu Ala Lys Lys Gly Cys Pro Gln Ser
   65                  70                  75                  80
   ggc cag gtc gcg att atc gcc gat gtc gac gag cgt acc cgt aag acc     288
   Gly Gln Val Ala Ile Ile Ala Asp Val Asp Glu Arg Thr Arg Lys Thr
                   85                  90                  95
   ggc gaa gcc ttc gct gcc ggc ctc gct cct gac tgt gcc atc acg gtc     336
   Gly Glu Ala Phe Ala Ala Gly Leu Ala Pro Asp Cys Ala Ile Thr Val
               100                 105                 110
   cac acc cag gca gac acg tcc agc ccc gat ccg ctg ttt aac cct ctc      384
   His Thr Gln Ala Asp Thr Ser Ser Pro Asp Pro Leu Phe Asn Pro Leu
           115                 120                 125
   aag act ggc gtc tgc caa ctg gat aac gcc aac gtg acc gac gcc atc      432
   Lys Thr Gly Val Cys Gln Leu Asp Asn Ala Asn Val Thr Asp Ala Ile
       130                 135                 140
   ctc agc agg gct gga ggt tcc atc gcc gac ttc acc ggc cat cgg cag      480
   Leu Ser Arg Ala Gly Gly Ser Ile Ala Asp Phe Thr Gly His Arg Gln
   145                 150                 155                 160
   acg gcg ttc cgc gag ctg gag cgg gtc ctt aat ttt ccc cag tcg aac      528
   Thr Ala Phe Arg Glu Leu Glu Arg Val Leu Asn Phe Pro Gln Ser Asn
                   165                 170                 175
   ctg tgc ctc aag cgt gag aag cag gac gag agc tgt tcc ctg acc cag      576
   Leu Cys Leu Lys Arg Glu Lys Gln Asp Glu Ser Cys Ser Leu Thr Gln
               180                 185                 190
   gca ctc ccg tcg gaa ctc aag ccc tcc gcg gac aac gtc tcc ctt acc      624
   Ala Leu Pro Ser Glu Leu Lys Pro Ser Ala Asp Asn Val Ser Leu Thr
           195                 200                 205
   ggt gcc gtt agc ctc gct tcc atg ctg acg gag atc ttc ctc ctg cag      672
   Gly Ala Val Ser Leu Ala Ser Met Leu Thr Glu Ile Phe Leu Leu Gln
       210                 215                 220
   caa gcg cag gga atg ccc gag cct ggg tgg ggc cgc att acc gat tct      720
   Gln Ala Gln Gly Met Pro Glu Pro Gly Trp Gly Arg Ile Thr Asp Ser
   225                 230                 235                 240
   cac cag tgg aac acc ctg ctc tcg ctt cac aac gcc cag ttc tat ctg      768
   His Gln Trp Asn Thr Leu Leu Ser Leu His Asn Ala Gln Phe Tyr Leu
                   245                 250                 255
   ctc caa cgc acg ccc gag gtt gcc cgc agc cgc gcc acc ccg ctg ctc      816
   Leu Gln Arg Thr Pro Glu Val Ala Arg Ser Arg Ala Thr Pro Leu Leu
               260                 265                 270
   gac ctc atc aag act gcg ctg acg ccc cac cct ccg cag aag cag gct      864
   Asp Leu Ile Lys Thr Ala Leu Thr Pro His Pro Pro Gln Lys Gln Ala
           275                 280                 285
   tac ggt gtc acc ctc ccc act tcc gtc ctg ttt atc gcc ggt cac gac      912
   Tyr Gly Val Thr Leu Pro Thr Ser Val Leu Phe Ile Ala Gly His Asp
       290                 295                 300
   acc aac ctg gcc aat ctc ggc ggc gct ctg gag ctc aac tgg acg ctt      960
   Thr Asn Leu Ala Asn Leu Gly Gly Ala Leu Glu Leu Asn Trp Thr Leu
   305                 310                 315                 320
   ccc gga cag ccg gat aac act ccc cct ggc ggt gag ctg gtg ttc gaa     1008
   Pro Gly Gln Pro Asp Asn Thr Pro Pro Gly Gly Glu Leu Val Phe Glu
                   325                 330                 335
   cgc tgg cgt cgg ctc agc gac aac tcc cag tgg att cag gtt tcg ctg     1056
   Arg Trp Arg Arg Leu Ser Asp Asn Ser Gln Trp Ile Gln Val Ser Leu
               340                 345                 350
   gtc ttc cag acc ctg cag cag atg cgc gac aaa acg ccc ctg tcc ctc     1104
   Val Phe Gln Thr Leu Gln Gln Met Arg Asp Lys Thr Pro Leu Ser Leu
           355                 360                 365
   aat acc cct ccc ggc gag gtc aag ctg acc ctg gca ggc tgt gaa gag      1152
   Asn Thr Pro Pro Gly Glu Val Lys Leu Thr Leu Ala Gly Cys Glu Glu
       370                 375                 380
   cgc aac gcc cag ggc atg tgc tct ctc gct ggc ttt acg caa atc gtg      1200
   Arg Asn Ala Gln Gly Met Cys Ser Leu Ala Gly Phe Thr Gln Ile Val
   385                 390                 395                 400
   aac gag gcc cgc atc ccc gct tgc tct ctg                              1230
   Asn Glu Ala Arg Ile Pro Ala Cys Ser Leu
                   405                 410
<210> 12
   <211> 410
   <212> PRT
   <213> Artificial Sequence
<220>
   <223> DNA PhyM3
<400> 12
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Trp Leu Thr
           35                  40                  45
   Pro Arg Gly Gly Glu Leu Ile Ala Tyr Leu Gly His Tyr Gln Arg Gln
       50                  55                  60
   Arg Leu Val Ala Asp Gly Leu Leu Ala Lys Lys Gly Cys Pro Gln Ser
   65                  70                  75                  80
   Gly Gln Val Ala Ile Ile Ala Asp Val Asp Glu Arg Thr Arg Lys Thr
                   85                  90                  95
   Gly Glu Ala Phe Ala Ala Gly Leu Ala Pro Asp Cys Ala Ile Thr Val
               100                 105                 110
   His Thr Gln Ala Asp Thr Ser Ser Pro Asp Pro Leu Phe Asn Pro Leu
           115                 120                 125
   Lys Thr Gly Val Cys Gln Leu Asp Asn Ala Asn Val Thr Asp Ala Ile
       130                 135                 140
   Leu Ser Arg Ala Gly Gly Ser Ile Ala Asp Phe Thr Gly His Arg Gln
   145                 150                 155                 160
   Thr Ala Phe Arg Glu Leu Glu Arg Val Leu Asn Phe Pro Gln Ser Asn
   165 170 175
   Leu Cys Leu Lys Arg Glu Lys Gln Asp Glu Ser Cys Ser Leu Thr Gln
   180 185 190
   Ala Leu Pro Ser Glu Leu Lys Pro Ser Ala Asp Asn Val Ser Leu Thr
   195 200 205
   Gly Ala Val Ser Leu Ala Ser Met Leu Thr Glu Ile Phe Leu Leu Gln
   210 215 220
   Gln Ala Gln Gly Met Pro Glu Pro Gly Trp Gly Arg Ile Thr Asp Ser
   225 230 235 240
   His Gln Trp Asn Thr Leu Leu Ser Leu His Asn Ala Gln Phe Tyr Leu
   245 250 255
   Leu Gln Arg Thr Pro Glu Val Ala Arg Ser Arg Ala Thr Pro Leu Leu
   260 265 270
   Asp Leu Ile Lys Thr Ala Leu Thr Pro His Pro Pro Gln Lys Gln Ala
   275 280 285
   Tyr Gly Val Thr Leu Pro Thr Ser Val Leu Phe Ile Ala Gly His Asp
   290 295 300
   Thr Asn Leu Ala Asn Leu Gly Gly Ala Leu Glu Leu Asn Trp Thr Leu
   305 310 315 320
   Pro Gly Gln Pro Asp Asn Thr Pro Pro Gly Gly Glu Leu Val Phe Glu
   325 330 335
   Arg Trp Arg Arg Leu Ser Asp Asn Ser Gln Trp Ile Gln Val Ser Leu
   340 345 350
   Val Phe Gln Thr Leu Gln Gln Met Arg Asp Lys Thr Pro Leu Ser Leu
   355 360 365
   Asn Thr Pro Pro Gly Glu Val Lys Leu Thr Leu Ala Gly Cys Glu Glu
   370 375 380
   Arg Asn Ala Gln Gly Met Cys Ser Leu Ala Gly Phe Thr Gln Ile Val
   385 390 395 400
   Asn Glu Ala Arg Ile Pro Ala Cys Ser Leu
   405 410
<210> 13
   <211> 1230
   <212> DNA
   <213> Artificial Sequence
<220>
   <223> DNA PhyM7
<220>
   <221> CDS
   <222> (1)..(1230)
   <223>
<400> 13
   cag agc gag ccc gag ctg aag ctg gag tcg gtc gtg atc gtc agc cgc      48
   Gln Ser Glu Pro Glu Leu Lys Leu Glu Ser Val Val Ile Val Ser Arg
   1               5                   10                  15
   cac ggc gtg cgt gct cct acc aag gcc acg cag ctg atg cag gac gtc      96
   His Gly Val Arg Ala Pro Thr Lys Ala Thr Gln Leu Met Gln Asp Val
               20                  25                  30
   acc cct gac gcc tgg ccc acc tgg ccc gtc aag ctt ggc tgg ctg act     144
   Thr Pro Asp Ala Trp Pro Thr Trp Pro Val Lys Leu Gly Trp Leu Thr
           35                  40                  45
   cct cgc ggc ggt gag ctc atc gcc tac ctc gga cac tac caa cgc cag     192
   Pro Arg Gly Gly Glu Leu Ile Ala Tyr Leu Gly His Tyr Gln Arg Gln
       50                  55                  60
   cgt ctg gtt gcc gac gga ctc ctg gct gac aag gga tgc ccg cag tct     240
   Arg Leu Val Ala Asp Gly Leu Leu Ala Asp Lys Gly Cys Pro Gln Ser
   65                  70                  75                  80
   ggc cag gtc gcg att atc gcc gat gtc gac gag cgt acc cgt aag acc     288
   Gly Gln Val Ala Ile Ile Ala Asp Val Asp Glu Arg Thr Arg Lys Thr
                   85                  90                  95
   ggc gaa gcc ttc gct gcc ggc ctc gct cct gac tgt gcc atc acg gtc     336
   Gly Glu Ala Phe Ala Ala Gly Leu Ala Pro Asp Cys Ala Ile Thr Val
               100                 105                 110
   cac acc cag gca gac acg tcc agc ccc gat ccg ctg ttt aac cct ctc     384
   His Thr Gln Ala Asp Thr Ser Ser Pro Asp Pro Leu Phe Asn Pro Leu
           115                 120                 125
   aag act ggc gtc tgc caa ctg gat aac gcc aac gtg acc gac gcc atc     432
   Lys Thr Gly Val Cys Gln Leu Asp Asn Ala Asn Val Thr Asp Ala Ile
       130                 135                 140
   ctc agc agg gct gga ggt tcc atc gcc gac ttc acc ggc cat cgg cag     480
   Leu Ser Arg Ala Gly Gly Ser Ile Ala Asp Phe Thr Gly His Arg Gln
   145                 150                 155                 160
   acg gcg ttc cgc gag ctg gag cgg gtc ctt aat ttt ccc cag tcg aac     528
   Thr Ala Phe Arg Glu Leu Glu Arg Val Leu Asn Phe Pro Gln Ser Asn
                   165                 170                 175
   ctg tgc ctc aag cgt gag aag cag gac gag agc tgt tcc ctg acc cag     576
   Leu Cys Leu Lys Arg Glu Lys Gln Asp Glu Ser Cys Ser Leu Thr Gln
               180                 185                 190
   gca ctc ccg tcg gaa ctc aag tac agc gcc gac aac gtc tcc ctt acc     624
   Ala Leu Pro Ser Glu Leu Lys Tyr Ser Ala Asp Asn Val Ser Leu Thr
           195                 200                 205
   ggt gcc gtt agc ctc gct tcc atg ctg acg gag atc ttc ctc ctg cag        672
   Gly Ala Val Ser Leu Ala Ser Met Leu Thr Glu Ile Phe Leu Leu Gln
       210                 215                 220
   caa gcg cag gga atg ccc gag cct ggg tgg ggc cgc att acc gat tct        720
   Gln Ala Gln Gly Met Pro Glu Pro Gly Trp Gly Arg Ile Thr Asp Ser
   225                 230                 235                 240
   cac cag tgg aac acc ctg ctc tcg ctt cac aac gcc cag ttc tat ctg        768
   His Gln Trp Asn Thr Leu Leu Ser Leu His Asn Ala Gln Phe Tyr Leu
                   245                 250                 255
   ctc caa cgc acg ccc gag gtt gcc cgc agc cgc gcc acc ccg ctg ctc        816
   Leu Gln Arg Thr Pro Glu Val Ala Arg Ser Arg Ala Thr Pro Leu Leu
               260                 265                 270
   gac ctc atc aag act gcg ctg acg ccc cac cct ccg cag aag cag gct        864
   Asp Leu Ile Lys Thr Ala Leu Thr Pro His Pro Pro Gln Lys Gln Ala
           275                 280                 285
   tac ggt gtc acc ctc ccc act tcc gtc ctg ttt atc gcc ggt cac gac        912
   Tyr Gly Val Thr Leu Pro Thr Ser Val Leu Phe Ile Ala Gly His Asp
       290                 295                 300
   acc aac ctg gcc aat ctc ggc ggc gct ctg gag ctc aac tgg acg ctt        960
   Thr Asn Leu Ala Asn Leu Gly Gly Ala Leu Glu Leu Asn Trp Thr Leu
   305                 310                 315                 320
   ccc gga cag ccg gat aac act ccc cct ggc ggt gag ctg gtg ttc gaa       1008
   Pro Gly Gln Pro Asp Asn Thr Pro Pro Gly Gly Glu Leu Val Phe Glu
                   325                 330                 335
   cgc tgg cgt cgg ctc agc gac aac tcc cag tgg att cag gtt tcg ctg       1056
   Arg Trp Arg Arg Leu Ser Asp Asn Ser Gln Trp Ile Gln Val Ser Leu
               340                 345                 350
   gtc ttc cag acc ctg cag cag atg cgc gac aaa acg ccc ctg tcc ctc       1104
   Val Phe Gln Thr Leu Gln Gln Met Arg Asp Lys Thr Pro Leu Ser Leu
           355                 360                 365
   aat acc cct ccc ggc gag gtc aag ctg acc ctg gca ggc tgt gaa gag       1152
   Asn Thr Pro Pro Gly Glu Val Lys Leu Thr Leu Ala Gly Cys Glu Glu
       370                 375                 380
   cgc aac gcc cag ggc atg tgc tct ctc gct ggc ttt acg caa atc gtg       1200
   Arg Asn Ala Gln Gly Met Cys Ser Leu Ala Gly Phe Thr Gln Ile Val
   385                 390                 395                 400
   aac gag gcc cgc atc ccc gct tgc tct ctg                               1230
   Asn Glu Ala Arg Ile Pro Ala Cys Ser Leu
                   405                 410
<210> 14
   <211> 410
   <212> PRT
   <213> Artificial Sequence
<220>
   <223> DNA PhyM7
<400> 14
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
   20 25 30
   Thr Pro Asp Ala Trp Pro Thr Trp Pro Val Lys Leu Gly Trp Leu Thr
   35 40 45
   Pro Arg Gly Gly Glu Leu Ile Ala Tyr Leu Gly His Tyr Gln Arg Gln
   50 55 60
   Arg Leu Val Ala Asp Gly Leu Leu Ala Asp Lys Gly Cys Pro Gln Ser
   65 70 75 80
   Gly Gln Val Ala Ile Ile Ala Asp Val Asp Glu Arg Thr Arg Lys Thr
   85 90 95
   Gly Glu Ala Phe Ala Ala Gly Leu Ala Pro Asp Cys Ala Ile Thr Val
   100 105 110
   His Thr Gln Ala Asp Thr Ser Ser Pro Asp Pro Leu Phe Asn Pro Leu
   115 120 125
   Lys Thr Gly Val Cys Gln Leu Asp Asn Ala Asn Val Thr Asp Ala Ile
   130 135 140
   Leu Ser Arg Ala Gly Gly Ser Ile Ala Asp Phe Thr Gly His Arg Gln
   145 150 155 160
   Thr Ala Phe Arg Glu Leu Glu Arg Val Leu Asn Phe Pro Gln Ser Asn
   165 170 175
   Leu Cys Leu Lys Arg Glu Lys Gln Asp Glu Ser Cys Ser Leu Thr Gln
   180 185 190
   Ala Leu Pro Ser Glu Leu Lys Tyr Ser Ala Asp Asn Val Ser Leu Thr
   195 200 205
   Gly Ala Val Ser Leu Ala Ser Met Leu Thr Glu Ile Phe Leu Leu Gln
   210 215 220
   Gln Ala Gln Gly Met Pro Glu Pro Gly Trp Gly Arg Ile Thr Asp Ser
   225 230 235 240
   His Gln Trp Asn Thr Leu Leu Ser Leu His Asn Ala Gln Phe Tyr Leu
   245 250 255
   Leu Gln Arg Thr Pro Glu Val Ala Arg Ser Arg Ala Thr Pro Leu Leu
   260 265 270
   Asp Leu Ile Lys Thr Ala Leu Thr Pro His Pro Pro Gln Lys Gln Ala
   275 280 285
   Tyr Gly Val Thr Leu Pro Thr Ser Val Leu Phe Ile Ala Gly His Asp
   290 295 300
   Thr Asn Leu Ala Asn Leu Gly Gly Ala Leu Glu Leu Asn Trp Thr Leu
   305 310 315 320
   Pro Gly Gln Pro Asp Asn Thr Pro Pro Gly Gly Glu Leu Val Phe Glu
   325 330 335
   Arg Trp Arg Arg Leu Ser Asp Asn Ser Gln Trp Ile Gln Val Ser Leu
   340 345 350
   Val Phe Gln Thr Leu Gln Gln Met Arg Asp Lys Thr Pro Leu Ser Leu
   355 360 365
   Asn Thr Pro Pro Gly Glu Val Lys Leu Thr Leu Ala Gly Cys Glu Glu
   370 375 380
   Arg Asn Ala Gln Gly Met Cys Ser Leu Ala Gly Phe Thr Gln Ile Val
   385 390 395 400
   Asn Glu Ala Arg Ile Pro Ala Cys Ser Leu
   405 410
<210> 15
   <211> 1230
   <212> DNA
   <213> Artificial Sequence
<220>
   <223> DNA PhyM9
<220>
   <221> CDS
   <222> (1)..(1230)
   <223>
<400> 15
   cag agc gag ccc gag ctg aag ctg gag tcg gtc gtg atc gtc agc cgc        48
   Gln Ser Glu Pro Glu Leu Lys Leu Glu Ser Val Val Ile Val Ser Arg
   1               5                   10                  15
   cac ggc gtg cgt gct cct acc aag gcc acg cag ctg atg cag gac gtc        96
   His Gly Val Arg Ala Pro Thr Lys Ala Thr Gln Leu Met Gln Asp Val
               20                  25                  30
   acc cct gac gcc tgg ccc acc tgg ccc gtc aag ctt ggc tgg ctg act       144
   Thr Pro Asp Ala Trp Pro Thr Trp Pro Val Lys Leu Gly Trp Leu Thr
           35                  40                  45
   cct cgc ggc ggt gag ctc atc gcc tac ctc gga cac tac caa cgc cag       192
   Pro Arg Gly Gly Glu Leu Ile Ala Tyr Leu Gly His Tyr Gln Arg Gln
       50                  55                  60
   cgt ctg gtt gcc gac gga ctc ctg gct aag aag gga tgc ccg cag tct      240
   Arg Leu Val Ala Asp Gly Leu Leu Ala Lys Lys Gly Cys Pro Gln Ser
   65                  70                  75                  80
   ggc cag gtc gcg att atc gcc gat gtc gac gag cgt acc cgt aag acc      288
   Gly Gln Val Ala Ile Ile Ala Asp Val Asp Glu Arg Thr Arg Lys Thr
                   85                  90                  95
   ggc gaa gcc ttc gct gcc ggc ctc gct cct gac tgt gcc atc acg gtc      336
   Gly Glu Ala Phe Ala Ala Gly Leu Ala Pro Asp Cys Ala Ile Thr Val
               100                 105                 110
   cac acc cag gca gac acg tcc agc ccc gat ccg ctg ttt aac cct ctc      384
   His Thr Gln Ala Asp Thr Ser Ser Pro Asp Pro Leu Phe Asn Pro Leu
           115                 120                 125
   aag act ggc gtc tgc caa ctg gat aac gcc aac gtg acc gac gcc atc      432
   Lys Thr Gly Val Cys Gln Leu Asp Asn Ala Asn Val Thr Asp Ala Ile
       130                 135                 140
   atc tct aga gct gga ggt tcc atc gcc gac ttc acc ggc cat cgg cag      480
   Ile Ser Arg Ala Gly Gly Ser Ile Ala Asp Phe Thr Gly His Arg Gln
   145                 150                 155                 160
   acg gcg ttc cgc gag ctg gag cgg gtc ctt aat ttt ccc cag tcg aac      528
   Thr Ala Phe Arg Glu Leu Glu Arg Val Leu Asn Phe Pro Gln Ser Asn
                   165                 170                 175
   ctg tgc ctc aag cgt gag aag cag gac gag agc tgt tcc ctg acc cag      576
   Leu Cys Leu Lys Arg Glu Lys Gln Asp Glu Ser Cys Ser Leu Thr Gln
               180                 185                 190
   gca ctc ccg tcg gaa atc aag gtg tcc gcg gac aac gtc tcc ctt acc      624
   Ala Leu Pro Ser Glu Ile Lys Val Ser Ala Asp Asn Val Ser Leu Thr
           195                 200                 205
   ggt gcc gtt agc ctc gct tcc atg ctg acg gag atc ttc ctc ctg cag      672
   Gly Ala Val Ser Leu Ala Ser Met Leu Thr Glu Ile Phe Leu Leu Gln
       210                 215                 220
   caa gcg cag gga atg ccc gag cct ggg tgg ggc cgc att acc gat tct      720
   Gln Ala Gln Gly Met Pro Glu Pro Gly Trp Gly Arg Ile Thr Asp Ser
   225                 230                 235                 240
   cac cag tgg aac acc ctg ctc tcg ctt cac aac gcc cag ttc tat ctg      768
   His Gln Trp Asn Thr Leu Leu Ser Leu His Asn Ala Gln Phe Tyr Leu
                   245                 250                 255
   ctc caa cgc acg ccc gag gtt gcc cgc agc cgc gcc acc ccg ctg ctc      816
   Leu Gln Arg Thr Pro Glu Val Ala Arg Ser Arg Ala Thr Pro Leu Leu
               260                 265                 270
   gac ctc atc aag act gcg ctg acg ccc cac cct ccg cag aag cag gct      864
   Asp Leu Ile Lys Thr Ala Leu Thr Pro His Pro Pro Gln Lys Gln Ala
           275                 280                 285
   tac ggt gtc acc ctc ccc act tcc gtc ttg ttt atc gcc ggt cac gac      912
   Tyr Gly Val Thr Leu Pro Thr Ser Val Leu Phe Ile Ala Gly His Asp
       290                 295                 300
   acc aac ctg gcc aat ctc ggc ggc gct ctg gag ctc aac tgg acg ctt      960
   Thr Asn Leu Ala Asn Leu Gly Gly Ala Leu Glu Leu Asn Trp Thr Leu
   305                 310                 315                 320
   ccc gga cag ccg gat aac act ccc cct ggc ggt gag ctg gtg ttc gaa      1008
   Pro Gly Gln Pro Asp Asn Thr Pro Pro Gly Gly Glu Leu Val Phe Glu
                   325                 330                 335
   cgc tgg cgt cgg ctc agc gac aac tcc cag tgg att cag gtt tcg ctg      1056
   Arg Trp Arg Arg Leu Ser Asp Asn Ser Gln Trp Ile Gln Val Ser Leu
               340                 345                 350
   gtc ttc cag acc ctg cag cag atg cgc gac aaa acg ccc ctg tcc ctc      1104
   Val Phe Gln Thr Leu Gln Gln Met Arg Asp Lys Thr Pro Leu Ser Leu
           355                 360                 365
   aat acc cct ccc ggc gag gtc aag ctg acc ctg gca ggc tgt gaa gag      1152
   Asn Thr Pro Pro Gly Glu Val Lys Leu Thr Leu Ala Gly Cys Glu Glu
       370                 375                 380
   cgc aac gcc cag ggc atg tgc tct ctc gct ggc ttt acg caa atc gtg      1200
   Arg Asn Ala Gln Gly Met Cys Ser Leu Ala Gly Phe Thr Gln Ile Val
   385                 390                 395                 400
   aac gag gcc cgc atc ccc gct tgc tct ctg                              1230
   Asn Glu Ala Arg Ile Pro Ala Cys Ser Leu
                   405                 410
<210> 16
   <211> 410
   <212> PRT
   <213> Artificial Sequence
<220>
   <223> DNA PhyM9
<400> 16
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Trp Leu Thr
           35                  40                  45
   Pro Arg Gly Gly Glu Leu Ile Ala Tyr Leu Gly His Tyr Gln Arg Gln
       50                  55                  60
   Arg Leu Val Ala Asp Gly Leu Leu Ala Lys Lys Gly Cys Pro Gln Ser
   65                  70                  75                  80
   Gly Gln Val Ala Ile Ile Ala Asp Val Asp Glu Arg Thr Arg Lys Thr
                   85                  90                  95
   Gly Glu Ala Phe Ala Ala Gly Leu Ala Pro Asp Cys Ala Ile Thr Val
               100                 105                 110
   His Thr Gln Ala Asp Thr Ser Ser Pro Asp Pro Leu Phe Asn Pro Leu
           115                 120                 125
   Lys Thr Gly Val Cys Gln Leu Asp Asn Ala Asn Val Thr Asp Ala Ile
       130                 135                 140
   Ile Ser Arg Ala Gly Gly Ser Ile Ala Asp Phe Thr Gly His Arg Gln
   145                 150                 155                 160
   Thr Ala Phe Arg Glu Leu Glu Arg Val Leu Asn Phe Pro Gln Ser Asn
                   165                 170                 175
   Leu Cys Leu Lys Arg Glu Lys Gln Asp Glu Ser Cys Ser Leu Thr Gln
               180                 185                 190
   Ala Leu Pro Ser Glu Ile Lys Val Ser Ala Asp Asn Val Ser Leu Thr
           195                 200                 205
   Gly Ala Val Ser Leu Ala Ser Met Leu Thr Glu Ile Phe Leu Leu Gln
       210                 215                 220
   Gln Ala Gln Gly Met Pro Glu Pro Gly Trp Gly Arg Ile Thr Asp Ser
   225                 230                 235                 240
   His Gln Trp Asn Thr Leu Leu Ser Leu His Asn Ala Gln Phe Tyr Leu
                   245                 250                 255
   Leu Gln Arg Thr Pro Glu Val Ala Arg Ser Arg Ala Thr Pro Leu Leu
               260                 265                 270
   Asp Leu Ile Lys Thr Ala Leu Thr Pro His Pro Pro Gln Lys Gln Ala
           275                 280                 285
   Tyr Gly Val Thr Leu Pro Thr Ser Val Leu Phe Ile Ala Gly His Asp
       290                 295                 300
   Thr Asn Leu Ala Asn Leu Gly Gly Ala Leu Glu Leu Asn Trp Thr Leu
   305                 310                 315                 320
   Pro Gly Gln Pro Asp Asn Thr Pro Pro Gly Gly Glu Leu Val Phe Glu
                   325                 330                 335
   Arg Trp Arg Arg Leu Ser Asp Asn Ser Gln Trp Ile Gln Val Ser Leu
               340                 345                 350
   Val Phe Gln Thr Leu Gln Gln Met Arg Asp Lys Thr Pro Leu Ser Leu
           355                 360                 365
   Asn Thr Pro Pro Gly Glu Val Lys Leu Thr Leu Ala Gly Cys Glu Glu
   370 375 380
   Arg Asn Ala Gln Gly Met Cys Ser Leu Ala Gly Phe Thr Gln Ile Val
   385 390 395 400
   Asn Glu Ala Arg Ile Pro Ala Cys Ser Leu
   405 410
<210> 17
   <211> 1230
   <212> DNA
   <213> Artificial Sequence
<220>
   <223> DNA PhyM10
<220>
   <221> CDS
   <222> (1)..(1230)
   <223>
<400> 17
   cag agc gag ccc gag ctg aag ctg gag tcg gtc gtg atc gtc agc cgc       48
   Gln Ser Glu Pro Glu Leu Lys Leu Glu Ser Val Val Ile Val Ser Arg
   1               5                   10                  15
   cac ggc gtg cgt gct cct acc aag gcc acg cag ctg atg cag gac gtc       96
   His Gly Val Arg Ala Pro Thr Lys Ala Thr Gln Leu Met Gln Asp Val
               20                  25                  30
   acc cct gac gcc tgg ccc acc tgg ccc gtc aag ctt ggc tgg ctg act      144
   Thr Pro Asp Ala Trp Pro Thr Trp Pro Val Lys Leu Gly Trp Leu Thr
           35                  40                  45
   cct cgc ggc ggt gag ctc atc gcc tac ctc gga cac tac caa cgc cag      192
   Pro Arg Gly Gly Glu Leu Ile Ala Tyr Leu Gly His Tyr Gln Arg Gln
       50                  55                  60
   cgt ctg gtt gcc gac gga ctc ctg gct gac aag gga tgc ccg cag tct      240
   Arg Leu Val Ala Asp Gly Leu Leu Ala Asp Lys Gly Cys Pro Gln Ser
   65                  70                  75                  80
   ggc cag gtc gcg att atc gcc gat gtc gac gag cgt acc cgt aag acc      288
   Gly Gln Val Ala Ile Ile Ala Asp Val Asp Glu Arg Thr Arg Lys Thr
                   85                  90                  95
   ggc gaa gcc ttc gct gcc ggc ctc gct cct gac tgt gcc atc acg gtc      336
   Gly Glu Ala Phe Ala Ala Gly Leu Ala Pro Asp Cys Ala Ile Thr Val
               100                 105                 110
   cac acc cag gca gac acg tcc agc ccc gat ccg ctg ttt aac cct ctc      384
   His Thr Gln Ala Asp Thr Ser Ser Pro Asp Pro Leu Phe Asn Pro Leu
           115                 120                 125
   aag act ggc gtc tgc caa ctg gat aac gcc cgg gtg acc gag gcc atc      432
   Lys Thr Gly Val Cys Gln Leu Asp Asn Ala Arg Val Thr Glu Ala Ile
       130                 135                 140
   ctc agc agg gct gga ggt tcc atc gcc gac ttc acc ggc cat cgg cag      480
   Leu Ser Arg Ala Gly Gly Ser Ile Ala Asp Phe Thr Gly His Arg Gln
   145                 150                 155                 160
   acg gcg ttc cgc gag ctg gag cgg gtc ctt aat ttt ccc cag tcg aac      528
   Thr Ala Phe Arg Glu Leu Glu Arg Val Leu Asn Phe Pro Gln Ser Asn
                   165                 170                 175
   ctg tgc ctc aag cgt gag aag cag gac gag agc tgt tcc ctg acc cag      576
   Leu Cys Leu Lys Arg Glu Lys Gln Asp Glu Ser Cys Ser Leu Thr Gln
               180                 185                 190
   gca ctc ccg tcg gaa ctc aag ccc tcc gcg gac aac gtc tcc ctt acc      624
   Ala Leu Pro Ser Glu Leu Lys Pro Ser Ala Asp Asn Val Ser Leu Thr
           195                 200                 205
   ggt gcc gtt agc ctc gct tcc atg ctg acg gag atc ttc ctc ctg cag      672
   Gly Ala Val Ser Leu Ala Ser Met Leu Thr Glu Ile Phe Leu Leu Gln
       210                 215                 220
   caa gcg cag gga atg ccc gag cct ggg tgg ggc cgc att acc gat tct      720
   Gln Ala Gln Gly Met Pro Glu Pro Gly Trp Gly Arg Ile Thr Asp Ser
   225                 230                 235                 240
   cac cag tgg aac acc ctg ctc tcg ctt cac aac gcc cag ttc tat ctg      768
   His Gln Trp Asn Thr Leu Leu Ser Leu His Asn Ala Gln Phe Tyr Leu
                   245                 250                 255
   ctc caa cgc acg ccc gag gtt gcc cgc agc cgc gcc acc ccg ctg ctc      816
   Leu Gln Arg Thr Pro Glu Val Ala Arg Ser Arg Ala Thr Pro Leu Leu
               260                 265                 270
   gac ctc atc aag act gcg ctg acg ccc cac cct ccg cag aag cag gct      864
   Asp Leu Ile Lys Thr Ala Leu Thr Pro His Pro Pro Gln Lys Gln Ala
           275                 280                 285
   tac ggt gtc acc ctc ccc act tcc gtc ctg ttt atc gcc ggt cac gac      912
   Tyr Gly Val Thr Leu Pro Thr Ser Val Leu Phe Ile Ala Gly His Asp
       290                 295                 300
   acc aac ctg gcc aat ctc ggc ggc gct ctg gag ctc aac tgg acg ctt      960
   Thr Asn Leu Ala Asn Leu Gly Gly Ala Leu Glu Leu Asn Trp Thr Leu
   305                 310                 315                 320
   ccc gga cag ccg gat aac act ccc cct ggc ggt gag ctg gtg ttc gaa     1008
   Pro Gly Gln Pro Asp Asn Thr Pro Pro Gly Gly Glu Leu Val Phe Glu
                   325                 330                 335
   cgc tgg cgt cgg ctc agc gac aac tcc cag tgg att cag gtt tcg ctg     1056
   Arg Trp Arg Arg Leu Ser Asp Asn Ser Gln Trp Ile Gln Val Ser Leu
               340                 345                 350
   gtc ttc cag acc ctg cag cag atg cgc gac aaa acg ccc ctg tcc ctc     1104
   Val Phe Gln Thr Leu Gln Gln Met Arg Asp Lys Thr Pro Leu Ser Leu
           355                 360                 365
   aat acc cct ccc ggc gag gtc aag ctg acc ctg gca ggc tgt gaa gag     1152
   Asn Thr Pro Pro Gly Glu Val Lys Leu Thr Leu Ala Gly Cys Glu Glu
       370                 375                 380
   cgc aac gcc cag ggc atg tgc tct ctc gct ggc ttt acg caa atc gtg     1200
   Arg Asn Ala Gln Gly Met Cys Ser Leu Ala Gly Phe Thr Gln Ile Val
   385                 390                 395                 400
   aac gag gcc cgc atc ccc gct tgc tct ctg                             1230
   Asn Glu Ala Arg Ile Pro Ala Cys Ser Leu
                   405                 410
<210> 18
   <211> 410
   <212> PRT
   <213> Artificial Sequence
<220>
   <223> DNA PhyM10
<400> 18
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Trp Leu Thr
           35                  40                  45
   Pro Arg Gly Gly Glu Leu Ile Ala Tyr Leu Gly His Tyr Gln Arg Gln
       50                  55                  60
   Arg Leu Val Ala Asp Gly Leu Leu Ala Asp Lys Gly Cys Pro Gln Ser
   65                  70                  75                  80
   Gly Gln Val Ala Ile Ile Ala Asp Val Asp Glu Arg Thr Arg Lys Thr
                   85                  90                  95
   Gly Glu Ala Phe Ala Ala Gly Leu Ala Pro Asp Cys Ala Ile Thr Val
               100                 105                 110
   His Thr Gln Ala Asp Thr Ser Ser Pro Asp Pro Leu Phe Asn Pro Leu
           115                 120                 125
   Lys Thr Gly Val Cys Gln Leu Asp Asn Ala Arg Val Thr Glu Ala Ile
       130                 135                 140
   Leu Ser Arg Ala Gly Gly Ser Ile Ala Asp Phe Thr Gly His Arg Gln
   145                 150                 155                 160
   Thr Ala Phe Arg Glu Leu Glu Arg Val Leu Asn Phe Pro Gln Ser Asn
                   165                 170                 175
   Leu Cys Leu Lys Arg Glu Lys Gln Asp Glu Ser Cys Ser Leu Thr Gln
               180                 185                 190
   Ala Leu Pro Ser Glu Leu Lys Pro Ser Ala Asp Asn Val Ser Leu Thr
           195                 200                 205
   Gly Ala Val Ser Leu Ala Ser Met Leu Thr Glu Ile Phe Leu Leu Gln
       210                 215                 220
   Gln Ala Gln Gly Met Pro Glu Pro Gly Trp Gly Arg Ile Thr Asp Ser
   225                 230                 235                 240
   His Gln Trp Asn Thr Leu Leu Ser Leu His Asn Ala Gln Phe Tyr Leu
                   245                 250                 255
   Leu Gln Arg Thr Pro Glu Val Ala Arg Ser Arg Ala Thr Pro Leu Leu
               260                 265                 270
   Asp Leu Ile Lys Thr Ala Leu Thr Pro His Pro Pro Gln Lys Gln Ala
           275                 280                 285
   Tyr Gly Val Thr Leu Pro Thr Ser Val Leu Phe Ile Ala Gly His Asp
       290                 295                 300
   Thr Asn Leu Ala Asn Leu Gly Gly Ala Leu Glu Leu Asn Trp Thr Leu
   305                 310                 315                 320
   Pro Gly Gln Pro Asp Asn Thr Pro Pro Gly Gly Glu Leu Val Phe Glu
                   325                 330                 335
   Arg Trp Arg Arg Leu Ser Asp Asn Ser Gln Trp Ile Gln Val Ser Leu
               340                 345                 350
   Val Phe Gln Thr Leu Gln Gln Met Arg Asp Lys Thr Pro Leu Ser Leu
           355                 360                 365
   Asn Thr Pro Pro Gly Glu Val Lys Leu Thr Leu Ala Gly Cys Glu Glu
       370                 375                 380
   Arg Asn Ala Gln Gly Met Cys Ser Leu Ala Gly Phe Thr Gln Ile Val
   385                 390                 395                 400
   Asn Glu Ala Arg Ile Pro Ala Cys Ser Leu
                   405                 410
<210> 19
   <211> 1371
   <212> DNA
   <213> Artificial Sequence
<220>
   <223> DNA Phy2005
<220>
   <221> CDS
   <222> (1)..(1371)
   <223>
<400> 19
   ttc tcc tac ggc gct gcc att cct cag tca acc cag gag aag cag ttc      48
   Phe Ser Tyr Gly Ala Ala Ile Pro Gln Ser Thr Gln Glu Lys Gln Phe
   1               5                   10                  15
   tct cag gag ttc cgc gat ggc tac agc atc ctc aag cac tac ggt ggt      96
   Ser Gln Glu Phe Arg Asp Gly Tyr Ser Ile Leu Lys His Tyr Gly Gly
               20                  25                  30
   aac gga ccc tac tcc gag cgt gtg tcc tac ggt atc gcc cgc gat ccc     144
   Asn Gly Pro Tyr Ser Glu Arg Val Ser Tyr Gly Ile Ala Arg Asp Pro
           35                  40                  45
   ccg act agt gag ctg aag ctg gag tcg gtc gtg atc gtc agc cgc cac     192
   Pro Thr Ser Glu Leu Lys Leu Glu Ser Val Val Ile Val Ser Arg His
       50                  55                  60
   ggc gtg cgt gct cct acc aag gcc acg cag ctg atg cag gac gtc acc     240
   Gly Val Arg Ala Pro Thr Lys Ala Thr Gln Leu Met Gln Asp Val Thr
   65                  70                  75                  80
   cct gac gcc tgg ccc acc tgg ccc gtc aag ctt ggc tgg ctg act cct     288
   Pro Asp Ala Trp Pro Thr Trp Pro Val Lys Leu Gly Trp Leu Thr Pro
                   85                  90                  95
   cgc ggc ggt gag ctc atc gcc tac ctc gga cac tac caa cgc cag cgt     336
   Arg Gly Gly Glu Leu Ile Ala Tyr Leu Gly His Tyr Gln Arg Gln Arg
               100                 105                 110
   ctg gtt gcc gac gga ctc ctg gct aag aag gga tgc ccg cag tct ggc     384
   Leu Val Ala Asp Gly Leu Leu Ala Lys Lys Gly Cys Pro Gln Ser Gly
           115                 120                 125
   cag gtc gcg att atc gcc gat gtc gac gag cgt acc cgt aag acc ggc     432
   Gln Val Ala Ile Ile Ala Asp Val Asp Glu Arg Thr Arg Lys Thr Gly
       130                 135                 140
   gaa gcc ttc gct gcc ggc ctc gct cct gac tgt gcc atc acg gtc cac     480
   Glu Ala Phe Ala Ala Gly Leu Ala Pro Asp Cys Ala Ile Thr Val His
   145                 150                 155                 160
   acc cag gca gac acg tcc agc ccc gat ccg ctg ttt aac cct ctc aag     528
   Thr Gln Ala Asp Thr Ser Ser Pro Asp Pro Leu Phe Asn Pro Leu Lys
                   165                 170                 175
   act ggc gtc tgc caa ctg gat aac gcc aac gtg acc gac gcc atc ctc     576
   Thr Gly Val Cys Gln Leu Asp Asn Ala Asn Val Thr Asp Ala Ile Leu
               180                 185                 190
   agc agg gct gga ggt tcc atc gcc gac ttc acc ggc cat cgg cag acg     624
   Ser Arg Ala Gly Gly Ser Ile Ala Asp Phe Thr Gly His Arg Gln Thr
           195                 200                 205
   gcg ttc cgc gag ctg gag cgg gtc ctt aat ttt ccc cag tcg aac ctg     672
   Ala Phe Arg Glu Leu Glu Arg Val Leu Asn Phe Pro Gln Ser Asn Leu
       210                 215                 220
   tgc ctc aag cgt gag aag cag gac gag agc tgt tcc ctg acc cag gca     720
   Cys Leu Lys Arg Glu Lys Gln Asp Glu Ser Cys Ser Leu Thr Gln Ala
   225                 230                 235                 240
   ctc ccg tcg gaa ctc aag gtg agc gcc gac aac gtc tcc ctt acc ggt     768
   Leu Pro Ser Glu Leu Lys Val Ser Ala Asp Asn Val Ser Leu Thr Gly
                   245                 250                 255
   gcc gtt agc ctc gct tcc atg ctg acg gag atc ttc ctc ctg cag caa     816
   Ala Val Ser Leu Ala Ser Met Leu Thr Glu Ile Phe Leu Leu Gln Gln
               260                 265                 270
   gcg cag gga atg ccc gag cct ggg tgg ggc cgc att acc gat tct cac      864
   Ala Gln Gly Met Pro Glu Pro Gly Trp Gly Arg Ile Thr Asp Ser His
           275                 280                 285
   cag tgg aac acc ctg ctc tcg ctt cac aac gcc cag ttc tat ctg ctc      912
   Gln Trp Asn Thr Leu Leu Ser Leu His Asn Ala Gln Phe Tyr Leu Leu
       290                 295                 300
   caa cgc acg ccc gag gtt gcc cgc agc cgc gcc acc ccg ctg ctc gac      960
   Gln Arg Thr Pro Glu Val Ala Arg Ser Arg Ala Thr Pro Leu Leu Asp
   305                 310                 315                 320
   ctc atc aag act gcg ctg acg ccc cac cct ccg cag aag cag gct tac     1008
   Leu Ile Lys Thr Ala Leu Thr Pro His Pro Pro Gln Lys Gln Ala Tyr
                   325                 330                 335
   ggt gtc acc ctc ccc act tcc gtc ctg ttt atc gcc ggt cac gac acc     1056
   Gly Val Thr Leu Pro Thr Ser Val Leu Phe Ile Ala Gly His Asp Thr
               340                 345                 350
   aac ctg gcc aat ctc ggc ggc gct ctg gag ctc aac tgg acg ctt ccc     1104
   Asn Leu Ala Asn Leu Gly Gly Ala Leu Glu Leu Asn Trp Thr Leu Pro
           355                 360                 365
   gga cag ccg gat aac act ccc cct ggc ggt gag ctg gtg ttc gaa cgc     1152
   Gly Gln Pro Asp Asn Thr Pro Pro Gly Gly Glu Leu Val Phe Glu Arg
       370                 375                 380
   tgg cgt cgg ctc agc gac aac tcc cag tgg att cag gtt tcg ctg gtc     1200
   Trp Arg Arg Leu Ser Asp Asn Ser Gln Trp Ile Gln Val Ser Leu Val
   385                 390                 395                 400
   ttc cag acc ctg cag cag atg cgc gac aaa acg ccc ctg tcc ctc aat     1248
   Phe Gln Thr Leu Gln Gln Met Arg Asp Lys Thr Pro Leu Ser Leu Asn
                   405                 410                 415
   acc cct ccc ggc gag gtc aag ctg acc ctg gca ggc tgt gaa gag cgc     1296
   Thr Pro Pro Gly Glu Val Lys Leu Thr Leu Ala Gly Cys Glu Glu Arg
               420                 425                 430
   aac gcc cag ggc atg tgc tct ctc gct ggc ttt acg caa atc gtg aac     1344
   Asn Ala Gln Gly Met Cys Ser Leu Ala Gly Phe Thr Gln Ile Val Asn
           435                 440                 445
   gag gcc cgc atc ccc gct tgc tct ctg                                 1371
   Glu Ala Arg Ile Pro Ala Cys Ser Leu
       450                 455
<210> 20
   <211> 457
   <212> PRT
   <213> Artificial Sequence
<220>
   <223> DNA Phy2005
<400> 20
   Phe Ser Tyr Gly Ala Ala Ile Pro Gln Ser Thr Gln Glu Lys Gln Phe
   1               5                   10                  15
   Ser Gln Glu Phe Arg Asp Gly Tyr Ser Ile Leu Lys His Tyr Gly Gly
               20                  25                  30
   Asn Gly Pro Tyr Ser Glu Arg Val Ser Tyr Gly Ile Ala Arg Asp Pro
           35                  40                  45
   Pro Thr Ser Glu Leu Lys Leu Glu Ser Val Val Ile Val Ser Arg His
       50                  55                  60
   Gly Val Arg Ala Pro Thr Lys Ala Thr Gln Leu Met Gln Asp Val Thr
   65                  70                  75                  80
   Pro Asp Ala Trp Pro Thr Trp Pro Val Lys Leu Gly Trp Leu Thr Pro
                   85                  90                  95
   Arg Gly Gly Glu Leu Ile Ala Tyr Leu Gly His Tyr Gln Arg Gln Arg
               100                 105                 110
   Leu Val Ala Asp Gly Leu Leu Ala Lys Lys Gly Cys Pro Gln Ser Gly
           115                 120                 125
   Gln Val Ala Ile Ile Ala Asp Val Asp Glu Arg Thr Arg Lys Thr Gly
       130                 135                 140
   Glu Ala Phe Ala Ala Gly Leu Ala Pro Asp Cys Ala Ile Thr Val His
   145                 150                 155                 160
   Thr Gln Ala Asp Thr Ser Ser Pro Asp Pro Leu Phe Asn Pro Leu Lys
                   165                 170                 175
   Thr Gly Val Cys Gln Leu Asp Asn Ala Asn Val Thr Asp Ala Ile Leu
               180                 185                 190
   Ser Arg Ala Gly Gly Ser Ile Ala Asp Phe Thr Gly His Arg Gln Thr
           195                 200                 205
   Ala Phe Arg Glu Leu Glu Arg Val Leu Asn Phe Pro Gln Ser Asn Leu
       210                 215                 220
   Cys Leu Lys Arg Glu Lys Gln Asp Glu Ser Cys Ser Leu Thr Gln Ala
   225                 230                 235                 240
   Leu Pro Ser Glu Leu Lys Val Ser Ala Asp Asn Val Ser Leu Thr Gly
                   245                 250                 255
   Ala Val Ser Leu Ala Ser Met Leu Thr Glu Ile Phe Leu Leu Gln Gln
               260                 265                 270
   Ala Gln Gly Met Pro Glu Pro Gly Trp Gly Arg Ile Thr Asp Ser His
   275 280 285
   Gln Trp Asn Thr Leu Leu Ser Leu His Asn Ala Gln Phe Tyr Leu Leu
   290 295 300
   Gln Arg Thr Pro Glu Val Ala Arg Ser Arg Ala Thr Pro Leu Leu Asp
   305 310 315 320
   Leu Ile Lys Thr Ala Leu Thr Pro His Pro Pro Gln Lys Gln Ala Tyr
   325 330 335
   Gly Val Thr Leu Pro Thr Ser Val Leu Phe Ile Ala Gly His Asp Thr
   340 345 350
   Asn Leu Ala Asn Leu Gly Gly Ala Leu Glu Leu Asn Trp Thr Leu Pro
   355 360 365
   Gly Gln Pro Asp Asn Thr Pro Pro Gly Gly Glu Leu Val Phe Glu Arg
   370 375 380
   Trp Arg Arg Leu Ser Asp Asn Ser Gln Trp Ile Gln Val Ser Leu Val
   385 390 395 400
   Phe Gln Thr Leu Gln Gln Met Arg Asp Lys Thr Pro Leu Ser Leu Asn
   405 410 415
   Thr Pro Pro Gly Glu Val Lys Leu Thr Leu Ala Gly Cys Glu Glu Arg
   420 425 430
   Asn Ala Gln Gly Met Cys Ser Leu Ala Gly Phe Thr Gln Ile Val Asn
   435 440 445
   Glu Ala Arg Ile Pro Ala Cys Ser Leu
   450 455
<210> 21
   <211> 1458
   <212> DNA
   <213> Artificial Sequence
<220>
   <223> DNA Phy2006
<220>
   <221> CDS
   <222> (1) .. (1458)
   <223>
<400> 21
   ttc tcc tac ggc gct gcc att cct cag tca acc cag gag aag cag ttc        48
   Phe Ser Tyr Gly Ala Ala Ile Pro Gln Ser Thr Gln Glu Lys Gln Phe
   1               5                   10                  15
   tct cag gag ttc cgc gat ggc tac agc atc ctc aag cac tac ggt ggt       96
   Ser Gln Glu Phe Arg Asp Gly Tyr Ser Ile Leu Lys His Tyr Gly Gly
               20                  25                  30
   aac gga ccc tac tcc gag cgt gtg tcc tac ggt atc gcc cgc gat ccc      144
   Asn Gly Pro Tyr Ser Glu Arg Val Ser Tyr Gly Ile Ala Arg Asp Pro
           35                  40                  45
   ccg act agt gag ctg aag ctg gag tcg gtc gtg atc gtc agc cgc cac      192
   Pro Thr Ser Glu Leu Lys Leu Glu Ser Val Val Ile Val Ser Arg His
       50                  55                  60
   ggc gtg cgt gct cct acc aag gcc acg cag ctg atg cag gac gtc acc      240
   Gly Val Arg Ala Pro Thr Lys Ala Thr Gln Leu Met Gln Asp Val Thr
   65                  70                  75                  80
   cct gac gcc tgg ccc acc tgg ccc gtc aag ctt ggc tgg ctg act cct      288
   Pro Asp Ala Trp Pro Thr Trp Pro Val Lys Leu Gly Trp Leu Thr Pro
                   85                  90                  95
   cgc ggc ggt gag ctc atc gcc tac ctc gga cac tac caa cgc cag cgt      336
   Arg Gly Gly Glu Leu Ile Ala Tyr Leu Gly His Tyr Gln Arg Gln Arg
               100                 105                 110
   ctg gtt gcc gac gga ctc ctg gct aag aag gga tgc ccg cag tct ggc      384
   Leu Val Ala Asp Gly Leu Leu Ala Lys Lys Gly Cys Pro Gln Ser Gly
           115                 120                 125
   cag gtc gcg att atc gcc gat gtc gac gag cgt acc cgt aag acc ggc      432
   Gln Val Ala Ile Ile Ala Asp Val Asp Glu Arg Thr Arg Lys Thr Gly
       130                 135                 140
   gaa gcc ttc gct gcc ggc ctc gct cct gac tgt gcc atc acg gtc cac      480
   Glu Ala Phe Ala Ala Gly Leu Ala Pro Asp Cys Ala Ile Thr Val His
   145                 150                 155                 160
   acc cag gca gac acg tcc agc ccc gat ccg ctg ttt aac cct ctc aag      528
   Thr Gln Ala Asp Thr Ser Ser Pro Asp Pro Leu Phe Asn Pro Leu Lys
                   165                 170                 175
   act ggc gtc tgc caa ctg gat aac gcc aac gtg acc gac gcc atc ctc      576
   Thr Gly Val Cys Gln Leu Asp Asn Ala Asn Val Thr Asp Ala Ile Leu
               180                 185                 190
   agc agg gct gga ggt tcc atc gcc gac ttc acc ggc cat cgg cag acg      624
   Ser Arg Ala Gly Gly Ser Ile Ala Asp Phe Thr Gly His Arg Gln Thr
           195                 200                 205
   gcg ttc cgc gag ctg gag cgg gtc ctt aat ttt ccc cag tcg aac ctg      672
   Ala Phe Arg Glu Leu Glu Arg Val Leu Asn Phe Pro Gln Ser Asn Leu
       210                 215                 220
   tgc ctc aag cgt gag aag cag gac gag agc tgt tcc ctg acc cag gca      720
   Cys Leu Lys Arg Glu Lys Gln Asp Glu Ser Cys Ser Leu Thr Gln Ala
   225                 230                 235                 240
   ctc ccg tcg gaa ctc aag gtg agc gcc gac aac gtc tcc ctt acc ggt      768
   Leu Pro Ser Glu Leu Lys Val Ser Ala Asp Asn Val Ser Leu Thr Gly
                   245                 250                 255
   gcc gtt agc ctc gct tcc atg ctg acg gag atc ttc ctc ctg cag caa      816
   Ala Val Ser Leu Ala Ser Met Leu Thr Glu Ile Phe Leu Leu Gln Gln
               260                 265                 270
   gcg cag gga atg ccc gag cct ggg tgg ggc cgc att acc gat tct cac      864
   Ala Gln Gly Met Pro Glu Pro Gly Trp Gly Arg Ile Thr Asp Ser His
           275                 280                 285
   cag tgg aac acc ctg ctc tcg ctt cac aac gcc cag ttc tat ctg ctc      912
   Gln Trp Asn Thr Leu Leu Ser Leu His Asn Ala Gln Phe Tyr Leu Leu
       290                 295                 300
   caa cgc acg ccc gag gtt gcc cgc agc cgc gcc acc ccg ctg ctc gac      960
   Gln Arg Thr Pro Glu Val Ala Arg Ser Arg Ala Thr Pro Leu Leu Asp
   305                 310                 315                 320
   ctc atc aag act gcg ctg acg ccc cac cct ccg cag aag cag gct tac     1008
   Leu Ile Lys Thr Ala Leu Thr Pro His Pro Pro Gln Lys Gln Ala Tyr
                   325                 330                 335
   ggt gtc acc ctc ccc act tcc gtc ctg ttt atc gcc ggt cac gac acc     1056
   Gly Val Thr Leu Pro Thr Ser Val Leu Phe Ile Ala Gly His Asp Thr
               340                 345                 350
   aac ctg gcc aat ctc ggc ggc gct ctg gag ctc aac tgg acg ctt ccc     1104
   Asn Leu Ala Asn Leu Gly Gly Ala Leu Glu Leu Asn Trp Thr Leu Pro
           355                 360                 365
   gga cag ccg gat aac act ccc cct ggc ggt gag ctg gtg ttc gaa cgc     1152
   Gly Gln Pro Asp Asn Thr Pro Pro Gly Gly Glu Leu Val Phe Glu Arg
       370                 375                 380
   tgg cgt cgg ctc agc gac aac tcc cag tgg att cag gtt tcg ctg gtc     1200
   Trp Arg Arg Leu Ser Asp Asn Ser Gln Trp Ile Gln Val Ser Leu Val
   385                 390                 395                 400
   ttc cag acc ctg cag cag atg cgc gac aaa acg ccc ctg tcc ctc aat     1248
   Phe Gln Thr Leu Gln Gln Met Arg Asp Lys Thr Pro Leu Ser Leu Asn
                   405                 410                 415
   acc cct ccc ggc gag gtc aag ctg acc ctg gca ggc tgt gaa gag cgc     1296
   Thr Pro Pro Gly Glu Val Lys Leu Thr Leu Ala Gly Cys Glu Glu Arg
               420                 425                 430
   aac gcc cag ggc atg tgc tct ctc gct ggc ttt acg caa atc gtg aac     1344
   Asn Ala Gln Gly Met Cys Ser Leu Ala Gly Phe Thr Gln Ile Val Asn
           435                 440                 445
   gag gcc cgg atc ccc gct tgc tct ctg ttg agc ttc tgg tgg aac tac     1392
   Glu Ala Arg Ile Pro Ala Cys Ser Leu Leu Ser Phe Trp Trp Asn Tyr
       450                 455                 460
   aac acc acg acg gag ctg aac tac cgc tct agc cct att gcc tgc cag     1440
   Asn Thr Thr Thr Glu Leu Asn Tyr Arg Ser Ser Pro Ile Ala Cys Gln
   465                 470                 475                 480
   gag ggt gat gct atg gac                                             1458
   Glu Gly Asp Ala Met Asp
                   485
<210> 22
   <211> 486
   <212> PRT
   <213> Artificial Sequence
<220>
   <223> DNA Phy2006
<400> 22
   Phe Ser Tyr Gly Ala Ala Ile Pro Gln Ser Thr Gln Glu Lys Gln Phe
   1               5                   10                  15
   Ser Gln Glu Phe Arg Asp Gly Tyr Ser Ile Leu Lys His Tyr Gly Gly
               20                  25                  30
   Asn Gly Pro Tyr Ser Glu Arg Val Ser Tyr Gly Ile Ala Arg Asp Pro
           35                  40                  45
   Pro Thr Ser Glu Leu Lys Leu Glu Ser Val Val Ile Val Ser Arg His
       50                  55                  60
   Gly Val Arg Ala Pro Thr Lys Ala Thr Gln Leu Met Gln Asp Val Thr
   65                  70                  75                  80
   Pro Asp Ala Trp Pro Thr Trp Pro Val Lys Leu Gly Trp Leu Thr Pro
                   85                  90                  95
   Arg Gly Gly Glu Leu Ile Ala Tyr Leu Gly His Tyr Gln Arg Gln Arg
               100                 105                 110
   Leu Val Ala Asp Gly Leu Leu Ala Lys Lys Gly Cys Pro Gln Ser Gly
           115                 120                 125
   Gln Val Ala Ile Ile Ala Asp Val Asp Glu Arg Thr Arg Lys Thr Gly
       130                 135                 140
   Glu Ala Phe Ala Ala Gly Leu Ala Pro Asp Cys Ala Ile Thr Val His
   145                 150                 155                 160
   Thr Gln Ala Asp Thr Ser Ser Pro Asp Pro Leu Phe Asn Pro Leu Lys
                   165                 170                 175
   Thr Gly Val Cys Gln Leu Asp Asn Ala Asn Val Thr Asp Ala Ile Leu
               180                 185                 190
   Ser Arg Ala Gly Gly Ser Ile Ala Asp Phe Thr Gly His Arg Gln Thr
           195                 200                 205
   Ala Phe Arg Glu Leu Glu Arg Val Leu Asn Phe Pro Gln Ser Asn Leu
       210                 215                 220
   Cys Leu Lys Arg Glu Lys Gln Asp Glu Ser Cys Ser Leu Thr Gln Ala
   225                 230                 235                 240
   Leu Pro Ser Glu Leu Lys Val Ser Ala Asp Asn Val Ser Leu Thr Gly
                   245                 250                 255
   Ala Val Ser Leu Ala Ser Met Leu Thr Glu Ile Phe Leu Leu Gln Gln
               260                 265                 270
   Ala Gln Gly Met Pro Glu Pro Gly Trp Gly Arg Ile Thr Asp Ser His
           275                 280                 285
   Gln Trp Asn Thr Leu Leu Ser Leu His Asn Ala Gln Phe Tyr Leu Leu
       290                 295                 300
   Gln Arg Thr Pro Glu Val Ala Arg Ser Arg Ala Thr Pro Leu Leu Asp
   305                 310                 315                 320
   Leu Ile Lys Thr Ala Leu Thr Pro His Pro Pro Gln Lys Gln Ala Tyr
                   325                 330                 335
   Gly Val Thr Leu Pro Thr Ser Val Leu Phe Ile Ala Gly His Asp Thr
               340                 345                 350
   Asn Leu Ala Asn Leu Gly Gly Ala Leu Glu Leu Asn Trp Thr Leu Pro
           355                 360                 365
   Gly Gln Pro Asp Asn Thr Pro Pro Gly Gly Glu Leu Val Phe Glu Arg
       370                 375                 380
   Trp Arg Arg Leu Ser Asp Asn Ser Gln Trp Ile Gln Val Ser Leu Val
   385                 390                 395                 400
   Phe Gln Thr Leu Gln Gln Met Arg Asp Lys Thr Pro Leu Ser Leu Asn
                   405                 410                 415
   Thr Pro Pro Gly Glu Val Lys Leu Thr Leu Ala Gly Cys Glu Glu Arg
               420                 425                 430
   Asn Ala Gln Gly Met Cys Ser Leu Ala Gly Phe Thr Gln Ile Val Asn
           435                 440                 445
   Glu Ala Arg Ile Pro Ala Cys Ser Leu Leu Ser Phe Trp Trp Asn Tyr
       450                 455                 460
   Asn Thr Thr Thr Glu Leu Asn Tyr Arg Ser Ser Pro Ile Ala Cys Gln
   465                 470                 475                 480
   Glu Gly Asp Ala Met Asp
                   485

## Beschreibung

Die Erfindung betrifft ein rekombinantes DNA-Molekül, das ein Polypeptid mit Phytaseaktivität und erhöhter Temperaturstabilität und erhöhter proteolytischer Stabilität der Enzymaktivität codiert sowie das codierte Polypeptid als solches. Insbesondere betrifft die Erfindung ein rekombinantes DNA-Molekül, das ein Polypeptid mit Phytaseaktivität und erhöhter Temperaturstabilität und erhöhter proteolytischer Stabilität der Enzymaktivität codiert, wobei die DNA-Sequenz durch eine Variation der reifen Wildtyp-*E. coli*-Phytase erhalten worden ist, wobei definierte Aminosäurepositionen im Vergleich zur Wildtypsequenz modifiziert sind bzw. N- und/oder C-terminale Extensionen vorhanden sind. Die Erfindung betrifft ferner ein Verfahren zur Expression der rekombinanten Phytase sowie deren Verwendung in der Lebensmittel- und Futtermitteltechnologie.

Phytinsäure oder Myoinositol-1,2,3,4,5,6-hexakisdihydrogenphosphat (abgekürzt Myoinositol-Hexakisphosphat) ist die Hauptquelle von Inositol und die primäre Speicherform von Phosphat in Pflanzensamen. In den Samen von Hülsenfrüchten liegt etwa 70% des Phosphatgehalts als ein gemischtes Kalium-, Magnesium- und Calciumsalz der Phytinsäure vor. Samen, Getreidekörner und Hülsenfrüchte sind wichtige Komponenten von Lebens- und Futtermittelzubereitungen, insbesondere von Tierfutterpräparaten; aber auch in der menschlichen Nahrung gewinnen Getreide- und Hülsenfrüchte zunehmend an Bedeutung. Die Phosphateinheiten der Phytinsäure binden als Komplex zweiwertige und dreiwertige Kationen wie Metallionen, d.h. ernährungsphysiologisch wichtige Ionen wie Calcium, Eisen, Zink und Magnesium sowie die Spurenelemente Mangan, Kupfer und Molybdän. Daneben bindet Phytinsäure auch zu einem gewissen Ausmaß Proteine durch elektrostatische Wechselwirkung.

Phytinsäure und ihre Salze, die Phytate, werden oft nicht metabolisiert, da sie aus dem Magen-Darm-Trakt nicht absorbiert werden, d.h. weder der darin enthaltene Phosphor noch die chelatierten Metallionen, noch die gebundenen Proteine sind ernährungsphysiologisch verfügbar.

Da Phosphor ein wesentliches Element für das Wachstum aller Organismen ist, müssen Lebensmittel und Futtermittel mit anorganischem Phosphat supplementiert werden. Sehr oft müssen auch die ernährungsphysiologisch essenziellen Ionen wie Eisen und Calcium supplementiert werden. Ferner wird der ernährungsphysiologische Wert jeder Diät vermindert, da Proteine durch Phytinsäure gebunden werden. Folglich wird Phytinsäure oft als Anti-Nährwertfaktor bezeichnet.

Weiterhin wird aufgrund fehlender Metabolisierung von Phytinsäure der Phosphor des Phytats über den Magen-Darm-Trakt der Tiere ausgeschieden, was zu einer unerwünschten Phosphatverunreinigung der Umwelt führt, in deren Folge es beispielsweise zur Eutrophierung von Gewässern und zu exzessivem Al-genwachstum kommen kann.

Phytinsäure oder Phytate (diese Ausdrücke werden im Folgenden synonym verwendet außer anders angegeben) können durch Phytasen abgebaut werden. Phytasen katalysieren die Hydrolyse von Phytat in Myo-Inositol und/oder Mono-, Di-, Tri-, Tetra- und/oder Pentaphosphate sowie anorganisches Phosphat. Man unterscheidet zwei verschiedene Arten von mikrobiellen Phytasen: 1) 3-Phytase/Myo-Inositolhexaphosphat-3-phosphohydrolase, EC 3.1.3.8; 2) 6-Phytase/Myo-Inositolhexaphosphat-6-phosphohydrolase, EC 3.1.3.26. Die 3-Phytase hydrolisiert bevorzugt zuerst die Esterbindung in der 3-Position, die 6-Phytase bevorzugt zuerst die Esterbindung in der 6-Position. Phytinsäure enthaltende Pflanzensamen enthalten endogene Phytaseenzyme. Bei deren Aufnahme sind die Phytate in Lebensmitteln bzw. Futtermitteln theoretisch durch die endogenen Pflanzen-Phytasen, durch Phytasen aus der Darmflora und durch Phytasen aus der Darmschleimhaut hydrolisierbar. In der Praxis ist jedoch das Hydrolysepotenzial der endogenen Pflanzen-Phytasen und der im Darm vorkommenden Phytasen, sofern vorhanden, bei weitem nicht ausreichend, um signifikant die Bioverfügbarkeit des in den Phytaten gebundenen Phosphors zu gewährleisten. Daher werden Lebens- bzw. Futtermitteln häufig exogene Phytasen zugesetzt.

Phytasen können durch Pflanzen sowie durch Mikroorganismen produziert werden. Unter den Mikroorganismen sind Phytase produzierende Bakterien sowie Phytase produzierende Pilze und Hefen bekannt.

Die natürlich vorkommenden Phytase-Produzenten haben jedoch den Nachteil, dass Phytase nur in bestimmten Mengen und mit definierten Eigenschaften gebildet wird. Wie vorstehend dargelegt besteht jedoch ein erhöhter Bedarf an Phytase insbesondere für die Lebensmittel- und Futtermittelindustrie.

Obgleich in der Lebensmittel- und Futtermittelindustrie ein erhöhter Bedarf an Phytase besteht und die Verwendung von Phytase vorteilhaft sein kann, haben nur wenige der bekannten Phytasen eine breite Akzeptanz in der Lebensmittel- und Futtermittelindustrie gefunden. Typische Bedenken betreffen die relativ hohen Herstellungskosten und/oder schlechte Stabilität bzw. Aktivität des Enzyms in der gewünschten Anwendungsumgebung. Weiterhin muss ein derartiges Enzym eine Reihe von Kriterien erfüllen, um industriell einsetzbar zu sein. Diese umfassen eine hohe spezifische Gesamtaktivität, ein niedriges pH-Optimum, Resistenz gegenüber gastrointestinalen Proteasen sowie Temperaturstabilität bzw. Thermostabilität. Die Temperaturstabilität ist eine wichtige Voraussetzung für eine erfolgreiche industrielle Anwendung, da beispielsweise in Pelletierprozessen Enzyme Temperaturen zwischen 60°C und 95°C ausgesetzt werden. Alle bekannten mikrobiellen Phytasen entfalten sich bei Temperaturen zwischen 56°C und 78°C (Lehman et al., 2000), wodurch sie ihre Aktivität verlieren. Daher besteht insbesondere ein Bedarf an Phytasen, die auch bei erhöhten Temperaturen eine technologisch ausreichende Aktivität besitzen, bzw. nicht inaktiviert werden.

Der vorliegenden Erfindung liegt somit die Aufgabe zu Grunde, ein Polypeptid mit Phytaseaktivität bereit zu stellen, das eine erhöhte Thermostabilität aufweist bzw. bei erhöhten Temperaturen eine technologisch ausreichende Aktivität besitzt. Ferner soll das Polypeptid mit Phytaseaktivität auch eine erhöhte proteolytische Stabilität besitzen.

Das Polypeptid soll ökonomisch produziert werden können. Insbesondere soll die Phytase thermostabiler sein als das Wild-Typ-Enzym. Die Phytase soll ferner die wesentlichen Eigenschaften der natürlichen *E. coli*-Wildtyp-Phytase beibehalten, sich jedoch durch eine verbesserte Thermostabilität auszeichnen. Zu den wesentlichen Eigenschaften der natürlichen Wildtyp-Phytase zählen insbesondere die Fähigkeit, die Verfügbarkeit von Phosphat in-vivo und in-vitro zu verbessern durch seine Aktivität als Phytase wie auch als Phosphatase, sein pH-Optimum im sauren mit hoher Restaktivität im stark sauren, sowie die Eignung als Backhilfsmittel.

Der vorliegenden Erfindung liegt ferner die Aufgabe zu Grunde, ein Gen für ein Polypeptid mit Phytaseaktivität mit erhöhter Thermostabilität sowie erhöhter proteolytischen Stabilität bereit zu stellen. Das Polypeptid soll ökonomisch und kostengünstig zu produzieren sein. Insbesondere soll die Expression des Polypeptids in eukaryotischen Mikroorganismen zu einem Polypeptid mit erhöhter Thermostabilität verglichen mit der ähnlich hergestellten Wildtyp-Phytase führen. Bereitgestellt werden sollen ferner die das Polypeptid codierenden DNA-Sequenzen, entsprechende DNA-Konstrukte und Vektoren sowie eine Quelle für das rekombinante Enzym, die für die kommerzielle Verwendung für Nahrungs- und Futtermittel und in industriellen Prozessen geeignet ist und das erfindungsgemäße Enzym enthaltende Zusammensetzungen.

Es wurde nun überraschenderweise gefunden, dass Mutationen an bestimmten Positionen der *E. coli*-Wildtyp-Phytasesequenz zu einer intrinsischen Erhöhung der Thermostabilität bzw. Temperaturstabilität sowie zu einer Erhöhung der proteolytischen Stabilität des Proteins Phytase führen, ohne die sonstigen Wirkungen und wesentlichen Eigenschaften der Wildtyp-*E. coli*-Phytase nachteilig zu beeinflussen.

Überraschenderweise wurde gefunden, dass eine Mutation bei Position 74 (K74D) der Aminosäurensequenz, und/oder eine Kombination von Mutationen bei den Positionen 139 (N139R) und 142 (D142E), und/oder eine Kombination von Mutationen bei den Positionen 145 (L1451) und 198 (L1981) und/oder eine Mutation bei Position 200 (V200P) der Wildtyp-Phytase von *E*. *coli* sowie Kombinationen dieser Mutationen zu einer verbesserten Thermostabilität des Proteins Phytase führen ohne die günstigen Wirkungen und wesentlichen Eigenschaften der Wildtyp-*E. coli*-Phytase zu beeinträchtigen. Weiterhin wurde gefunden, dass die Ergänzung der *E. coli*-Phytase durch Sequenzen der sauren Phosphatase aus *Aspergillus niger* var. *awamori* am N-terminalen oder C-terminalen Ende, bzw. an den N- und C-terminalen Enden, auch in Verbindung mit den vorhergenannten Mutationen, zu einer Verbesserung der Thermostabilität und der proteolytischen Stabilität des Enzyms führen.

Die Erfindung betrifft somit ein rekombinantes DNA-Molekül das nach Expression in einer prokaryotischen oder eukaryotischen Wirtszelle ein Polypeptid mit Phytaseaktivität codiert, wobei das rekombinante DNA-Molekül eine DNA-Sequenz, ausgewählt aus
a) DNA-Sequenzen, die ein Polypeptid mit Phytaseaktivität codieren, das durch Variation der reifen Wildtyp-*E. coli*-Phytasesequenz erhalten worden ist, wobei die Variation die Mutation Lysin → Asparaginsäure an Position 74 (K74D), und gegebenenfalls eine
   Kombination der Mutationen Leucin → Isoleucin an Position 145 (L145I) und Leucin → Isoleucin an Position 198 (L198I), und/oder eine
   Mutation Valin → Prolin an Position 200 (V200P) umfasst, und
b) DNA-Sequenzen, die aufgrund der Degeneriertheit des genetischen Codes mit den Sequenzen gemäß a) und b) verwandt sind,
umfasst, wobei das rekombinante DNA-Molekül bei Expression in einer geeigneten Wirtszelle mit einer erhöhten Temperatur- und Proteasestabilität der Enzymaktivität des so codierten Proteins einhergeht.

Mehrere Phytasen aus *E*. *coli* sind in der Literatur beschrieben, z. B. das für eine Phytase codierende appA-Gen aus *E*. *coli* K-12 (Dassa et al., J. Bacteriol. 172: 5497-5500 (1990)). Dieses Gen codiert für ein periplasmatisches Enzym, das sowohl saure Phosphataseaktivität als auch Phytaseaktivität aufweist (siehe Greiner et al., Arch. Biochim, Biophys. 303: 107-113 (1993)). Natürliche Mutanten dieses Enzyms sind bekannt (vgl. z. B. Rodriguez et al., Biochem. Biophys. Res. Comm. 257: 117-123 (1999)). Genetisch modifizierte Mutanten der *E. coli*-Phytase wurden ebenfalls beschrieben, die zu einer erhöhten Temperaturstabilität und/oder höheren spezifischen Aktivität führten. Rodriguez et al. (Arch. Biochem. Biophys. 382: 105-112 (2000)) exprimierten Wildtyp-AppA und mehrere durch ortsspezifische Mutagenese erzeugte Mutanten in *Pichia pastoris,* um die Wirkung von N-Glycosylierung auf die Temperaturstabilität des AppA-Proteins zu untersuchen. Obwohl die Glycosylierung nicht verstärkt wurde, war eine Mutante bei pH 3,5 bis 5,5 aktiver und wies nach Hitzebehandlung mehr Aktivität auf als das in *P*. *pastoris* produzierte Wildtypprotein.

Die Patentfamilie basierend auf WO 03/057248 beinhaltet die Patentanmeldungen US 2003/0170293 A1 und US 2003/0157646 A1. Darin wird die mikrobielle Herstellung einer thermotoleranten Phytase für Tierfutter beschrieben. Die Mutante (Nov9X) der *E*. *coli* Stamm B Phytase (appA) wird in *E*. *coli, Pichia pastoris*, und *Schizosaccharomyces pombe* zur Expression gebracht. Die Mutante Nov9X enthält 8 Aminosäuremutationen gegenüber dem Wildtyp Enzym. Die Mutante hat in Flüssigkeit bei 70°C gegenüber dem Wildtyp-Enzym eine verbesserte Thermostabilität. Der Wirt in dem das Enzym zur Produktion gebracht wird, hat auch einen Einfluss auf dessen Thermostabilität wie von der gleichen Arbeitsgruppe in der US Patentanmeldung US 2003/0157646 A1 impliziert wird. Die Aminosäurepositionszählweise ist bei NOV9X um 2 Positionen höher als in der vorliegenden Erfindung (W48 NOV9X entspricht W46 in der vorliegenden Erfindung).

In den Patentanmeldungen WO 02/095003 und WO 2004/015084 sind eine Reihe von Punktmutationen der *E*. *coli*-Phytase beschrieben, die jedoch alle nicht zu einer Erhöhung der Thermostabilität führten. Die Nummerierung in WO 2004/015084 ist im Vergleich zur vorliegenden Erfindung um 30-Aminosäurepositionen höher.

Auch die Offenlegungsschrift DE 10 2004 050 410 beschreibt *E*. *coli*-Phytase-Mutanten, jedoch mit dem Zweck der Erhöhung der Sekretionseffizienz bei der Produktion in filamentösen Pilzen. Aussagen zur Erhöhung der Thermostabilität der Mutanten liegen in der genannten Druckschrift nicht vor.

Ferner beschreibt die Druckschrift Garrett et al.; Applied Environ. Microbiol., 2004, 70 (5), 3041-3046, Mutanten der *E. coli*-Phytase mit einer erhöhten thermischen und gastrointestinalen Stabilität.

Es ist praktisch nicht möglich, Vorhersagen über die Wirkung einer bzw. mehrerer Mutationen auf das Verhalten der Enzymaktivität unter Bedingungen erhöhter Temperatur zu treffen. Generell erfolgt bei höheren Temperaturen eine Denaturierung bzw. Entfaltung der Sekundär- und Tertiärstruktur des Proteins.

Die Temperaturstabilität bzw. Thermostabilität von Proteinen ist abhängig von einer Vielzahl an Wechselwirkungen. Die Konformation von Proteinen wird durch eine große Anzahl schwacher Wechselwirkungen aufrechterhalten. Die Stabilisierung kann alle Hierarchieebenen der Proteinstruktur umfassen: Lokale Packung der Polypeptidekette, Sekundär- und Supersekundärstrukturelemente, Domänen und Untereinheiten eines multimeren Proteins. Es gibt zahlreiche Gründe, die hinsichtlich der erhöhten Temperaturstabilität eines Proteins berichtet werden, wobei die häufigsten (a) die Zusammenlagerung von Ionenpaaren innerhalb und/oder zwischen Untereinheiten; (b) verbesserte Packung des hydrophoben Kerns (van-der-Waals-Wechselwirkungen); (c) zusätzliche Netzwerke aus Wasserstoffbrücken; (d) verstärkte Neigung zur Sekundärstrukturbildung; (e) erhöhte Dipolstabilisierung innerhalb der Helix; (f) eine vergrößerte polare Oberfläche; (g) eine verringerte Anzahl und ein verringertes Gesamtvolumen von Kavitäten; (h) die Verringerung von konformationeller Spannung (Schleifenstabilisierung) und (i) Widerstandsfähigkeit gegenüber chemischer Modifikation (Oxidation von Methioninresten und/oder Desaminierung von Asparagin- und Glutaminresten), sind.

Im Stand der Technik sind keine Hinweise dahingehend bekannt, wie und in welcher Art die *E. coli-*Wildtyp-Phytasesequenz gezielt zu verändern ist, um eine Erhöhung der Thermostabilität zu erzielen. Der Stand der Technik enthält ferner keine Hinweise auf die vorstehend genannten Mutationen in der *E*. *coli-*Phytasesequenz.

Der Stand der Technik enthält insbesondere keine Hinweise dahingehend, dass eine Mutation bei Position 74 (K74D) der Aminosäuresequenz oder eine Kombination von Mutationen bei den Positionen 139 (N139R) und 142 (D142E) oder eine Kombination von Mutationen bei den Positionen 145 (L1451) und 198 (L198I) oder eine Mutation bei Position 200 (V200P) der Wildtyp-Phytase von *E*. *coli* oder Kombinationen dieser Mutationen zu einer verbesserten Thermostabilität des Proteins Phytase führen ohne die günstigen Wirkungen und wesentlichen Eigenschaften der Wildtyp-*E*. *coli*-Phytase zu beeinträchtigen. Ferner enthält der Stand der Technik keine Hinweise darauf, dass die Ergänzung der *E*. *coli*-Phytasesequenz durch Sequenzen der sauren Phosphatase aus *Aspergillus niger* var. *awamori* am N-terminalen oder C-terminalen Ende, bzw. N- und C-terminalen Enden, auch in Verbindung mit den vorher genannten Mutationen, zu einer Verbesserung der Thermostabilität des Enzyms führt.

Bevorzugte erfindungsgemäße rekombinante DNA-Moleküle mit den erfindungsgemäßen Variationen der reifen Wildtyp-*E*. *coli*-Phytasesequenz sind die Konstrukte PhyM7 und PhyM10. Sie und weitere Konstrukte sind in der nachstehenden Tabelle 1 dargestellt. Die Konstruktion PhyM1 wurde als Vergleichsprodukt aufgenommen.

**Tabelle 1: Genotypen der Mutationen PhyM1, PhyM2, PhyM3, PhyM7, PhyM9 und PhyM10**

| **Genotyp** | **AS Position** | **Wildtypsequenz** | **Mutationen** |
|---|---|---|---|
| ***PhyM1*** | **200** | **E-L-K** Val²⁰⁰ **S-A-D** | **E-L-K** Tyr²⁰⁰ **S-A-D** |
| ***PhyM2*** | **139** | **D-N-A**-Asn¹³⁹-**V-T-D¹⁴²-A** | **D-N-A**-Arg¹³⁹-**V-T-E¹⁴²-A** |
| | **142** | **D-N-A-N¹³⁹-V-T**-Asp¹⁴²-**A** | **D-N-A-R¹³⁹-V-T-**Glu¹⁴²-**A** |
| ***PhyM3*** | **200** | **E-L-K** Val²⁰⁰ **S-A-D** | **E-L-K** Pro²⁰⁰ **S-A-D** |
| ***PhyM7*** | **74** | **L-L-A**-Lys⁷⁴-**K-G** | **L-L-A-**Asp⁷⁴-**K-G** |
| ***PhyM9*** | **145** | **D-A-I** Leu¹⁴⁵**S-R-A** | **D-A-I** Ile¹⁴⁵**S-R-A** |
| | **198** | **P-S-E** Leu¹⁹⁸ **K-V-S** | **P-S-E** Ile¹⁹⁸ **K-V-S** |
| ***PhyM10*** | **74** | **L-L-A-**Lys⁷⁴-**K-G** | **L-L-A-**Asp⁷⁴**-K-G** |
| | **139** | **N-A**-Asn¹³⁹-**V-T-D¹⁴²-A** | **D-N-A**-Arg¹³⁹-**V-T-E¹⁴²-A** |
| | **142** | **N-A-N¹³⁹-V-T-**Asp¹⁴²-**A** | **D-N-A-R¹³⁹-V-T**-Glu¹⁴²-**A** |
| | **200** | **E-L-K** Val²⁰⁰ **S-A-D** | **E-L-K** Pro²⁰⁰ **S-A-D** |

Die folgenden Plasmide sind bei der Deutschen Sammlung von Mikroorganismen und Zellkulturen GmbH (DSMZ), Inhoffenstr. 7B, 38124 Braunschweig, am 18.10.2006 gemäß den Bedingungen des Budapester Vertrags hinterlegt worden.

| **Plasmid** | **Hinterlegungsnummer** |
|---|---|
| pET-PhyM2 | DSM 18715 |
| pUC-PhyM3 | DSM 18717 |
| pET-PhyM7 | DSM 18716 |
| pUC-PhyM9 | DSM 18718 |
| pUC-PhyM10 | DSM 18719 |
| pPhy2005 | DSM 18720 |
| pPhy2006 | DSM 18721 |

Es war überraschend, dass Mutationen, die die hydrophobe Oberfläche des Proteins beeinflussen (PhyM3, PhyM7 und PhyM9), sowie Mutationen, die eine ionische Bindung in Helix D (PhyM2) einfügen, bzw. den Loop B4 über eine ionische Brücke stabilisieren (PhyM7) signifikant zur Erhöhung der Thermostabilität der *E. coli*-Phytase beitragen.

Auch das Anfügen von Sequenzteilen der *Aspergillus niger* var. *awamori* sauren Phosphatase, die bei der sauren Phosphatase zu einer Di- und Tetramerbildung führen, hatte stabilisierende Effekte bei der *E. coli*-Phytase. Entsprechendes gilt für die Anfügung von Sequenzteilen der Phytase von *Aspergillus niger.*

Die erfindungsgemäßen Polypeptide mit Phytaseaktivität zeigen verglichen mit dem *E. coli*-Wildtyp-Enzym eine erhöhte Temperatur bzw. Thermostabilität ihrer Enzymaktivität. Diese Verbesserung der Thermostabilität kann in Flüssigkeit mittels Differential Scanning Calorimetry (DSC) gemessen werden. Insbesondere zeigt sich die Verbesserung der Thermostabilität an der stark erhöhten Restaktivität der Enzymmutanten gegenüber dem Wildtyp-Enzym, wenn sie der thermischen Belastung ausgesetzt werden, wie sie bei der Pelletierung von Tierfutter verwendet wird. Ferner besitzen die erfindungsgemäßen Polypeptide auch eine erhöhte Stabilität gegenüber proteolytischem Abbau wie er im speziellen im Magen von Geflügel und Monogastriern auftritt. Da die Phytase bei der Tierernährung speziell während der Magenpassage das Phosphat aus Phytinsäure und deren Abbauprodukten freisetzen muss, ist diese Stabilität von besonderem Interesse um die Dosage des Enzyms möglichst klein zu halten. Aufgrund der erhöhten Temperatur- und/oder proteolytischen Stabilität eignen sich die erfindungsgemäßen Phytasen insbesondere für Anwendungen, bei denen erhöhte Temperaturbedingungen auftreten bzw. bei denen mit einem erhöhten proteolytischen Abbau zu rechnen ist. Anwendungsbeispiele hierfür sind die Herstellung des Enzyms, bei der es vor Proteasen des Wirtsstammes geschützt werden muß, die Herstellung von pelletiertem Tierfutter, jedoch auch die Anwendung als Flüssigenzym bei der Post-pelleting Aufbringung auf das pelletierte Tierfutter. Eine erhöhte Temperaturstabilität erlaubt die frühere Aufbringung auf die noch nicht so weit abgekühlten Pellets, wodurch die Verdunstungszeit der aufgebrachten Flüssigkeit verkürzt wird. Auch mikrobiologisch bringt dies Vorteile, da dann der hohe Wassergehalt nur bei hohen Temperaturen kurzzeitig vorliegt, bei denen die meisten pathogenen Keime nicht wachsen können.

Die erfindungsgemäße eine Phytase codierende DNA-Sequenz umfasst die Mutation Lysin → Asparaginsäure an Position 74 (K74D), und gegebenenfalls
eine Kombination der Mutationen Leucin → Isoleucin an Position 145 (L145I) und Leucin → Isoleucin an Position 198 (L198I), und/oder
eine Mutation Valin → Prolin an Position 200 (V200P).

Eine bevorzugte Kombinationsmöglichkeit stellt die Kombination dar, die im Weiteren als Genotyp PhyM10 bezeichnet wird.

Ferner kann zusätzlich zu den beschriebenen Mutationen eine N-terminale oder C-terminale oder eine N- und C-terminale Anfügung eines Sequenzabschnitts der sauren Phosphatase von *Aspergillus niger* var. *awamori* vorhanden sein. Auch das alleinige Vorhandensein dieser Sequenzabschnitte führt bereits zu einer Erhöhung der Thermostabilität. Bei dem N-terminalen Abschnitt der sauren Phosphatase aus *A. niger* var. *awamori* handelt es sich um die ersten 51 Aminosäuren des reifen Proteins (FSYGAAIPQS TQEKQFSQEF RDGYSILKHY GGNGPYSERV SYGIARDPPTS). Die letzten 4 Aminosäuren dieses Polypeptids ersetzen dabei die ersten 4 Aminosäuren (QSEP) der reifen *E*. *coli* Phytase. Auch das Anfügen von Teilen dieses Abschnitts der sauren Phosphatase ist möglich. Alternativ kann auch der N-terminale Teil oder Teile davon, der ersten 40 Aminosäuren des reifen Proteins der *Aspergillus niger* Phytase verwendet werden (SCDTVDQGYQ CFSETSHLWG QYAPFFSLAN ESVISPEV-PA). Die Anfügung an die *E*. *coli* Phytase kann auch an anderen Stellen als an der beschriebenen Position 4 erfolgen, solange die Aktivität des Enzyms erhalten bleibt. Zur C-terminalen Verlängerung wurden die letzten 29 Aminosäuren (LSFWWNYNTT TELNYRSSPI ACQEGDAMD) der sauren Phosphatase aus *A*. *niger* var. *awamori* direkt an das C-terminale Ende der *E*. *coli* Phytase fusioniert. Diese Fusion, bzw. Fusionen, können auch mit weiteren Mutationen in der *E*. *coli* Phytase einhergehen und sind nicht beschränkt auf die in dieser Anmeldung aufgeführten weiteren Mutationen. Durch die Mutation Lys43Cys würde die Möglichkeit einer Disulphidbrückenbildung zwischen dem neuen C-terminalen Teil und dem *E*. *coli* Phytase Kern ermöglicht und damit eine verbesserte Dimerisierung. Weitere Mutationen, die van der Waals, hydrophobe oder ionische intermolekulare Brücken begünstigen oder stabilisieren und damit die Thermostabilität erhöhen oder noch weiter verbessern können, sind möglich. Beispiele für die N- bzw. C-terminalen Sequenzverlängerungen sind die nachstehend beschriebenen Ausführungsformen Phy2005 und Phy2006.

Die Thermostabilität der erfindungsgemäßen Phytase kann in Handelsprodukten noch weiter erhöht werden, z. B. durch Zugabe von Stoffen in das Ultrafiltrationskonzentrat vor der Trocknung oder Granulation. Beispielsweise kann eine stabile Formulierung des erfindungsgemäßen Phytaseenzyms dadurch hergestellt werden, dass ein Gemisch aus einer flüssigen Enzymlösung auf ein Füllmittel wie Maltodextrin gesprüht wird und dann das Gemisch getrocknet wird, oder der flüssigen Enzymlösung wird vor dem Trocknen 20% - 60% Magermilchpulver (w/w bezogen auf Gesamtprotein der Enzymlösung) und/oder 1 % - 5% Calciumpropionat (w/w bezogen auf Gesamtprotein der Enzymlösung) zugesetzt und der pH-Wert auf einen definierten Wert eingestellt, insbesondere auf pH 5,2 ± 0,5. Die Verringerung der Feuchtigkeit und die Bindungswechselwirkungen der Phytase mit dem Füllmittel schützen das Enzym zusätzlich zu seiner in seiner Struktur definierten Stabilität vor Umwelteinflüssen wie Temperaturextrema, die während der Herstellung des Futtermittels auftreten können. Trockene und flüssige Formulierungen können weiter stabilisiert werden, wenn die Aktivität potenzieller proteolytischer Enzyme verringert wird, die als Nebenprodukte in dem Flüssigfermentationsgemisch vorhanden sein können, das zur Herstellung des erfindungsgemäßen Enzyms verwendet wird.

Die der erfindungsgemäß mutierten Phytasesequenz entsprechende DNA-Sequenz kann unter Verwendung beliebiger Codon usages realisiert werden. So kann beispielsweise die Codon usage des zur Expression verwendeten Mikroorganismus verwendet werden, es kann aber auch die *E. coli*-Codon-usage bzw. eine Variante davon verwendet werden. Ferner kann die erfindungsgemä-βe mutierte *E. coli*-Phytasesequenz weitere Sequenzvariationen enthalten. Dabei können beliebige Variationen zusätzlich zu den vorstehend beschriebenen Mutationen vorgenommen werden, solange die Eigenschaft der erhöhten Temperaturstabilität nicht nachteilig beeinträchtigt wird und solange die enzymatische Aktivität und weitere wesentliche Eigenschaften der *E. coli*-Wildtyp-Phytase beibehalten werden.

Entsprechende Variationen sind einem Fachmann auf dem Gebiet der Technik der rekombinanten DNA gut bekannt und umfassen die vorstehenden Mutationen sowie die nachstehend dargelegten beispielhaften Variationen.

Erfindungsgemäß können Additions- und/oder Deletionsmoleküle des erfindungsgemäß modifizierten Polypeptids verwendet werden. So kann das erfindungsgemäß modifizierte Polypeptid mit Phytaseaktivität durch Anfügen weiterer Sequenzen am N-terminalen und/oder C-terminalen Ende verlängert werden. Dadurch können Hybridmoleküle hergestellt werden, die weitere vorteilhafte Eigenschaften besitzen. Beispielsweise können Fusionsproteine bzw. nativ in hohem Maße sekretierte Proteine angefügt werden, wodurch die Sekretionseffizienz verbessert wird. Bevorzugt wird dazu ein Teil des CBH2-Proteins aus *Trichoderma reesei,* von Aminosäure Met 1 bis Ser 86, verwendet.

Erfindungsgemäß können auch Sequenzabschnitte des Polypeptids mit Phytaseaktivität deletiert werden, solange die Eigenschaft der erhöhten Temperaturstabilität unter Beibehaltung der Phytaseaktivität nicht beeinträchtigt wird.

Die Mutationen, Elongationen und Verkürzungen können in an sich bekannter Weise nach auf dem Fachgebiet gut bekannten Verfahren durchgeführt werden.

Die vorstehend in Betracht gezogenen Veränderungen des Polypeptids mit Phytaseaktivität entsprechen entsprechenden Mutationen bzw. Modifikationen des dazugehörigen DNA-Moleküls. Auch beschrieben werden Sequenzen, die unter relaxierten oder stringenten Bedingungen mit den erfindungsgemäßen Sequenzen hybridisieren. Als stringente Bedingungen gelten: Hybridisierung bei 65°C, 18 h in Dextransulfat-Lösung (GenescreenPlus, DuPont), danach waschen der Filter jeweils 30 min zuerst mit 6 x SSC, zweimal 2 x SSC, zweimal 2 x SSC mit 0, 1 % SDS und anschließend mit 0,2 x SSC bei 65°C (Membrane transfer and detection methods, Amersham).

Auch beschrieben werden Sequenzen, die mit der beanspruchten Nukleotidsequenz bzw. den beanspruchten Teilen davon eine Homologie von mindestens 70%, bevorzugter mindestens 80%, noch bevorzugter mindestens 90% und insbesondere mindestens 95% aufweisen, solange die jeweiligen Sequenzen zur Erhöhung der Temperaturstabilität des durch sie codierten Polypeptids mit Phytaseaktivität führen. Bevorzugt beträgt die Homologie 70 bis zu 100 Prozent. Die Homologie ist dabei als Grad der Identität definiert. Der Grad der Identität wird dabei bevorzugt so bestimmt, dass man die Anzahl der Reste der kürzeren Sequenz, die an dem Vergleich beteiligt ist und die ein "entsprechendes" Gegenstück in der anderen Sequenz besitzt, ermittelt. Die Homologie wird dabei bevorzugt in üblicher Weise unter Verwendung der üblichen Algorithmen bestimmt. Zum Vergleich werden nur die cDNAs der jeweiligen reifen Proteine herangezogen. Als homologe Sequenzen werden ähnliche, bevorzugt identische, Sequenzgegenstücke mit Hilfe von bekannten Computerprogrammen ermittelt. Ein Beispiel für ein derartiges Programm ist das Programm Clone Manager Suite, das den Programmteil Align Plus enthält und von Scientific & Educational Software, Durham, NC, USA vertrieben wird. Dabei wird ein Vergleich zweier wie vorstehend definierter DNA-Sequenzen durchgeführt, unter der Option local alignment entweder nach der Methode FastScan - MaxScore oder nach der Methode Needleman-Wunsch unter Beibehaltung der Defaultwerte. Speziell wurde erfindungsgemäß zur Berechnung der Homologie die Programmversion "Clone Manager 7 Align Plus 5" mit den Funktionen "Compare Two Sequences/Local Fast Scan-Max Score/Compare DNA sequences" angewendet. Dabei wurden die aus den folgenden Quellen zugänglichen Algorithmen verwendet: Hirschberg, D.S. (1975) A linear space algorithm for computing longest common subsequences, Commun Assoc Comput Mach 18:341-343; Myers, E.W. and W. Miller. (1988) Optimal alignments in linear space, CABIOS 4:1, 11-17; Chao, K-M, W.R. Pearson and W. Miller. (1992) Aligning two sequences within a specified diagonal band, CABIOS 8:5, 481-487.

Die Erfindung betrifft ferner auch DNA-Sequenzen, die aufgrund der Degeneriertheit des genetischen Codes mit den erfindungsgemäßen Sequenzen verwandt sind. Die Degeneriertheit des genetischen Codes kann sich dabei aufgrund natürlicher Degeneriertheit oder aufgrund eines speziell gewählten Codongebrauchs ergeben. Natürlich vorkommende allelische Varianten können unter Verwendung gut bekannter Techniken der Molekularbiologie wie z. B. der Polymerase-Kettenreaktion (PCR) und Hybridisierungstechniken identifiziert werden.

Eine DNA-Sequenz, die ein erfindungsgemäßes Polypeptid codiert, kann verwendet werden, um beliebige Wirtszellen wie beispielsweise Zellen von Pilzen, Hefen, Bakterien, Pflanzen oder Säugern zu transformieren. Derart transformierte Zellen zeichnen sich durch eine Produktion und ev. Sekretion an Phytase mit erhöhter Thermostabilität aus. Das so produzierte Phytaseenzym mit erhöhter Thermostabilität führt auch zu einer effizienten Phosphatfreisetzung aus Phytaten.

Die Ausdrücke Protein, Peptid und Polypeptid sollen austauschbar verwendet werden. Ein Polypeptid oder Enzym mit Phytaseaktivität oder eine Phytase soll jedes Enzym bezeichnen, das die Freisetzung von anorganischem Phosphat aus verschiedenen Myoinositol-Phosphaten bewirken kann. Beispiele für solche Myoinositol-Phosphat(Phytase)-Substrate sind Phytinsäure und beliebige Salze davon, beispielsweise Natriumphytat oder Kaliumphytat oder gemischte Salze. Auch beliebige Positionsisomere der Di-, Tri-, Tetra- oder Pentaphosphate von Myoinositol können als Phytasesubstrat dienen. Die Phytaseaktivität kann unter Verwendung jedes beliebigen Assays, bei dem eines dieser Substrate verwendet wird, bestimmt werden. Eine Phytase-Variante umfasst Polypeptidvarianten, die von einer speziellen Phytase durch Deletion oder Addition einer oder mehrerer Aminosäuren an das N-terminale und/oder C-terminale Ende des nativen Proteins, Deletion oder Addition einer oder mehrerer Aminosäuren an einer oder mehreren Stellen in dem nativen Protein oder Substitution einer oder mehrerer Aminosäuren an einer oder mehreren Stellen in der Phytase abgeleitet sind. Die Erzeugung solcher Varianten ist allgemein auf dem Fachgebiet bekannt. Beispielsweise können Aminosäuresequenz-Varianten der Polypeptide durch Mutation in der DNA hergestellt werden. Verfahren zur Mutagenese und Nucleotidsequenz-Veränderung sind auf dem Fachgebiet gut bekannt (vgl. beispielsweise Kunkel, Proc. Natl. Acad. Sci. USA, 82:488 (1985), Kunkel et al., Methods in Enzymol., 154:367 (1987), US-Patentnr. 4,873,192, Walker und Gaastra, eds., Techniques in Molecular Biology, Mac Millan Publishing Company, New York (1983)). Hinweise über geeignete Aminosäuresubstitutionen, die die biologische Aktivität des Proteins von Interesse nicht beeinträchtigen, finden sich in dem Model von Dayhoff et al., Atlas of Protein Sequence and Structure, Natl. Biomed. Res. Found., Washington, D.C. (1978). Konservative Substitutionen wie der Austausch einer Aminosäure gegen eine andere mit ähnlichen Eigenschaften sind bevorzugt.

Derartige innerhalb einer Gruppe austauschbare Aminosäuren sind, ohne dadurch beschränkt zu werden, in der nachstehenden Tabelle beispielhaft angeführt.

| | | |
|---|---|---|
| Aliphatisch | Nicht-Polar | GAP |
| | | I L V |
| | Polar und ungeladen | C S T M N Q |
| | Polar und geladen | D E |
| | | K R |
| Aromatisch | | H F W Y |

Die Erfindung betrifft auch isolierte oder im Wesentlichen gereinigte Nucleinsäure- oder Proteinzusammensetzungen. Dabei bezeichnet ein isoliertes und gereinigtes Polynucleotid/Polypeptid bzw. Segment davon ein Polynucleotid bzw. Polypeptid bzw. Segment davon, das isoliert aus seiner nativen Umgebung vorliegt. Ein isoliertes Polynucleinsäuresegment oder Polypeptid kann in gereinigter Form vorliegen oder kann in einer nicht nativen Umgebung vorliegen, wie beispielsweise in einer transgenen Wirtszelle. Beispielsweise ist ein isoliertes oder gereinigtes Polynucleotidsegment oder -Protein oder ein biologisch aktiver Teil davon im Wesentlichen frei von weiterem zellulärem Material oder Kulturmedium bei Produktion nach rekombinanten Techniken oder im Wesentlichen frei von chemischen Vorläufern oder anderen chemischen Verbindungen. Bevorzugt ist ein isoliertes Polynucleotid frei von Sequenzen (bevorzugt Proteincodierenden Sequenzen), die natürlicherweise die Nucleinsäure (d.h. Sequenzen, die an den 5'- und 3'-Enden der Nucleinsäure lokalisiert sind) in der genomischen DNA des Organismus, aus dem die Nucleinsäure stammt, flankieren. Beispielsweise kann gemäß verschiedenen Ausführungsformen das isolierte Nucleinsäuremolekül weniger als etwa 5 kb, 4 kb, 3 kb, 2 kb, 1 kb, 0,5 kb oder 0,1 kb Nucleotidsequenzen enthalten, die natürlicherweise das Nucleotinsäuremolekül in der genomischen DNA der Zelle, aus der die Nucleinsäure abgeleitet ist, flankieren. Ein Protein, das im Wesentlichen frei von zellulärem Material ist, umfasst Zusammensetzungen von Protein oder Polypeptid mit weniger als etwa 70%, 50%, 30%, 20%, 10%, 5% (bezogen auf das Trockengewicht) von verunreinigendem Protein. Wenn das erfindungsgemäße Protein oder ein biologisch aktives Fragment davon rekombinant produziert wird, umfasst bevorzugt das Kulturmedium weniger als etwa 70%, 50%, 30%, 20%, 10% oder 5% (bezogen auf das Trockengewicht) der chemischen Vorläufer oder nichtproteinartigen chemischen Substanzen. Fragmente und Varianten der erfindungsgemäßen Nucleotidsequenzen oder Proteine oder Proteinsegmente, die dadurch codiert werden, sind ebenfalls erfindungsgemäß umfasst. Fragment bezeichnet einen Teil der Nucleotidsequenz oder einen Teil der Aminosäuresequenz und daher einen Teil des Polypeptids oder Proteins, welches davon codiert wird.

Die Erfindung betrifft auch Expressionskassetten, die zur Einschleusung des eine erfindungsgemäße Phytase codierenden offenen Leserasters in eine Wirtszelle verwendet werden können. Sie umfassen bevorzugt eine Transkriptionsstartregion, die mit dem offenen Leseraster verknüpft ist. Eine derartige Expressionskassette kann eine Vielzahl von Restriktionsspaltstellen zur Insertion des offenen Leserasters und/oder anderer DNAs, z. B. einer Transkriptions-Regulator-Region und/oder selektierbare Markergene enthalten. Die Transkriptionskassette umfasst in der 5'→3'-Richtung der Transkription eine Transkriptions- und Translationsstartregion, die DNA-Sequenz von Interesse und eine Transkriptions- und Translationsstopregion, die in einer mikrobiellen Zelle funktionell ist. Die Terminationsregion kann bezüglich der Transkriptioninitiationsregion nativ sein, kann bezüglich der DNA-Sequenz von Interesse nativ sein oder kann von einer beliebigen anderen Quelle abgeleitet sein.

Der Ausdruck "offenes Leseraster" (ORF) bezeichnet die Aminosäure-Sequenz, die zwischen den Translationsstart- und -Stop-Codons einer codierenden Sequenz codiert wird. Die Ausdrücke "Start-Codon" und "Stop-Codon" bezeichnen eine Einheit aus drei benachbarten Nukleotiden (Codons) in einer codierenden Sequenz, die den Kettenstart und -stop der Proteinsynthese (mRNA-Translation) spezifizieren.

"Funktionelle Verknüpfung" bezeichnet im Zusammenhang mit einer Nukleinsäure eine Verbindung als Teil des gleichen Nukleinsäuremoleküls in geeigneter Positionierung und Orientierung zum Transkriptionsstart des Promotors. DNA in funktioneller Verknüpfung an einen Promotor befindet sich unter der Transkriptions-Initiations-Regulation des Promotors. Codierende Sequenzen können funktionell mit der Regulatorsequenz in Sense- oder Antisense-Orientierung verknüpft sein. Bei Bezugnahme auf Polypeptide bedeutet funktionelle Verknüpfung die Verbindung als Teil desselben Polypeptids, d.h. über Peptidylbindungen.

Erfindungsgemäß können beliebige Promotoren verwendet werden. Promotor bezeichnet die Nukleotidsequenz üblicherweise stromaufwärts (5') bezüglich der codierenden Sequenz und kontrolliert die Expression der codierenden Sequenz, indem die Erkennung für die RNA-Polymerase und anderer Faktoren, die für die richtige Transkription erforderlich sind, bereit gestellt wird. Der erfindungsgemäß verwendete Promotor kann einen Minimalpromotor umfassen, d.h. eine kurze DNA-Sequenz aus einer TATA-Box und anderen Sequenzen, die die Transkriptionsstartstelle spezifizieren, an die Regulatorelemente zur Kontrolle der Expression angefügt sind.

Der erfindungsgemäße Promotor kann auch eine Nucleotidsequenz umfassen, die einen Minimalpromotor und Regulatorelemente umfasst, der die Expression einer codierenden Sequenz oder funktionellen RNA kontrollieren kann. Dieser Typ von Promotorsequenz besteht aus proximalen und distalen stromaufwärts gelegenen Elementen, wobei die zuletzt genannten Elemente oft als Enhancer bezeichnet werden. Folglich ist ein Enhancer eine DNA-Sequenz, die die Promotor-Aktivität stimulieren kann und kann ein dem Promotor innewohnendes Element oder ein insertiertes heterologes Element sein, um die Expressionshöhe oder Gewebspezifität eines Promotors zu verstärken. Er kann in beiden Orientierungen funktionieren und kann selbst bei stromaufwärtiger oder stromabwärtiger Platzierung von dem Promotor funktionieren. Sowohl Enhancer als auch andere stromaufwärts gelegene Promotor-Elemente binden sequenzspezifisch DNA-bindende Proteine, die ihre Wirkungen vermitteln. Promotoren können in ihrer Gesamtheit von einem nativen Gen abgeleitet sein oder können aus verschiedenen Elementen zusammengesetzt sein, die von verschiedenen natürlich vorkommenden Promotoren abgeleitet sind oder können sogar aus synthetischen DNA-Segmenten zusammengesetzt sein. Ein Promotor kann auch DNA-Sequenzen enthalten, die an der Bindung von Proteinfaktoren beteiligt sind, die die Effizienz der Transkriptionsinitiation als Antwort auf physiologische oder entwicklungsbedingte Bedingungen kontrollieren.

Promotorelemente, insbesondere TATA-Elemente, die inaktiv sind oder eine stark verminderte Promotor-Aktivität in Abwesenheit einer stromaufwärtigen Aktivierung besitzen, werden als Minimalpromotoren oder Kernpromotoren bezeichnet. In Gegenwart eines geeigneten Transkriptionfaktors bzw. geeigneter Transkriptionsfaktoren liegt die Funktion des Minimalpromotors in der Ermöglichung der Transkription. Ein Minimal- oder Kernpromotor besteht somit nur aus allen Grundelementen, die für die Transkriptionsinitiation notwendig sind, z. B. einer TATA-Box und/oder einem Initiator.

Die Erfindung betrifft auch die erfindungsgemäße DNA enthaltende Vektoren. Diese Vektoren umfassen beliebige Plasmide, Cosmide, Phagen und andere Vektoren in doppelsträngiger oder einzelsträngiger, linearer oder zirkulärer Form, die gegebenenfalls selbst transmittierbar oder mobilisierbar sein können und die einen prokaryotischen oder eukaryotischen Wirt entweder durch Integration in das zelluläre Genom transformieren können oder die extrachromosomal vorliegen (z. B. autonom replizierende Plasmide mit einem Replikationsorigin).

Vektoren, Plasmide, Cosmide, künstliche Hefechromosomen (YACs), künstliche Bakterienchromosomen (BACs) und DNA-Segmente zur Verwendung zur Transformation von Zellen umfassen allgemein die erfindungsgemäße Phytase-codierende DNA sowie eine andere DNA, wie cDNA, ein Gen oder Gene, die in die Zellen eingeschleust werden sollen. Diese DNA-Konstrukte können weitere Strukturen wie Promotoren, Enhancer, Polylinker oder auch Regulatorgene, so weit erforderlich, umfassen. Eines der DNA-Segmente oder Gene, das bzw. die für die zelluläre Einschleusung ausgewählt wurde bzw. wurden, codiert/codieren zweckdienlicherweise ein Protein, das in den so erhaltenen transformierten (rekombinanten Zellen) exprimiert wird, was zu einem screenbaren oder selektierbaren Merkmal führt und/oder der transformierten Zelle einen verbesserten Phenotyp verleiht.

Die Konstruktion von Vektoren, die erfindungsgemäß verwendet werden können, ist einem Fachmann auf dem Gebiet angesichts der vorstehenden Offenbarung bekannt (vgl. z. B. Sambrook et al., Molecular Cloning: A Laboratory Manual (2. Aufl., Coldspring Harbor Laboratory Press, Plainview, N.Y. (1989)). Die erfindungsgemäße Expressionskassette kann ein oder mehrere Restriktionsschnittstellen enthalten, um das die Phytase-codierende Nucleotid unter die Regulation einer Regulatorsequenz zu stellen. Die Expressionskassette kann auch ein Terminationssignal in funktioneller Verknüpfung mit dem Polynukleotid sowie Regulatorsequenzen, die für die ordnungsgemäße Translation des Polynukleotids benötigt werden, enthalten. Die Expressionskassette, die das erfindungsgemäße Polynukleotid enthält, kann chimer sein, d.h. mindestens eine ihrer Komponenten ist bezogen auf mindestens eine der anderen Komponenten heterolog. Die Expression des Polynukleotids in der Expressionskassette kann unter der Kontrolle eines konstitutiven Promotors, eines induzierbaren Promotors, eines regulierten Promotors, eines viralen Promotors oder eines synthetischen Promotors stehen.

Die Vektoren können bereits Regulatorelemente enthalten, z. B. Promotoren, oder die erfindungsgemäßen DNA-Sequenzen können so manipuliert werden, dass sie solche Elemente enthalten. Geeignete Promotorelemente, die verwendet werden können, sind auf dem Fachgebiet bekannt und sind beispielsweise für *Trichoderma reesei* der cbh 1- oder der cbh-2-Promotor, für *Aspergillus ory*zae der amy-Promotor, für *Aspergillus niger* der xyl-, glaA-, alcA-, aphA-, tpiA-, gpdA-, sucl- und der pkiA-Promotor. Geeignete Promotorelemente, die zur Expression in Hefe verwendet werden können, sind auf dem Fachgebiet bekannt und sind beispielsweise der pho5-Promotor oder der gap-Promotor zur Expression in *Saccharomyces cerevisiae* und für *Pichia pastoris,* z. B. aoxl-Promotor oder der fmd-Promotor oder der mox-Promotor für *H. polymorpha.*

DNA, die zur Einschleusung in Zellen geeignet ist, kann neben der erfindungsgemäßen DNA auch DNA, die aus einer beliebigen Quelle abgeleitet wurde oder daraus isoliert ist, umfassen. Ein Beispiel für eine abgeleitete DNA ist eine DNA-Sequenz, die als nützliches Fragment in einem gegebenen Organismus identifiziert wurde und die dann in im Wesentlichen reiner Form chemisch synthetisiert wurde. Ein Beispiel für eine solche DNA ist eine geeignete DNA-Sequenz, die beispielsweise unter Verwendung von Restriktionsendonucleasen erhalten wurde, so dass sie weiter erfindungsgemäß manipuliert werden kann, beispielsweise amplifiziert werden kann. Eine derartige DNA wird üblicherweise als rekombinante DNA bezeichnet. Daher umfasst eine geeignete DNA vollständig synthetische DNA, semisynthetische DNA, aus biologischen Quellen isolierte DNA und von eingeschleuster RNA abgeleitete DNA. Im Allgemeinen ist die eingeschleuste DNA kein ursprünglicher Bestandteil des Genotyps der Empfänger-DNA, aber erfindungsgemäß kann auch ein Gen aus einem gegebenen Genotyp isoliert und eventuell verändert werden und anschließend können Mehrfachkopien des Gens in den gleichen Genotyp eingeschleust werden, z. B. um die Produktion eines gegebenen Genprodukts zu verstärken.

Die eingeschleuste DNA umfasst ohne Beschränkung DNA aus Genen, wie beispielsweise aus Bakterien, Hefen, Pilzen oder Viren. Die eingeschleuste DNA kann modifizierte oder synthetische Gene, Teile von Genen oder chimäre Gene einschließlich Genen aus dem gleichen oder einem unterschiedlichen Genotyp umfassen.

Die zur Transformation erfindungsgemäß verwendete DNA kann zirkulär oder linear, doppelsträngig oder einzelsträngig sein. Im Allgemeinen liegt die DNA in Form einer chimären DNA wie eine Plasmid-DNA vor, die auch codierende Regionen, die von Regulatorsequenzen flankiert sind, die die Expression der in der transformierten Zelle vorhandenen rekombinanten DNA unterstützen, enthalten. Beispielsweise kann die DNA selbst einen Promotor enthalten oder daraus bestehen, der in einer Zelle aktiv ist, der von einer Quelle, die sich von der Zelle unterscheidet, abgeleitet ist oder es kann ein Promotor verwendet werden, der bereits in der Zelle, d.h. der Transformationszielzelle, vorhanden ist.

Im Allgemeinen ist die eingeschleuste DNA relativ klein, weniger als etwa 30 kb, um die Empfindlichkeit gegenüber physikalischem, chemischem oder enzymatischem Abbau zu minimieren, der mit der Größe der DNA zunimmt.

Die Selektion eines geeigneten Expressionsvektors hängt von den Wirtszellen ab. Hefe- oder Pilzexpressionsvektoren können einen Replikationsorigin, einen geeigneten Promotor und Enhancer umfassen, und auch beliebige notwendige Ribosomenbindungsstellen, Polyadenylierungsstellen, Splice-Donor und -Akzeptor-Stellen, Transkriptionsterminationssequenzen und nicht-transkribierte 5'-flankierende Sequenzen umfassen.

Beispiele für geeignete Wirtszellen sind: Pilzzellen der Gattung *Aspergillus, Rhizopus, Trichoderma, Neurospora, Mucor, Penicillium,* etc., wie beispielsweise Hefen der Gattungen *Kluyveromyces, Saccharomyces, Schizosaccharomyces, Trichosporon, Schwanniomyces, Hansenula, Pichia* und dergleichen. Geeignete Wirtssysteme sind beispielsweise Pilze wie *Aspergilli,* z. B. *Aspergillus niger* (ATCC 9142) oder *Aspergillus ficuum* (NRLL 3135) oder *Trichoderma* (z. B. *Trichoderma reseei* QM6a) und Hefen wie *Saccharomyces,* z. B. *Saccharomyces cerevisiae* oder *Pichia,* wie z. B. *Pichia pastoris* oder *Hansenula,* z. B. *H. polymorpha* (DSMZ 70277). Derartige Mikroorganismen können von anerkannten Hinterlegungsstellen, z. B. der American Type Culture Collection (ATCC), dem Centraalbureau voor Schimmelcultures (CBS) oder der Deutschen Sammlung für Mikroorganismen und Zellkulturen GmbH (DSMZ) oder beliebigen anderen Hinterlegungsstellen erhalten werden.

Die Expressionskassette kann in der 5'-3'-Transkriptionsrichtung eine Transkriptions- und Translationstartregion des erfindungsgemäßen Polynucleotids und eine Transkriptions- und Terminationsregion, die in vivo oder in vitro funktionell ist, enthalten. Die Terminationsregion kann bezüglich der Transkriptionsinitiationsregion nativ sein oder kann bezüglich des Polynucleotids nativ oder anderer Herkunft sein. Die Regulatorsequenzen können stromaufwärts (5' nichtcodierende Sequenzen), innerhalb (Intron) oder stromabwärts (3' nichtcodierende Sequenzen) einer codierenden Sequenz lokalisiert sein und die Transkription, das RNA-Processing oder die Stabilität und/oder die Translation der assoziierten codierenden Sequenz beeinflussen. Regulatorsequenzen können ohne Beschränkung Enhancer, Promotoren, Repressorbindungsstellen, Translationsleadersequenzen, Introns oder Polyadenylierungsignalsequenzen umfassen. Sie können natürliche und synthetische Sequenzen sowie Sequenzen umfassen, die eine Kombination aus synthetischen und natürlichen Sequenzen sind.

Der erfindungsgemäß verwendete Vektor kann auch geeignete Sequenzen zur Amplifikation der Expression umfassen.

Beispiele für Promotoren, die erfindungsgemäß verwendet werden können, sind Promotoren, von denen bekannt ist, dass sie die Expression in den eukaryotischen Zellen kontrollieren. Beliebige Promotoren mit der Fähigkeit zur Expression in Fadenpilzen können verwendet werden. Beispiele sind ein Promotor, der stark durch Stärke oder Zellulose induziert wird, z. B. ein Promotor für Glucoamylase oder α-Amylase aus der Gattung *Aspergillus* oder Cellulase (Cellobiohydrolase) aus der Gattung *Trichoderma,* ein Promotor für Enzyme im glykolytischen Stoffwechselweg, wie beispielsweise Phosphoglyceratkinase (PGK) und Glycerinaldehyd-3-phosphat-dehydrogenase (GPD), etc. Bevorzugt ist der CelIobiohydrolase-I-, der Cellobiohydrolase-II-, der Amylase-, der Glucoamylase-, der Xylanase- oder der Enolase-Promotor.

Zwei Hauptmethoden zur Kontrolle der Expression sind bekannt, d.h. Überexpression und Unterexpression. Überexpression kann durch Insertion einer oder mehrerer Extra-Kopien des ausgewählten Gens erzielt werden. Für die Unterexpression gibt es zwei Hauptmethoden, die üblicherweise auf dem Fachgebiet als "Antisense Downregulation" und "Sense Downregulation" bezeichnet werden. Im Allgemeinen werden diese Verfahren als "Gene Silencing" bezeichnet. Beide dieser Methoden führen zu einer Hemmung der Expression des Targetgens.

Zusätzlich zur Verwendung eines speziellen Promotors können andere Typen von Elementen die Expression von Transgenen beeinflussen. Insbesondere wurde gezeigt, dass Introns das Potenzial zur Verstärkung der Transgenexpression besitzen.

Die Expressionskassette kann noch weitere Elemente umfassen, beispielsweise solche, die durch endogene oder exogene Elemente wie Zinkfinger-Proteine, einschließlich natürlich vorkommender Zinkfinger-Proteine oder chimärer Zinkfinger-Proteine, reguliert werden können.

Die erfindungsgemäß verwendete Expressionskassette kann ferner Enhancer-Elemente oder stromaufwärtige Promotor-Elemente enthalten.

Vektoren zur erfindungsgemäßen Verwendung können so konstruiert werden, dass sie ein Enhancer-Element enthalten. Die erfindungsgemäßen Konstrukte umfassen somit das Gen von Interesse zusammen mit einer 3'-DNA-Sequenz, die als Signal wirkt, um die Transkription zu terminieren und die Polyadenylierung der so erhaltenen mRNA zu erlauben. Es können beliebige Signalsequenzen verwendet werden, die die Sekretion aus dem gewählten Wirtsorganismus ermöglichen. Bevorzugte Signalsequenz ist die Phytase-Signalsequenz aus *Aspergillus niger* oder daraus abgeleitete Signalsequenzen für die Sekretion aus filamentösen Pilzen.

Es kann auch eine spezielle Leadersequenz verwendet werden, da die DNA-Sequenz zwischen der Transkriptionsstartstelle und dem Start der codierenden Sequenz, d.h. der nicht-translatierten Leadersequenz die Genexpression beeinflussen kann. Bevorzugte Leadersequenzen umfassen Sequenzen, die die optimale Expression des angehefteten Gens steuern, d.h sie umfassen eine bevorzugte Consensus-Leader-Sequenz, die die mRNA-Stabilität erhöht oder erhält und eine ungeeignete Translationsinitiation verhindert. Die Wahl solcher Sequenzen ist einem Fachmann auf dem Gebiet gut bekannt.

Um die Möglichkeit, die Transformanten zu identifizieren, zu verbessern, kann ein selektierbares oder screenbares Markergen in die Expressionskassette aufgenommen werden. Derartige Markergene sind einem Fachmann auf dem Gebiet gut bekannt.

Die Expressionskassette oder ein Vektorkonstrukt, das die Expressionskassette enthält, wird in eine Wirtszelle eingeführt. Eine Vielzahl von Techniken sind verfügbar und einem Fachmann auf dem Gebiet zur Einschleusung von Konstrukten in eine Wirtszelle gut bekannt. Die Transformation mikrobieller Zellen kann unter Verwendung von Polyethylenglykol, Calciumchlorid, viraler Infektion, DEAE-Dextran, Phageninfektionen, Elektroporation und anderen auf dem Fachgebiet bekannten Methoden durchgeführt werden. Die Transformation von Pilzen kann nach Penttilä et al., Gene 61:155-164, 1987 durchgeführt werden. Die Einschleusung eines rekombinanten Vektors in Hefen kann nach an sich bekannten Verfahren einschließlich Elektroporation, Verwendung von Spheroplasten, Lithiumacetat und dergleichen durchgeführt werden.

Sobald die erfindungsgemäße Expressionskassette bzw. DNA-Sequenz erhalten ist, kann sie in Vektoren nach an sich bekannten Verfahren eingefügt werden, um das codierte Polypeptid in geeigneten Wirtssystemen zu überexprimieren. Jedoch können auch DNA-Sequenzen als solche verwendet werden, um geeignete Wirtssysteme der Erfindung zu transformieren, um eine Überexpression des codierten Polypeptids zu erzielen.

Sobald eine erfindungsgemäße DNA-Sequenz in einer geeigneten Wirtszelle in einem geeigneten Medium exprimiert wurde, kann die codierte Phytase entweder aus dem Medium, falls die Phytase in das Medium sezerniert wird, oder aus dem Wirtsorganismus, falls die Phytase intrazellulär z. B. im periplasmatischen Raum vorhanden ist, nach an sich bekannten Verfahren aufkonzentriert und/oder isoliert werden. Bekannte Verfahren der Abtrennung der unlöslichen Bestandteile des Kulturmediums und der Biomasse gefolgt von Verfahren zur Aufkonzentrierung der Phytase können zur Herstellung von konzentrierten Phytaselösungen oder als Vorbereitung zur Trocknung der Phytase angewendet werden. Beispielsweise können Filtrationsverfahren oder Zentrifugationsverfahren zur Abtrennung der unlöslichen Bestandteile verwendet werden gefolgt von Ultrafiltrationsverfahren zur Aufkonzentration, oder es werden Cross-flow-Filtrationsverfahren angewendet. Die Trocknung kann durch Gefrier- und Sprühtrocknen, Granulationsverfahren, Extrudierung oder andere Verfahren erfolgen. Bekannte Verfahren der Proteinreinigung können verwendet werden, um die erfindungsgemäßen Phytasen zu isolieren. Beispielsweise können verschiedene chromatographische oder gelchromatographische Verfahren einzeln oder in Kombination verwendet werden. In Abhängigkeit von der verwendeten Wirtszelle bei einem rekombinanten Produktionsverfahren kann das erfindungsgemäße Enzym kovalent durch Glykosylierung modifiziert sein oder nicht. In eukaryotischen Zellen dient die Glykosylierung der sezernierten Proteine dazu, die Proteinfaltung, die Konformationsstabilität, die thermische Stabilität und die Resistenz gegenüber Proteolyse zu modulieren. Im Hinblick auf eine spezifische Anwendung der Phytase kann eine glykosylierte Variante des Enzyms gegenüber einer nicht-glykosilierten Variante bevorzugt sein. Beispielsweise dient die Verwendung einer glykosylierten Phytase in Tierfutter zum Schutz des Enzyms vor thermischer Denaturierung während der Futterpelletisierung und vor proteolytischer Inaktivierung bei der Passage durch den Magen des Tieres, wodurch die Verteilung des aktiven Enzyms im Darmtrakt und an die Wirkstelle gefördert wird. Für Anwendungen in der Nahrungsmittelverarbeitung, bei der die Enzymaktivität nur während der Verarbeitung und nicht im Endprodukt erwünscht ist, kann eine thermolabile, d.h. nicht-glykosylierte, und gegenüber protolytischem Abbau empfindlichere Phytase bevorzugt sein.

Die Erfindung betrifft auch Phytasezusammensetzungen, die das erfindungsgemäße Polypeptid enthalten. Im Allgemeinen sind Phytasezusammensetzungen flüssig oder trocken. Flüssige Zusammensetzungen enthalten das Phytaseenzym bevorzugt in einer gereinigten oder angereicherten Form. Jedoch können auch Hilfsstoffe wie beispielsweise ein Stabilisator wie Glycerin, Sorbit oder Monopropylenglykol, Additive wie Salze, Zucker, Konservierungsstoffe, Mittel zur Einstellung des pH-Werts, Proteine und Phytat oder Salze von Myoinositolphosphaten (ein Phytasesubstrat) zugesetzt werden. Typische Flüssigzusammensetzungen sind wässrige oder ölige Aufschlämmungen. Die Flüssigzusammensetzungen können einem Nahrungsmittel oder Futtermittel vor oder nach einer möglichen Pelletierung bzw. Verarbeitungsschritt zugesetzt werden.

Trockenzusammensetzungen können gefriergetrocknete, sprühgetrocknete, granulierte oder extrudierte Zusammensetzungen sein, die ausschließlich das Enzym enthalten können. Vor dem Trocknen können aber auch Stoffe zugesetzt werden um den pH-Wert zu regulieren, sowie weitere Additive und Füllstoffe wie Maltodextrine, Lactose, Magermilchpulver, Salze zweiwertiger Kationen wie die von Mg, Ca, Zn, etc.. Trockenzusammensetzungen können Granulate sein, die leicht beispielsweise mit Nahrungsmitteln oder Futterkomponenten gemischt werden können oder bevorzugter eine Komponente eines Prämix bilden. Die Teilchengröße des Enzymgranulats ist bevorzugt mit der der anderen Komponenten des Gemisches kompatibel. Dies ermöglicht sichere und zweckdienliche Mittel, um Enzyme beispielsweise in prozessierte Nahrungsmittel, Prämixe oder Tierfutter einzuarbeiten.

Beispielsweise kann eine stabile Formulierung des erfindungsgemäßen Phytaseenzyms dadurch hergestellt werden, dass ein Gemisch aus einer flüssigen Enzymlösung auf ein Füllmittel wie Maltodextrin gesprüht wird und dann das Gemisch getrocknet wird, oder der flüssigen Enzymlösung wird vor dem Trocknen 20%-60% Magermilchpulver (w/w bezogen auf Gesamtprotein der Enzymlösung) und/oder 1%-5% Calciumpropionat (w/w bezogen auf Gesamtprotein der Enzymlösung) zugesetzt und der pH-Wert auf einen definierten Wert eingestellt, insbesondere auf pH 5,2 ± 0,5. Die Verringerung der Feuchtigkeit und die Bindungswechselwirkungen der Phytase mit dem Füllmittel schützen das Enzym zusätzlich zu seiner in seiner Struktur definierten Stabilität vor Umwelteinflüssen wie Temperaturextrema, die während der Herstellung des Futtermittels auftreten können. Trockene und flüssige Formulierungen können weiter stabilisiert werden, wenn die Aktivität potenzieller proteolytischer Enzyme verringert wird, die als Nebenprodukte in dem Flüssigfermentationsgemisch vorhanden sein können, das zur Herstellung des erfindungsgemäßen Enzyms verwendet wird. Das so erhaltene Trockenenzym-Gemisch kann als Futterergänzungsmittel zur Verwendung in der Geflügel- und Schweinezucht verwendet werden. Ferner führt eine Verringerung der Phosphat-Supplementation zu einer Abnahme der Phosphat-Verunreinigung, die ihrerseits signifikant die Umweltbelastung durch intensive Tierzucht verringert.

Sobald ein Trockenenzym-Präparat erhalten ist, kann daraus in weiteren Schritten ein Agglomerations-Granulat hergestellt werden. Hierfür wird ein Mischer mit hohen Scherkräften verwendet, wodurch Füllmaterial und das Enzym coagglomerieren und ein Granulat gebildet wird. Absorptionsgranulate werden hergestellt, indem Kerne aus einem Trägermaterial durch das erfindungsgemä-ße Enzym beschichtet werden. Typische Füllmaterialien sind Salze wie Dinatriumsulfat. Andere Füllmaterialien umfassen Kaolin, Talk, Magnesiumaluminiumsilicat und Cellulosefasern. Gegebenenfalls werden Bindemittel wie Dextrine auch in das Agglomerationsgranulat aufgenommen.

Typische Trägermaterialien umfassen Stärke, z. B. in Form von Cassava, Kartoffel und Getreide, insbesondere Mais, Reis und Weizen, oder proteinhaltige Erzeugnisse wie z. B. Sojaprotein. Salze können auch verwendet werden. Das Granulat wird gegebenenfalls mit einem Beschichtungsgemisch umhüllt. Ein solches Gemisch umfasst Beschichtungsmittel, bevorzugt hydrophobe Beschichtungsmittel, dehydriertes Palmöl und Talk und gegebenenfalls andere Additive wie Calciumcarbonat oder Kaolin um die Bioverfügbarkeit an der bestimmungsgemäßen Wirkstätte zu verbessern.

Zusätzlich können die Mischungen mit Phytase andere Stoffe wie farbgebende Mittel, Aromaverbindungen, Stabilisatoren, Vitamine, Mineralien, andere Nahrungsmittel- oder Futter-Enzyme und dergleichen enthalten. Dies trifft insbesondere für die sogenannten Prämixe zu.

Ein Nahrungsmittel- oder Futtermitteladditiv ist eine im Wesentlichen reine Verbindung oder eine Zusammensetzung aus mehreren Verbindungen, die dazu bestimmt oder geeignet ist, Nahrungsmitteln oder Futtermitteln zugesetzt zu werden. Insbesondere ist es eine Substanz, die gemäß ihrem Verwendungszweck eine Komponente eines Nahrungsmittels oder Futtermittels wird oder Eigenschaften eines Nahrungsmittel- oder Futtermittelprodukts beeinflusst. So soll ein Phytaseadditiv eine Phytase bezeichnen, die kein natürlicher Bestandteil der hauptsächlich für Nahrungsmittel und Futtermittel verwendeten Substanzen ist oder in ihrer natürlichen Konzentration darin nicht vorhanden ist. Beispielsweise wird die Phytase dem Futtermittel separat von den Futtermittelsubstanzen allein oder in Kombination mit anderen Futtermitteladditiven zugesetzt. Ein typischer Prämix umfasst üblicherweise eine oder mehrere Verbindungen wie Vitamine, Mineralien oder Futter-verstärkende Enzyme und geeignete Träger und/oder Exzipienzien.

Ein gebrauchsfertiges Phytaseadditiv ist ein Additiv, das nicht in situ in Tierfutter oder in verarbeiteten Nahrungsmitteln produziert wird. Ein gebrauchsfertiges Phytaseadditiv kann Menschen oder Tieren direkt oder bevorzugt direkt nach dem Mischen mit anderen Bestandteilen des Futters oder der Nahrungsmittel verabreicht werden. Beispielsweise wird ein Futteradditiv gemäß diesem Aspekt der vorliegenden Erfindung mit anderen Futtermitteln und -additiven unter Erhalt eines Prämixes oder Ergänzungsfutters vereinigt. Solche anderen Futtermittelbestandteile umfassen eine oder mehrere andere (bevorzugt thermostabile) Enzymsupplemente, andere Futteradditive, mineralische Futtermittel und Aminosäuren. Die so erhaltenen (kombinierten) Futteradditive können mehrere verschiedene Verbindungstypen umfassen und können dann in ihrer geeigneten Menge mit den Futtermitteln wie Getreide- und Proteinträgern unter Bildung eines zusammengesetzten Tierfutters vermischt werden. Die Verarbeitung dieser Komponenten zu einem Tierfutter kann nach dem Mischen mit an sich bekannten Verarbeitungsvorrichtungen wie einer Doppelpelletiermaschine, eines Dampfpelletierers, eines Expanders oder eines Extruders durchgeführt werden.

Auf ähnliche Weise kann ein Nahrungsmitteladditiv gemäß dieser Ausführungsform der vorliegenden Erfindung mit anderen Nahrungsmittelkomponenten vereinigt werden, wodurch verarbeitete Nahrungsmittelprodukte erzeugt werden. Solche anderen Nahrungsmittelkomponenten umfassen ein oder mehrere Enzymsupplemente, Vitamine, Mineralien und Spurenelemente. Das so erhaltene kombinierte Nahrungsergänzungsmittel kann dann in einer geeigneten Menge mit anderen Nahrungsmittel-Komponenten wie Getreide und Pflanzenproteine vermischt werden, um ein verarbeitetes Nahrungsmittel zu ergeben. Die Verarbeitung dieser Komponenten zu einem verarbeiteten Nahrungsmittel kann unter Verwendung an sich bekannter Verarbeitungsvorrichtungen durchgeführt werden.

In einer bevorzugten Ausführungsform umfassen die erfindungsgemäßen Phytasezusammensetzungen zusätzlich eine wirksame Menge eines oder mehrerer Enzyme für Nahrungs- oder Futtermittel, bevorzugt ausgewählt aus alpha-Galactosidasen, beta-Galactosidasen, Laccasen, anderen Phytasen, Phosphatasen, Endoglucanasen, insbesondere endo-beta-1,4-Glucanasen, endo-beta-1,3(4)-Glucanasen, endo-1,2-beta-Glucanasen und endo-1,3-alpha-Glucanasen, Cellulasen, Xylosidasen, Galactanasen, insbesondere Arabinogalactan-endo-1,4-beta-Galactosidasen und Arabinogalactan-endo-1,3-beta-Galactosidasen, Pectin-abbauenden Enzymen, insbesondere Pectinasen, Pectinesterasen, Pectinlyasen, Polygalacturonasen, Arabananasen, Rhamnogalacturonasen, Rhamnogalacturonanacetylesterasen, Rhamnogalacturonan-alpha-Rhamnosidasen, Pectatlyasen und alpha-Galacturonidasen, Mannanasen, beta-Mannosidasen, Mannanacetylesterasen, Xylanacetylesterasen, Proteasen, Xylanasen, Arabinoxylanasen und lipolytischen Enzymen wie Lipasen, Phospholipasen und Cutinasen.

Das erfindungsgemäße Tierfutteradditiv wird dem Tier vor oder gleichzeitig mit dem Futter verabreicht. Bevorzugt wird das erfindungsgemäße Tierfutteradditiv dem Tier gleichzeitig mit dem Futter verabreicht.

Eine effektive Menge an Phytase in Nahrungsmitteln oder Futtermitteln beträgt etwa 10-20.000 PPU/kg, bevorzugt etwa 10-15.000 PPU/kg, bevorzugter etwa 10-10.000 PPU/kg, noch bevorzugter etwa 50-5.000 PPU/kg, insbesondere 50-2.000 PPU/kg Futter oder Nahrungsmittel.

Die Erfindung betrifft auch die Verwendung von Phytase zur Verarbeitung und Herstellung von Nahrungsmitteln und Futtermitteln. Getreide und Mehle für Nahrungsmittel können enzymatisch mit Phytase behandelt werden, um den Phytingehalt der Rohstoffe zu verringern. Verringerte Phytingehalte verbessern die Nahrungsmittelqualität, indem die Verfügbarkeit essenzieller Mineralien wie Eisen, Calcium und Zink erhöht wird. Zusätzlich zur Erhöhung der Qualität der Nahrungsmittel kann die Verwendung von Phytase während der Verarbeitung die Gesamteffizienz der Nahrungsmittelherstellung verbessern. Beispielsweise kann die Zugabe von Phytase zu weißen Sojabohnenflocken während der Herstellung eines Sojaproteinisolats signifikant die Ausbeute und Qualität des extrahierbaren Proteins erhöhen. Dabei ist die Phytase nur während der Herstellung und Verarbeitung aktiv und ist in dem Endprodukt nicht mehr aktiv. Dieser Aspekt ist insbesondere bei der Teigherstellung und beim Backen und der Produktion anderer verzehrfertiger Produkte auf Getreidebasis von Bedeutung. Auf ähnliche Weise können Tierfutterkomponenten wie getoastetes Sojabohnenmehl oder Canolamehl mit Phytase vor dem eigentlichen Herstellungsprozess vorbehandelt werden. Die Entfernung von anti-nutritiven Faktoren in Tierfutterkomponenten vor der Herstellung führt zu einer physiologisch höheren Qualität und zu wertvolleren Tierfutterinhaltsstoffen. Bei diesen Verarbeitungsverfahren ist die Phytase während der Herstellung aktiv und ist im Verdauungstrakt des Tiers nach Aufnahme des behandelten Futters in der Regel nicht mehr aktiv.

Zusätzlich zur Verwendung von Phytase als Futterverarbeitungshilfsmittel betrifft die vorliegende Erfindung die Verwendung der erfindungsgemäßen Phytase als Verdauungshilfe. Phytase in Tablettenform kann zusammen mit der Nahrungsaufnahme eingenommen werden, um das aktive Enzym im Magen-Darm-Trakt zu verteilen.

Die erfindungsgemäße Phytase kann ebenso in vorteilhafter Weise bei monogastrischen wie polygastrischen Tieren, insbesondere bei jungen Kälbern zum Einsatz kommen. Futter für Fische und Schalentiere kann ebenfalls mit Phytase supplementiert werden, um die Ausnutzung des Futters zu verbessern und den Gehalt an ausgeschiedenem Phosphor bei intensiver Tierzucht zu verringern. Das erfindungsgemäße Futtermittel kann auch Tieren wie Geflügel, z. B. Masthühnern, Truthühnern, Gänsen, Enten sowie Schweinen, Pferden, Rindern, Schafen, Ziegen, Hunden und Katzen sowie Fischen und Schalentieren verabreicht werden. Es ist jedoch besonders bevorzugt, dass das erfindungsgemäße Futtermittel Schweinen oder Geflügel verabreicht wird.

Erfindungsgemäße Phytaseformulierungen können auch mit anderen Inhaltsstoffen kombiniert werden, wodurch neue und besonders vorteilhafte Futterzusammensetzungen entstehen. Da, wie vorstehend ausgeführt, die Verfügbarkeit von pflanzlichem Phosphat in Sojabohnenmehl und Getreide in Folge der Bindung an Phytinsäure niedrig ist, wird anorganisches Phosphat dem Futter zugesetzt, um eine adäquate Phosphorversorgung der Tiere zu gewährleisten. Jedoch enthalten diese Futtermittel zu viel Gesamtphosphat und führen dadurch zu einer Verschmutzung der Umwelt mit Phosphat. Speziell umfasst das erfindungsgemäße Tierfuttermittel die Kombination einer erfindungsgemäßen Phytase mit Tierfutterinhaltsstoffen, um ein Futtermittel zu ergeben, das wesentlich erniedrigte Gehalte an zugesetztem anorganischem Phosphor enthält. In einer bevorzugten Ausführungsform umfasst das erfindungsgemäße Futtermittel typische Futterinhaltsstoffe, Mikronährstoffe, Spurenelemente, Vitamine, etc. sowie eine effektive Menge an Phytase und anorganischem Phosphor, wobei die Mengen der Phytase und des Phosphors zwischen 50 und 20.000 Einheiten Phytase/kg Futtermittel und weniger als 0,45% anorganischem Phosphor, bevorzugt zwischen Gehalten von 100-10.000 Einheiten Phytase/kg Futtermittel und weniger als 0,225% anorganischem Phosphor, bevorzugter Gehalte von 150-10.000 Einheiten Phytase/kg Futtermittel und weniger als 0,15% anorganischem Phosphor, noch bevorzugter Gehalte von 200-20.000 Einheiten Phytase/kg Futtermittel und keinen zusätzlichen Zusatz an anorganischem Phosphor, betragen.

Die Erfindung betrifft auch Verfahren zur Verbesserung der Gewichtszunahme und des Futterverwertungsvermögens (Feed Conversion Ratio, FCR) in der Tierernährung sowie die Verwendung der erfindungsgemäßen Phytasen in diesen Verfahren. Eine erfindungsgemäße Phytase erlaubt verbesserte Gewichtszunahmen und ein verbessertes Futterverwertungsvermögen, insbesondere im Zusammenhang mit Futter, das wenig anorganisches Phosphat enthält. Gemäß den erfindungsgemäßen Verfahren kann der Gehalt an anorganischem Phosphat in Futter unter Gehalte von 0,45%, bevorzugt unter 0,225%, gesenkt werden. Bevorzugt wird kein anorganisches Phosphat zugesetzt. Durch eine erhöhte Phosphat-Verfügbarkeit in Folge des Zusatzes des erfindungsgemäßen Enzyms kann die Knochenmineralisation der Tiere signifikant verbessert werden, was insbesondere bei rasch wachsenden Tieren von großer Bedeutung ist.

Gemäß einer noch weiteren Ausführungsform betrifft die Erfindung die Verwendung des erfindungsgemäßen Enzyms beim Backen, wodurch Entwicklung, Elastizität und/oder Stabilität des Teigs und/oder das Volumen, die Krumenstruktur und/oder der Widerstand des Backguts gegenüber dem Altbackenwerden verbessert wird. Obwohl das erfindungsgemäße Enzympräparat zur Herstellung von Teig oder gebackenen Produkten aus beliebigen Typen von Mehl, z. B. auf der Basis von Roggen, Gerste, Hafer oder Mais verwendet werden kann, erwies sich das erfindungsgemäße Enzympräparat als besonders nützlich bei der Herstellung von Teigen oder Backprodukten aus Weizen oder aus einem wesentlichen Weizenanteil. Die Backprodukte, die mit einem erfindungsgemäßen Enzympräparat hergestellt werden können, umfassen Brot, Brötchen, Baguette und dergleichen. Zum Backen kann das erfindungsgemäße Enzympräparat mit einer weiteren Enzymaktivität wie z. B. Xylanase, Lipase, Amylase, Oxidase oder Laccase neben der Phytase verwendet werden oder kann in Kombinationen mit weiteren Enzymen wie Lipase, Amylase, Oxidase (z. B. Glucoseoxidase, Peroxidase), verwendet werden.

Des weiteren sind folgende Gegenstände beschrieben:
1. Rekombinantes DNA-Molekül, das nach Expression in einer prokaryotischen oder eukaryotischen Wirtszelle ein Polypeptid mit Phytaseaktivität codiert, wobei das rekombinante DNA-Molekül eine DNA-Sequenz, ausgewählt aus
   a) DNA-Sequenzen, die ein Polypeptid mit Phytaseaktivität codieren, das durch Variation der reifen Wildtyp-*E. coli*-Phytasesequenz erhalten worden ist, wobei die Variation ausgewählt ist aus
      i) der Mutation Lysin → Asparaginsäure an Position 74 (K74D), und/oder
      ii) einer Kombination der Mutationen Asparagin → Arginin an Position 139 (N139R) und Asparaginsäure → Glutaminsäure an Position 142 (D142E), und/oder
      iii) einer Kombination der Mutationen Leucin → Isoleucin an Position 145 (L145I) und Leucin → Isoleucin an Position 198 (L198I), und/oder
      iv) einer Mutation Valin → Prolin an Position 200 (V200P), und/oder
      v) einer N- oder C-terminalen oder einer N- und C-terminalen Anfügung eines Sequenzabschnitts der sauren Phosphatase von *Aspergillus niger* var. *awamori* oder der Phytase von *Aspergillus niger,*
   b) DNA-Sequenzen, die eine Homologie von 70% bis 100% zu den Sequenzen gemäß a) aufweisen,
   c) DNA-Sequenzen, die aufgrund der Degeneriertheit des genetischen
      Codes mit den Sequenzen gemäß a) und b) verwandt sind,
      umfasst, wobei das rekombinante DNA-Molekül bei Expression in einer geeigneten Wirtszelle mit einer erhöhten Temperatur- und Proteasestabilität der Enzymaktivität des so codierten Proteins einhergeht, wobei die Variationen iii) und iv) nur in Kombination mit einer Variation i), ii) und/oder v) vorliegen, oder gemeinsam mit einer Variation i), ii) und/oder v) vorliegen.
2. Rekombinantes DNA-Molekül nach Gegenstand 1, dadurch **gekennzeichnet**, dass die DNA-Sequenz die Variation N139R und D142E des codierten Polypeptids umfasst.
3. Rekombinantes DNA-Molekül nach Gegenstand 1, dadurch **gekennzeichnet**, dass die DNA-Sequenz mindestens die Variation V200P des codierten Polypeptids umfasst.
4. Rekombinantes DNA-Molekül nach Gegenstand 1, dadurch **gekennzeichnet**, dass die DNA-Sequenz die Variation K74D des codierten Polypeptids umfasst.
5. Rekombinantes DNA-Molekül nach Gegenstand 1, dadurch **gekennzeichnet**, dass die DNA-Sequenz mindestens die Variation L145I und L198I des codierten Polypeptids umfasst.
6. Rekombinantes DNA-Molekül nach Gegenstand 1, dadurch **gekennzeichnet,** dass die DNA-Sequenz die Variation K74D, N139R, D142E, V200P des codierten Polypeptids umfasst.
7. Rekombinantes DNA-Molekül nach Gegenstand 1, dadurch **gekennzeichnet**, dass die N-terminale Anfügung des codierten Polypeptids die Sequenz FSYGAAIPQS TQEKQFSQEF RDGYSILKHY GGNGPYSERV SYGIARDPPTS umfasst.
8. Rekombinantes DNA-Molekül nach Gegenstand 1, dadurch **gekennzeichnet**, dass die C-terminale Anfügung des codierten Polypeptids die Sequenz LSFWWNYNTT TELNYRSSPI ACQEGDAMD umfasst.
9. Rekombinantes DNA-Molekül nach Gegenstand 1, dadurch **gekennzeichnet,** dass die N-terminale Anfügung des codierten Polypeptids die Sequenz FSYGAAIPQS TQEKQFSQEF RDGYSILKHY GGNGPYSERV SYGIARDPPTS und die C-terminale Anfügung des codierten Polypeptids die Sequenz LSFWWNYNTT TELNYRSSPI ACQEGDAMD umfasst.
10. Rekombinantes DNA-Molekül nach einem der Gegenstände 1 bis 8, dadurch **gekennzeichnet**, dass es eine Sequenz ausgewählt aus den Sequenzen SEQ ID NO:9, SEQ ID NO:11, SEQ ID NO:13, SEQ ID NO:15, SEQ ID NO:17, SEQ ID NO:19, SEQ ID NO:21 umfasst.
11. Polypeptid, das Phytaseaktivität besitzt und von einem rekombinanten DNA-Molekül nach einem der Gegenstände 1 bis 10 codiert wird oder durch Expression einer damit transformierten Wirtszelle erhalten wird.
12. Polypeptid nach Gegenstand 11, dadurch **gekennzeichnet,** dass es eine Sequenz, ausgewählt aus SEQ ID NO:10, SEQ ID NO:12, SEQ ID NO:14, SEQ ID NO:16, SEQ ID NO:18, SEQ ID NO:20, SEQ ID NO:22 umfasst.
13. DNA-Konstrukt mit der Fähigkeit, nach dem Einführen in eine geeignete Wirtszelle die Expression eines mutierten Phytasegens in einem Wirt zu steuern, dadurch **gekennzeichnet**, dass es gegebenenfalls einen Promotor, gegebenenfalls Signal- und Markersequenzen, eine DNA-Sequenz nach einem der Ansprüche 1 bis 10, einen Terminator und gegebenenfalls 5'- und 3'-flankierende Sequenzen enthält.
14. DNA-Konstrukt nach Gegenstand 13, dadurch **gekennzeichnet**, dass es sich bei dem Promotor um den Cellobiohydrolase-I-, den Cellobiohydrolase-II-, den Amylase-, den Glucoamylase-, den Xylanase- oder den Enolase-Promotor handelt.
15. DNA-Konstrukt nach Gegenstand 14, dadurch **gekennzeichnet**, dass es sich bei der Signalsequenz und/oder den 5'- und 3'-flankierenden Sequenzen um gegebenenfalls modifizierte Phytase-Signalsequenzen aus *Aspergillus niger* handelt.
16. Vektor mit der Fähigkeit, eine Wirtszelle zu transformieren, dadurch **gekennzeichnet,** dass der Vektor ein Konstrukt nach Gegenstand 13 enthält.
17. Vektor nach Gegenstand 16, dadurch **gekennzeichnet**, dass er ausgewählt ist aus dem Plasmid pET-PhyM2, hinterlegt unter der Hinterlegungsnummer DSM 18715, dem Plasmid pUC-PhyM3, hinterlegt unter der Hinterlegungsnummer DSM 18717, dem Plasmid pET-PhyM7, hinterlegt unter der Hinterlegungsnummer DSM 18716, dem Plasmid pUC-PhyM9, hinterlegt unter der Hinterlegungsnummer DSM 18718, dem Plasmid pUC-PhyM10, hinterlegt unter der Hinterlegungsnummer DSM 18719, dem Plasmid pPhy2005, hinterlegt unter der Hinterlegungsnummer DSM 18720, dem Plasmid pPhy2006, hinterlegt unter der Hinterlegungsnummer DSM 18721.
18. Transformierte Wirtszelle, ausgewählt aus Pilz, Hefe, Bakterien und Säugerzellen, enthaltend ein rekombinantes DNA-Molekül nach einem der Gegenstände 1 bis 10 und ausgestattet mit der Fähigkeit, ein Polypeptid mit Phytaseaktivität zu exprimieren.
19. Transformierte Wirtszelle nach Gegenstand 18, dadurch **gekennzeichnet**, dass sie der Gattung Aspergillus, Rhizopus, Trichoderma, Neurospora, Mucor oder Penicillium angehört.
20. Verfahren zur Produktion von Phytase, dadurch **gekennzeichnet,** dass eine transformierte Wirtszelle nach Gegenstand 18 oder 19 unter Bedingungen gezüchtet wird, die der Bildung von Phytase förderlich sind und die so produzierte Phytase isoliert wird.
21. Zusammensetzung, umfassend ein Polypeptid nach Gegenstand 11 oder 12, gegebenenfalls zusammen mit weiteren Hilfs- und/oder Wirkstoffen.
22. Zusammensetzung nach Gegenstand 21, dadurch **gekennzeich**n e t , dass sie Magermilchpulver und/oder Calciumpropionat enthält.
23. Zusammensetzung nach Gegenstand 21, dadurch **gekennzeich**n e t , dass die Zusammensetzung eine Nahrungsmittel- oder Futtermittelzusammensetzung ist.
24. Zusammensetzung nach Gegenstand 21, 22 oder 23, dadurch **gekennzeichnet**, dass die Zusammensetzung ein Backmittel ist.
25. Verwendung eines Polypeptids nach Gegenstand 11 oder 12 zur Herstellung eines Präparats zur Verbesserung der Phosphatverwertung aus der Nahrung bei Tieren und Menschen.
26. Verwendung eines Polypeptids nach Gegenstand 11 oder 12 zur Verbesserung der rheologischen Eigenschaften von Teigen zur Herstellung von Backwaren.
27. Verwendung einer Variation der reifen Wildtyp-*E. coli*-Phytasesequenz, ausgewählt aus
   i) der Mutation Lysin → Asparaginsäure an Position 74 (K74D), und/oder
   ii) einer Kombination der Mutationen Asparagin → Arginin an Position 139 (N139R) und Asparaginsäure → Glutaminsäure an Position 142 (D142E), und/oder
   iii) einer Kombination der Mutationen Leucin → Isoleucin an Position 145 (L145I) und Leucin → Isoleucin an Position 198 (L198I), und/oder
   iv) einer Mutation Valin → Prolin an Position 200 (V200P), und/oder
   v) einer N- oder C-terminalen oder einer N- und C-terminalen Anfügung eines Sequenzabschnitts der sauren Phosphatase von *Aspergillus niger var. awamori* oder der Phytase von *Aspergillus niger*
      zur Herstellung eines rekombinanten DNA-Moleküls, das nach Expression in einer prokaryotischen oder eukaryotischen Wirtszelle ein Polypeptid mit Phytaseaktivität codiert, das eine erhöhte Temperatur- und Proteasestabilität besitzt.
28. Verwendung nach Gegenstand 27, dadurch **gekennzeichnet,** dass die Variation K74D ist.
29. Verwendung nach Gegenstand 27, dadurch **gekennzeichnet,** dass die Variation N139R und D142E ist.
30. Verwendung nach Gegenstand 27, dadurch **gekennzeichnet,** dass die Variation L145I und L198I ist.
31. Verwendung nach Gegenstand 27, dadurch **gekennzeichnet,** dass die Variation V200P ist.
32. Verwendung nach Gegenstand 27, dadurch **gekennzeichnet,** dass die Variation K74D, N139R, D142E, V200P ist
33. Verwendung nach Gegenstand 27, dadurch **gekennzeichnet**, dass die Variation die N-terminale Anfügung der Sequenz FSYGAAIPQS TQEKQFSQEF RDGYSILKHY GGNGPYSERV SYGIARDPPTS ist.
34. Verwendung nach Gegenstand 27, dadurch **gekennzeichnet**, dass die Variation die C-terminale Anfügung die Sequenz LSFWWNYNTT TELNYRSSPI ACQEGDAMD ist.
35. Verwendung nach Gegenstand 27, dadurch **gekennzeichnet**, dass die Variation die N-terminale Anfügung der Sequenz FSYGAAIPQS TQEKQFSQEF RDGYSILKHY GGNGPYSERV SYGIARDPPTS und die C-terminale Anfügung die Sequenz LSFWWNYNTT TELNYRSSPI ACQEGDAMD ist.

Die beigefügten Figuren zeigen:
- Fig. 1:: Plasmidkarte von pET-PhyM2
- Fig. 2:: Plasmidkarte von pAB490-PhyM2
- Fig. 3:: Plasmidkarte von pUC-PhyM3
- Fig. 4:: Plasmidkarte von pAB489-PhyM3
- Fig. 5:: Plasmidkarte von pUC-PhyM10
- Fig. 6:: Plasmidkarte von pAB600-PhyM10
- Fig. 7:: Plasmidkarte von pPhy2005
- Fig. 8:: Plasmidkarte von pAB-Phy2005
- Fig. 9:: Plasmidkarte von pPhy2006
- Fig. 10:: Plasmidkarte von pAB-Phy2006

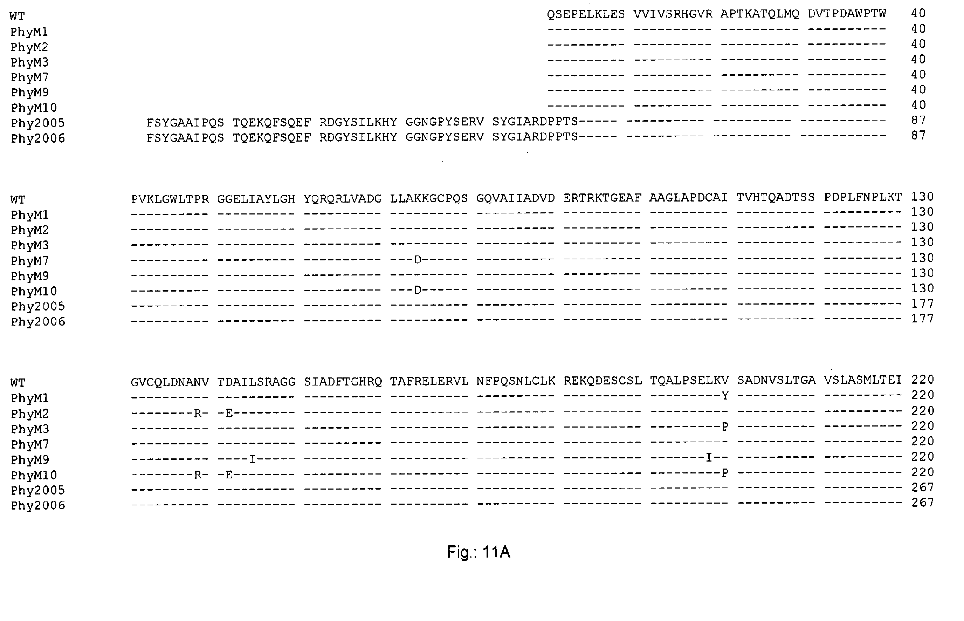

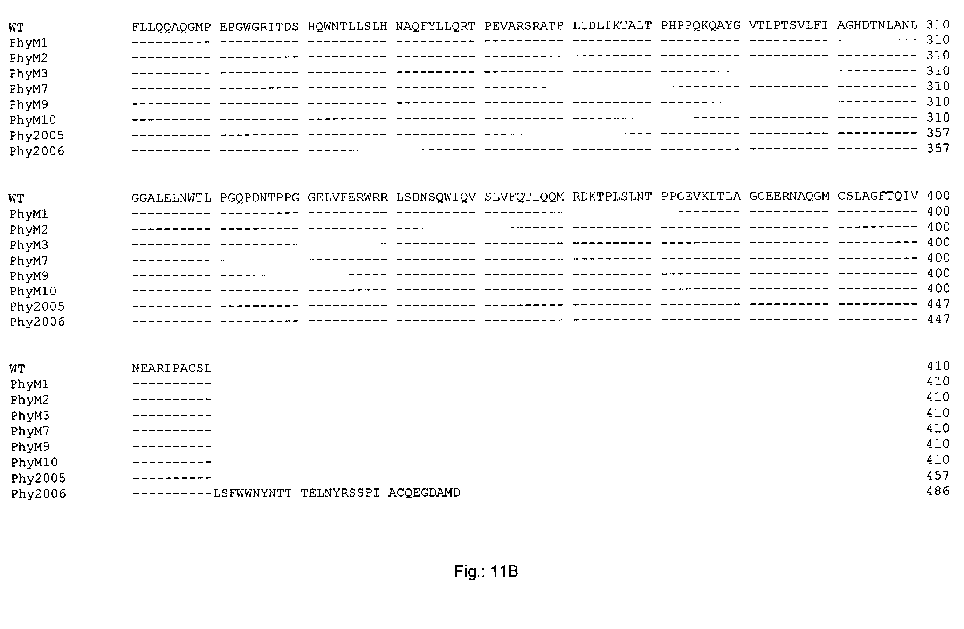

- Fig. 11:: Sequenzvergleich einzelner Mutanten und Varianten auf Basis der Wild-Typ Sequenz (Dassa). Die Mutationen bzw. Variationen sind hervorgehoben.

Die nachstehenden Beispiele erläutern die Erfindung näher.

### Beispiele

### Beispiel 1 - Bestimmung der Phytaseaktivität

Die Phytaseaktivität wurde in einem Assay-Gemisch aus 0,5% Phytinsäure (etwa 5 mM), 200 mM Natriumcitrat, pH 5,0, gemessen. Nach 15-minütiger Inkubation bei 37°C wurde die Reaktion durch Zugabe eines gleichen Volumens 15% Trichloressigsäure gestoppt. Die freigesetzten Phosphationen wurden durch Mischen von 100 µl des Assay-Gemisches mit 900 µl H₂O und 1 ml 0,6 M H₂SO₄, 2% Ascorbinsäure und 0,5% Ammoniummolybdat nach Inkubation bei 50°C und einer Dauer von 20 min, bei 820 nm quantitativ bestimmt. Kaliumphosphat-Standardlösungen wurden als Referenz verwendet.

### Beispiel 2 - Konstruktion der Plasmide pET-PhyM2 und pAB490-PhyM2 (Genotyp PhyM2)

Die Konstruktion des Plasmids pAB490-PhyM2 erfolgte durch folgende Schritte:

### 1. Konstruktion von pET-PhyM2

Das Plasmid pET-PhyM2 enthält die *E*. *coli* Phytase Sequenz (Dassa et al. 1990, Accession Nr. M58740) mit den Änderungen der Aminosäuren N139R und D142E.

Die DNA Sequenz, die mit dem CAG (Gln)-Codon in Position 1 ein offenes Leseraster von 1230 bp umfasst und ein Enzym mit 410 Aminosäuren codiert, wurde unter Verwendung des Codon-Gebrauchs von *T. reesei* (http://www.kazusa.or.jp/codon) in das Plasmid pET26b(+) (Novagen, Deutschland, modifiziert durch Brain, Deutschland) eingefügt. Zur Realisierung der Mutagenesen wurde eine PCR-basierte Oligonucleotid-gerichtete Methode angewandt. Ausgehend von dem Plasmid, das das Gen kodierend für die Wildtyp-Dassa-Aminosäuresequenz enthält, wurden für jedes auszutauschende Triplet zwei komplementäre Primer mit den jeweiligen Mutationen synthetisiert, wobei die Mutation jeweils in der Mitte der Primer lokalisiert ist. Mithilfe der beiden Primer wurde dann das gesamte Plasmid amplifiziert. Das erhaltene Plasmid mit den Mutationen für den Genotyp M2 hat die Bezeichnung pET-PhyM2.

Zur Konstruktion wurden die folgenden Primer benutzt.
a) Für die Mutationen an Position 139:
   ccaactggataacgcccgggtgaccgacgccat (SEQ ID NO:1)
   atggcgtcggtcacccgggcgttatccagttgg (SEQ ID NO:2)
b) Für die anschließend eingebaute zusätzliche Mutationen an Position 142:
   gcccgggtgaccgaggccatcctcagc (SEQ ID NO:3)
   gctgaggatggcctcggtcacccgggc (SEQ ID NO:4)

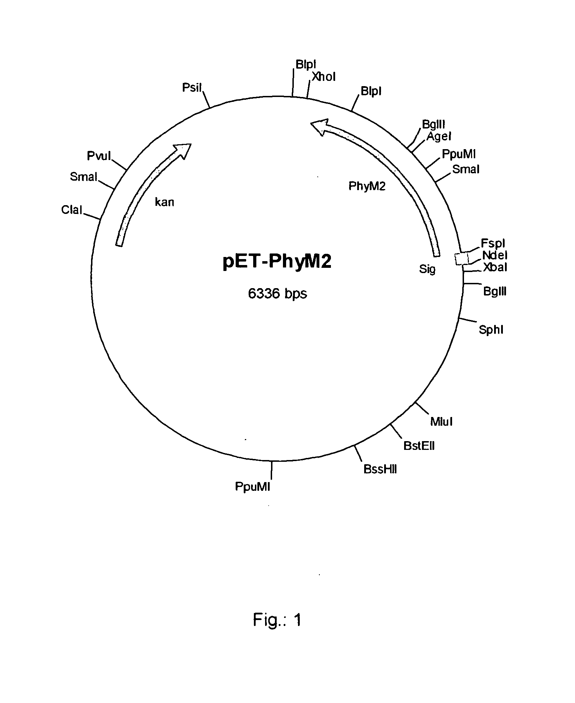

Das Plasmid ist in Fig. 1 dargestellt und wurde unter der Hinterlegungsnummer DSM 18715 am 18.10.2006 hinterlegt.

### 2. Konstruktion von pAB490-PhyM2

Zur Konstruktion des Plasmids pAB490-PhyM2 wurde das für *E. coli*-Phytase codierende Gen PhyM2 aus dem Plasmid pET-PhyM2 mittels PCR amplifiziert. Das PCR-Produkt wurde mit den Restriktionsenzymen SpeI und PacI geschnitten und in die SpeI- und PacI-Schnittstellen nach dem *T. reesei* cbhII-Genfragment in dem Plasmid pAB490 inseriert. Dadurch entstand ein offenes Leseraster, das die Fusion CBHII-KexII-PhyM2 codiert.

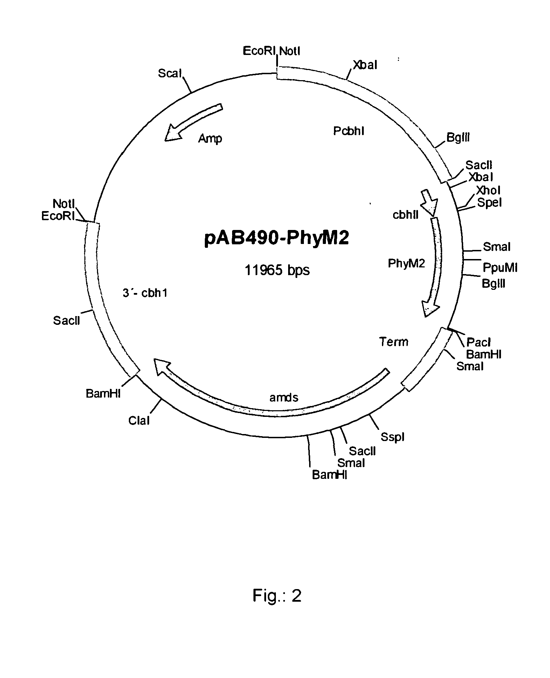

Das entstandene Plasmid hat die Bezeichnung pAB490-PhyM2 (Fig. 2) und wurde durch Restriktionsendonucleasen kartiert. Die Phytasesequenz wurde durch Sequenzierung bestätigt.

Das Plasmid pAB490 basiert auf pUC18 und enthält das cbhII Genfragment (Nucleotide 1-307 entsprechen den Aminosäuren M1 bis S86, Teeri et al, 1987, Gene 51: 43-52, Accession Nr. 16190) unter der Kontrolle des cbhl-Promotors und cbhl-Terminators aus dem Plasmid pALK487 (WO 94/28117). Ein glattendiges 4,78 kb langen EcoRI/SpeI-Fragment des Plasmids pALK424 (WO 93/24621), das den amdS Marker und die 3'-flankierenden cbhI-Sequenzen enthält, wurde in die Stul Schnittsstelle am 3'-Ende des cbhl Terminator eingebaut. Darüber hinaus wurden weitere Schnittstellen (SpeI und PacI) downstream des cbhII-Genfragments eingefügt. Diese Schnittstellen wurden für die direkte Klonierung der Phytasevarianten verwendet.

Die aus dem Plasmid pAB490-PhyM2 isolierte Expressionskassette enthält das folgende genetische Material:

cbhl (Cellobiohydrolase I) Promotor: Das 2,2 kb EcoRI/SacII-Fragment, das den cbhl-Promotor enthält, stammt von *Trichoderma reesei* QM6a. Der PromotorBereich funktioniert auch als homologe DNA (zusammen mit dem cbhI 3'-Fragment; siehe nachstehend), um die Integration der transformierenden DNA in den cbhl Lokus zu steuern.

cbhII-Genfragment: Das 307-bp cbhll Genfragment mit seiner Signalsequenz steht unmittelbar unter der Kontrolle des cbhI-Promotors.

Die *E. coli*-Phytase Sequenz, die die Mutation der Aminosäuren N139R und D142E enthält, ist mittels einer KexII-Schnittstelle am 3'-Ende des cbhII-Genfragments fusioniert. Die Kex-Sequenz enthält folgende Aminosäuren: RTLVKR.

cbhl Terminator: Das 0,75 kb lange BamHI/StuI-Fragment, das den cbhI-Terminator enthält, wurde im Anschluss an die *E. coli*-Phytase hinzugefügt, um die Termination der Transkription zu gewährleisten.

amdS-Gen: Das Gen inkl. seines Promotors und seines Terminators wurde aus *Aspergillus nidulans* VH1-TRSX6 isoliert und codiert für Acetamidase (Hynes et al., 1983, Mol. Cell. Biol. 3: 1430-1439; Kelly and Hynes, 1985, EMBO J. 4:475-479). Acetamidase erlaubt dem Stamm unter Verwendung von Acetamid als alleiniger Stickstoffquelle zu wachsen und dieses Charakteristikum wurde zur Selektion der Transformanten verwendet.

cbhI 3' Fragment: Das Fragment (1,7 kb, BamHI/EcoRI, beginnend 1,4 kb nach dem Stopcodon des Gens) wurde aus *T. reesei* ALK02466 isoliert. Der Stamm ALKO2466 stammt von dem Stamm ALKO233 (Harkki et al., 1991, Enzyme Microb. Technol. 13: 227-233) ab. Das 3'-Fragment wird zusammen mit dem Promotorbereich verwendet, um die Phytase-Expressionskassette in den cbhI-Lokus durch homologe Rekombination zielgerecht zu integrieren.

Die Sequenz des Plasmids pAB490-PhyM2 wurde durch Kartierung mittels Restriktionsenzymen und Sequenzierung bestätigt.

### Beispiel 3 - Konstruktion der Plasmide pET-PhyM7 und pAB490-PhyM7 (Genotyp PhyM7)

Das Plasmid pET-PhyM7 enthält die *E. coli-*Phytase (Dassa et al. 1990, Accession Nr. M58740) Sequenz unter Verwendung des Codon-Gebrauchs von *T*. *reesei* (http://www.kazusa.or.jp/codon) mit der Mutation K74D. Die Konstruktion sowie die Klonierung des Plasmids pET-PhyM7 erfolgte analog zu der in Beispiel 2 beschriebenen Herstellung des Plasmids pET-PhyM2. Die zur Einfügung der Mutationen an Aminosäureposition 74 benutzten Primer sind:
cggactcctggctgacaagggatgcccgc (SEQ ID NO:5)
gcgggcatcccttgtcagccaggagtccg (SEQ ID NO:6)

Das Plasmid pET-PhyM7_ist unter der Hinterlegungsnummer DSM 18716 am 18.10.2006 hinterlegt worden.

Die Konstruktion sowie die Klonierung des Plasmids pAB490-PhyM7 erfolgte analog zu der in Beispiel 2 beschriebenen Herstellung des Plasmids pAB490-PhyM2. Die Sequenz des Plasmids pAB490-PhyM7 wurde durch Sequenzierung bestätigt. Die aus dem Plasmid pAB490-PhyM7 isolierte Expressionskassette enthält daher mit Ausnahme der neuen spezifischen Phytasesequenz des Genotyps PhyM7 die gleichen Elemente wie in Beispiel 2 beschrieben.

### Beispiel 4 - Konstruktion der Plasmide pUC-PhyM3 und pAB489-PhyM3 (Genotyp PhyM3)

Die Phytasevariante PhyM3 enthält die *E*. *coli* Phytase (Dassa et al. 1990, Accession Nr. M58740) Sequenz unter Verwendung des Codon-Gebrauchs von *T*. *reesei* (http://www.kazusa.or.jp/codon) mit der Mutation V200P. Die DNA-Sequenz mit dem CAG (Gln)-Codon in Position 1 umfasst ein offenes Leseraster von 1230 bp und codiert ein Enzym mit 410 Aminosäuren. Das 18 Aminosäuren lange Signalpeptid der Phytase von *A*. *niger* wurde verwendet, um die Phytasemutante von *E*. *coli* aus *Trichoderma reesei* zu sezernieren.

Ausgehend von dem Plasmid, das das Gen kodierend für die Wildtyp-Dassa-Aminosäuresequenz enthält, wurde die Mutation V200P mittels der PCR Methode analog dem Prinzip von Tomic et al. (1990, Nucleic Acids Research, 18 (6), 1656) und Vallette et al. (1989, Nucleic Acids Research, 17 (2), 723-733) durchgeführt. Das PCR Produkt wurde mit AvrII und Pacl geschnitten und in die SpeI und PacI Spaltstellen nach dem *T*. *reesei* cbh1 Promotor in dem Plasmid pAB489 inseriert. In dem neu entstandenen Plasmid pAB489-PhyM3 steht das Gen der *E*. *coli* Phytasevariante PhyM3 mit der modifizierten *A*. *niger* Phytase Signalsequenz -MGVSAILLPLYLLSGVTS- (Mullaney et al., Appl Microbiol Biotechnol, 1991, 35(5), 611-614, Accession Nr. M94550) direkt unter der Kontrolle des cbh1-Promotors. Das 18 Aminosäuren lange Signalpeptid der Phytase von *A*. *niger* wurde verwendet, um die Phytasemutante von *E*. *coli* aus *Trichoderma reesei* zu sezernieren. Die 16 Basenpaare (CCGCGGACTGCGCATC atg) stromaufwärts des Startcodons, die zu dem *T*. *reesei*-Promotor (Shoemaker et al. 1983, Bio/Technology 1, 691-696) gehören, wurden nach dem Einbauen des AvrII-PacI Phytase Fragments in die SpeI und PacI Spaltstellen in dem Plasmid pAB489 zu CCGCGGACTAGGCATC atg geändert.

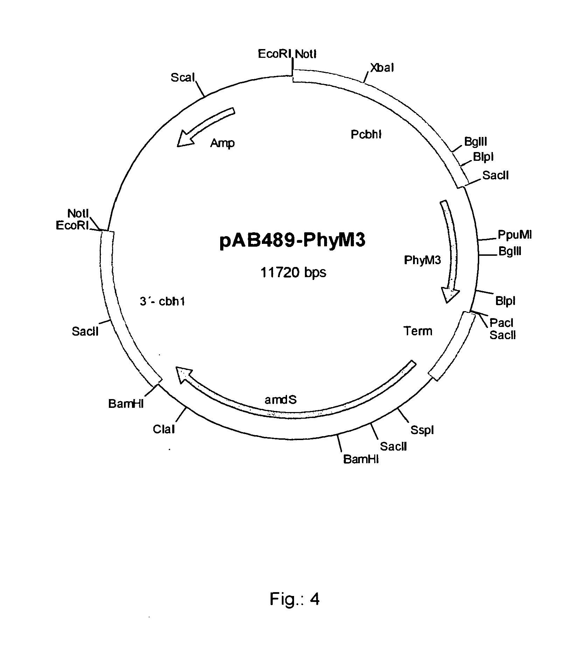

Die Konstruktion pAB489-PhyM3 (Fig. 4) wurde durch Kartierung und Sequenzierung bestätigt.

Die Konstruktion des Plasmids pAB489 erfolgte durch folgende Schritte:

Aus dem Plasmid pALK487 (WO94/28117) wurde durch Einfügen weiterer Schnittstellen (SpeI und PacI, CCGCGGACTAGTCCTTAATTAACCGCGG) in die SacII-Stelle zwischen dem cbhl-Promotor und cbhl-Terminator das Plasmid pAB487 hergestellt. Die Spel-Pacl-Schnittstellen werden für die direkte Klonierung der Phytasevarianten verwendet. Ein glattendiges 4,78 kb langes EcoRI/Spel-Fragment des Plasmids pALK424 (WO 93/24621), das den amdS-Marker und die 3'-flankierenden cbhl-Sequenzen enthielt, wurde in die Stul-Spaltstelle von pAB487 inseriert, wodurch der Vektor pAB489 erhalten wurde.

Die aus dem pAB489-PhyM3 isolierte Expressionskassette enthält das folgende genetische Material:

Cbhl (Cellobiohydrolase I)-Promotor: Das 2,2 kb EcoRI/SacII-Fragment, das den cbhl-Promotor enthält, stammt von *Trichoderma reesei* QM6a. Der Promotor-Bereich funktioniert auch als homologe DNA (zusammen mit dem cbhl-3'-Fragment; siehe nachstehend), um die Integration der transformierenden DNA in den cbhl-Lokus zu steuern.

Signalsequenz: Das modifizierte Signalpeptid der *A*. *niger*-Phytase wurde verwendet, um die *E. coli*-Phytase aus *Trichoderma reesei* zu sezernieren.

Die *E. coli*-Phytase mit der Mutation V200P Sequenz inkl. der *A. niger*-Phytase-Signalsequenz wurde zwischen den cbhl Promotor und den cbhl Terminator inseriert.

cbhI-Terminator: Das 0,75 kb lange BamHI/StuI-Fragment, das den cbhl-Terminator enthält, wurde im Anschluss an die *E. coli*-Phytase hinzugefügt, um die Termination der Transkription zu gewährleisten.

amdS-Gen: Das Gen inkI. seines Promotors und seines Terminators wurde aus *Aspergillus nidulans* VH1-TRSX6 isoliert und codiert für Acetamidase (Hynes et al., 1983, Mol. Cell. Biol. 3: 1430-1439; Kelly and Hynes, 1985, EMBO J. 4:475-479). Acetamidase erlaubt dem Stamm unter Verwendung von Acetamid als alleiniger Stickstoffquelle zu wachsen und dieses Charakteristikum wurde zur Selektion der Transformanten verwendet.

cbhI 3' Fragment: Das Fragment (1,7 kb, BamHI/EcoRI, beginnend 1,4 kb nach dem Stopcodon des Gens) wurde aus *T. reesei* ALK02466 isoliert. Der Stamm ALKO2466 stammt von dem Stamm ALKO233 (Harkki et al., 1991, Enzyme Microb. Technol. 13: 227-233) ab. Das 3'-Fragment wurde zusammen mit dem Promotorbereich verwendet, um die Phytase-Expressionskassette in den cbhl-Lokus durch homologe Rekombination zielgerecht zu integrieren.

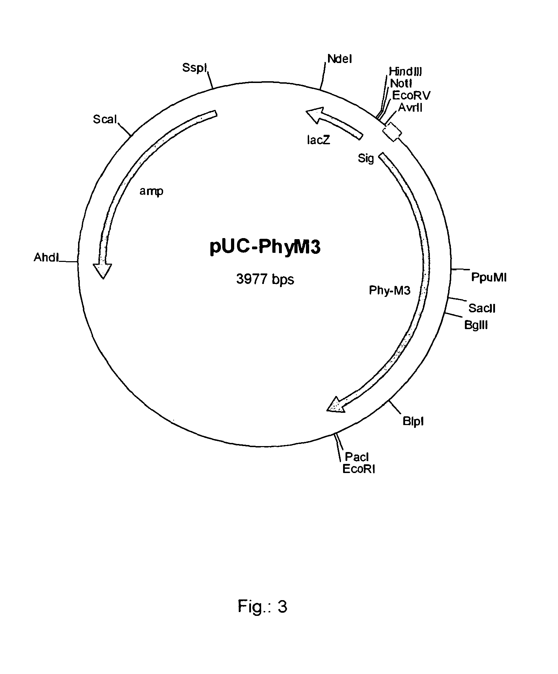

Das Phytasevariante PhyM3 Gen wurde in das Plasmid pAB2004 kloniert. Das erhaltene Plasmid hat die Bezeichnung pUC-PhyM3 (Fig. 3) und als DSM 18717 gemäß der vorstehenden Bedingungen hinterlegt.

Das Plasmid pAB2004 basiert auf dem Plasmid pUC18 und wurde durch Einfügen weitere Schnittstellen in die HindIII-EcoRI sites hergestellt.

### Beispiel 5 - Konstruktion der Plasmide pUC-PhyM9 und pAB489-PhyM9 (Genotyp PhyM9)

Die Phytasevariante PhyM9 enthält die *E*. *coli* Phytase (Dassa et al. 1990, *Ac*cession Nr. M58740) Sequenz als synthetisches Gen unter Verwendung des Codon-Gebrauchs von *T. reesei* (http://www.kazusa.or.jp/codon) mit den Mutationen L1451 und L1981. Die DNA-Sequenz mit dem CAG (Gln)-Codon in Position 1 umfasst ein offenes Leseraster von 1230 bp und codiert ein Enzym mit 410 Aminosäuren.

In dem Plasmid pAB489-PhyM9 steht das Gen kodierend für die *E*. *coli* Phytasevariante PhyM9 mit der *A*. *niger* Phytase Signalsequenz (MGVSAVLLPLYLLSGVTS) Mullaney et al., Appl Microbiol Biotechnol, 1991, 35(5), 611-614, Accession Nr. M94550) direkt unter der Kontrolle des cbh1-Promotors. Das 18 Aminosäuren lange Signalpeptid der Phytase von *A. niger* wurde verwendet, um die Phytasemutante von *E*. *coli* aus *Trichoderma reesei* zu sezernieren.

Die Konstruktion von pAB489-PhyM9 und pUC-PhyM9 erfolgte analog zu Plasmid pAB489-PhyM3 und pUC-PhyM3 in Beispiel 4 und enthält damit mit Ausnahme der *A. niger* Signalsequenz und der spezifischen *E*. *coli* Phytasesequenz die gleichen genetischen Elemente.

Das Plasmid pUC-PhyM9 ist unter der Hinterlegungsnummer DSM 18718 am 18.10.2006 hinterlegt worden.

### Beispiel 6 - Konstruktion der Plasmide pUC-PhyM10 und pAB600-PhyM10, die für multiple Mutanten codieren (Genotyp PhyM10)

Das Plasmid pAB600-PhyM10 enthält die *E*. *coli* Phytase Sequenz mit den Änderungen der Aminosäuren K74D, N139R, D142E und V200P. Aus den Plasmiden pAB490-PhyM2, pAB490-PhyM7 und pAB489-PhyM3 wurde das Gen kodierend für die Phytasevariante PhyM10 mittels der PCR Methode analog dem Prinzip von Tomic et al. (1990, Nucleic Acids Research, 18 (6), 1656) und Vallette et al. (1989, Nucleic Acids Research, 17 (2), 723-733) hergestellt. Das PCR Produkt wurde mit SpeI und PacI geschnitten und in die Spel- und Pacl-Spaltstellen in dem Plasmid pAB600 inseriert.

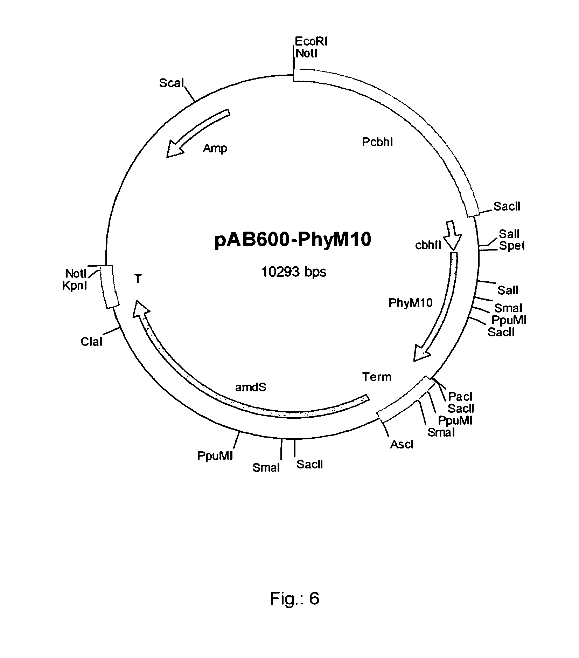

Das entstandene Plasmid pAB600-PhyM10 (Fig. 6) wurde durch Kartierung und Sequenzierung bestätigt.

In dem Plasmid pAB600-PhyM10 wurde die *E*. *coli* Phytase Sequenz mittels einer Kexll-Schnittstelle am 3'Ende des CBHII-Genfragments fusioniert. Die Kex Sequenz enthält folgende Aminosäuren RTLVKR.

Die Konstruktion des Plasmids pAB600 erfolgte durch folgende Schritte:

Das Plasmid pAB1280 basiert auf pUC18 und enthält das cbhll-Genfragment (Nucleotide 1-307 entspricht Aminosäure M1 bis S86, Teeri et al, 1987, Gene 51: 43-52, Accession Nr. 16190) unter der Kontrolle des cbhl-Promotor und cbhl-Terminator aus dem Plasmid pALK487 (WO 94/28117). Darüber hinaus wurden weitere Schnittstellen (SpeI und PacI) downstream des cbhII-Genfragments eingefügt. Diese Schnittstellen werden für die direkte Klonierung der Phytasevariante verwendet.

Aus dem Plasmid p3SR2 (Hynes et al., 1983, Mol. Cell. Biol. 3, 1430-1439; Kelly and Hynes, 1985, EMBO J. 4, 475-479) wurde das Acetamidase (*amd*S)-Gen mittels der PCR-Methode als Ascl-Nrul-Fragment isoliert und in das mit Ascl/Stul geschnittene Plasmid pAB1280 inseriert.

Für die Amplifizierung des *amd*S-Gens wurden folgende Primer aus der Sequenzinformation des *A*. *nidulans*-Acetamidase-Gens abgeleitet:
AmdS-AscI aggcgcgccctagtcatcattggataggcagattactcag (SEQ ID NO:7)
AmdS-NruI ggaattctcgcgaaaggcaacaaccagctcacccctgag (SEQ ID NO:8)

Das erhaltene Plasmid hat die Bezeichnung pAB600 wurde durch Kartierung und Sequenzierung bestätigt.

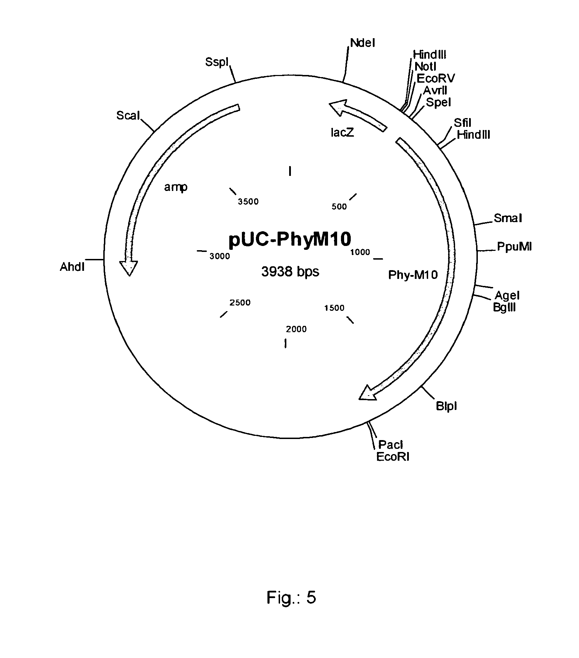

Das Gen der Phytasevariante PhyM10 wurde in das Plasmid pAB2004 kloniert. Das Plasmid pUC-PhyM10 ist in Fig. 5 dargestellt und ist unter der Hinterlegungsnummer DSM 18719 am 18.10.2006 hinterlegt worden.

### Beispiel 7 - Konstruktion der Plasmide pPhy2005 und pAB-Phy2005 (Genotyp Phy2005)

Die Konstruktion des Plasmids pAB-Phy2005 erfolgte durch folgende Schritte:

### 1. Konstruktion von pPhy2005

Das Plasmid pPhy2005 enthält die Fusion aus dem *A. niger* var. *awamori-*saure-Phosphatase (ap)-Gen (Piddington et al., 1993, Gene 133 (1), 55-62; Accession Nr. L02420) und dem *E. coli*-Phytase-Gen Gen unter Verwendung des Codon-Gebrauchs von *T. reesei* (http://www.kazusa.or.jp/codon) (Dassa et al. 1990, Accession Nr. M58740). Dadurch entsteht ein offenes Leseraster, das für eine Phytasevariante mit 457 Aminosäuren codiert, wobei die ersten vier (4) Aminosäuren des *E. coli*-Phytase-Gens durch die ersten einundfünfzig (51) Aminosäuren des sauren Phosphatase-Gens ersetzt sind. Zur Sekretion des Proteins bestehend aus der Fusion saure Phosphatase-*E. coli*-Phytase in *T. reesei* wurde die Signalsequenz der sauren Phosphatase verwendet.

Die Fusion aus der *A. niger* var. *awamori* sauren Phosphatase Sequenz und der unter Verwendung des Codon-Gebrauchs von *T. reesei* erzeugten *E. coli* Phytase Sequenz wurde synthetisiert und in das Plasmid pUC18 kloniert. Das erhaltene Plasmid pPhy2005 wurde durch Sequenzierung bestätigt.

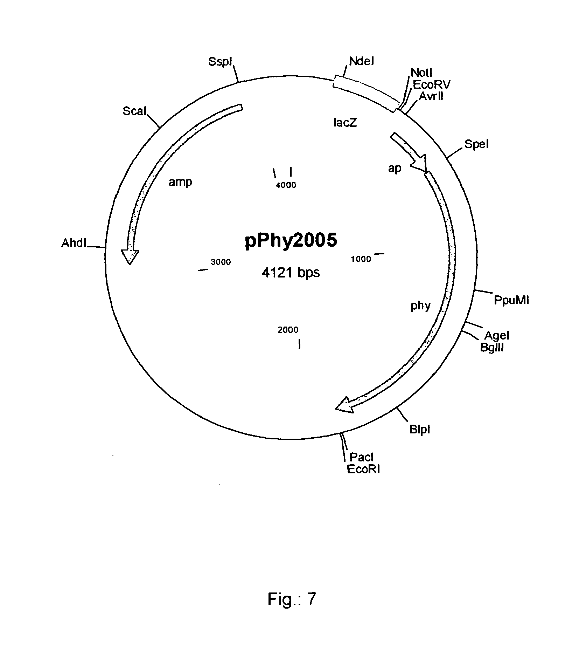

Das Plasmid ist in Fig. 7 dargestellt und ist unter der Hinterlegungsnummer DSM 18720 am 18.10.2006 hinterlegt worden

### 2. Konstruktion von pAB-Phy2005

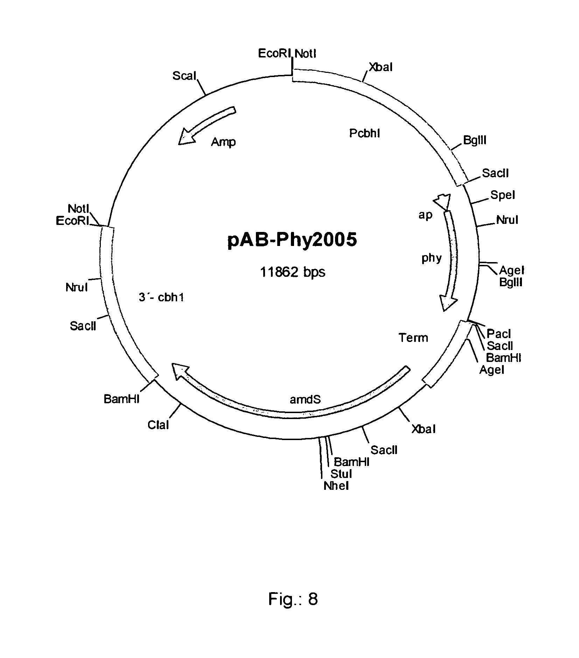

Zur Konstruktion des Plasmids pAB-Phy2005 wurde die Sequenz der Fusion Phosphatase-Phytase aus dem Plasmid pPhy2005 mit Avrll und Pacl geschnitten und in die Spel- und Pacl-Spaltstellen nach dem *T. reesei*-cbhl-Promotor in dem Plasmid pAB489 inseriert. Das entstandene Plasmid hat die Bezeichnung pAB-Phy2005 (Fig. 8) und wurde durch Restriktionsendonucleasen kartiert und die Phytasesequenz durch Sequenzierung bestätigt.

Die aus dem isolierten Expressionskassette pAB-Phy2005 enthält das folgende genetische Material:

cbhl (Cellobiohydrolase I) Promotor: Das 2,2 kb EcoRI/Sacll-Fragment, das den cbhl-Promotor enthält, stammt von *Trichoderma reesei* QM6a. Der PromotorBereich funktioniert auch als homologe DNA (zusammen mit dem cbhl 3'-Fragment; siehe nachstehend), um die Integration der transformierenden DNA in den cbhl Lokus zu steuern.

Saure Phosphatase-Genfragment: Das saure Phosphatase-Genfragment mit seiner Signalsequenz steht direkt unter der Kontrolle des cbhl Promotors. Die 16 Basenpaare (CCGCGGACTGCGCATC atg) stromaufwärts des Startcodons, die zu dem *T. reesei*-Promotor (Shoemaker et al. 1983, Bio/Technology 1, 691-696) gehören, wurden nach dem Einbauen des AvrII-PacI Fusion Phosphatase-Phytase Fragments zu CCGCGGACTAGGCATC atg geändert.

*E. coli*-Phytase-Gen: Das synthetische *E. coli*-Phytase-Gen ist direkt am 3'-Ende der sauren Phosphatase-Sequenz zu einem offenem Leseraster fusioniert.

cbhl-Terminator: Das 0,75 kb lange BamHI/Stul-Fragment, das den cbhl-Terminator enthält, wurde im Anschluss an die *E. coli*-Phytase hinzugefügt, um die Termination der Transkription zu gewährleisten.

amdS-Gen: Das Gen inkl. seines Promotors und seines Terminators wurde aus *Aspergillus nidulans* VH1-TRSX6 isoliert und codiert für Acetamidase (Hynes et al., 1983, Mol. Cell. Biol. 3: 1430-1439; Kelly and Hynes, 1985, EMBO J. 4:475-479). Acetamidase erlaubt dem Stamm unter Verwendung von Acetamid als alleiniger Stickstoffquelle zu wachsen und dieses Charakteristikum wurde zur Selektion der Transformanten verwendet.

cbhl 3' Fragment: Das Fragment (1,7 kb, BamHI/EcoRI, beginnend 1,4 kb nach dem Stopcodon des Gens) wurde aus *T. reesei* ALKO2466 isoliert. Der Stamm ALKO2466 stammt von dem Stamm ALKO233 (Harkki et al., 1991, Enzyme Microb. Technol. 13: 227-233) ab. Das 3'-Fragment wird zusammen mit dem Promotorbereich verwendet, um die Phytase-Expressionskassette in den cbhl Lokus durch homologe Rekombination zielgerecht zu integrieren.

### Beispiel 8 - Konstruktion der Plasmide pPhy2006 und pAB-Phy2006 (Genotyp Phy2006)

Die Konstruktion des Plasmids pAB-Phy2006 erfolgte durch folgende Schritte:

### 1. Konstruktion von pPhy2006

Zur Konstruktion des Plasmids pPhy2006 wurde das 87 bp lange ap-Genfragment, das für die zum C-Terminus des *ap*-Gens der sauren Phosphatase aus *A. niger* var. *awamori* gehörenden 29 Aminosäuren codiert, direkt an die für die letzte Aminosäure (Leucin) der *E. coli* Phytase codierende DNA Sequenz in dem Plasmid pPhy2005 fusioniert. Dadurch entsteht ein offenes Leseraster von 486 Aminosäuren.

Die Fusion Phy2006 aus der *A. niger* var. *awamori* saure Phosphatase Sequenz und der unter Verwendung des Codon-Gebrauchs von *T. reesei* erzeugten *E. coli* Phytase Sequenz wurde synthetisiert und in das Plasmid pUC18 kloniert. Die in dem erhaltene Plasmid pPhy2006 enthaltene neue Sequenz wurde durch Sequenzierung bestätigt.

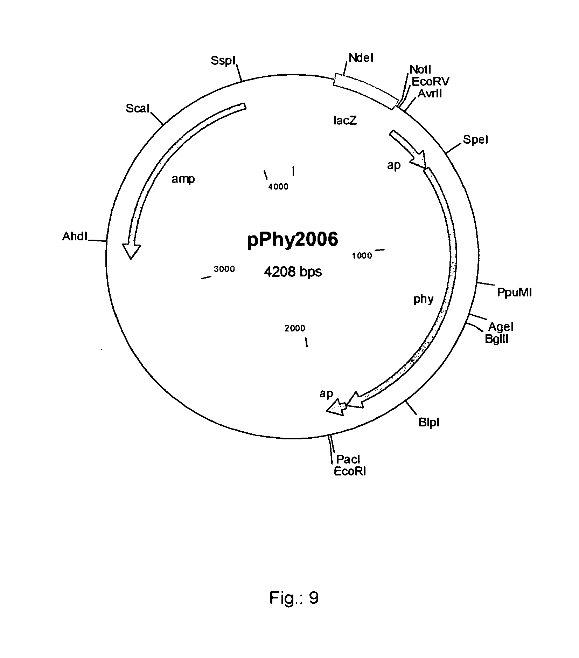

Das Plasmid ist in Fig. 9 dargestellt und ist unter der Hinterlegungsnummer DSM 18721 am 18.10.2006 hinterlegt worden.

### 2. Konstruktion von pAB-Phy2006

Die Konstruktion sowie die Klonierung des Plasmids pAB-Phy2006 ist identisch mit der in Beispiel 7 beschriebenen Herstellung des Plasmids pAB-Phy2005.

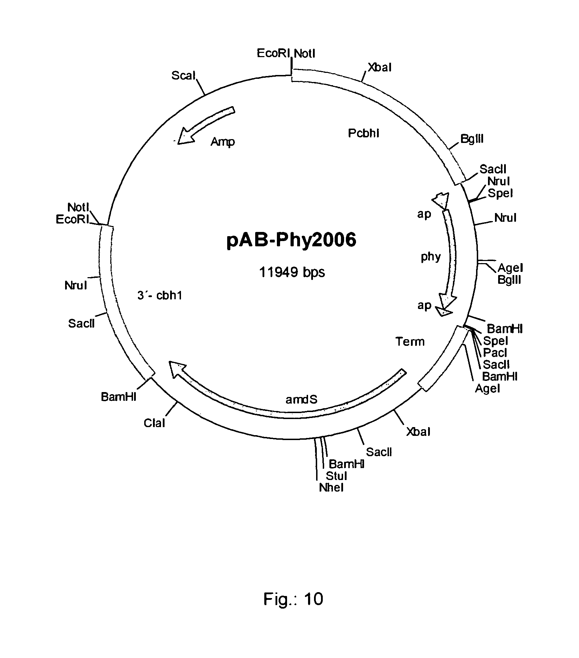

Die Sequenz der Phytasevariante pAB-Phy2006 (Fig. 10) wurde durch Sequenzierung bestätigt.

### Beispiel 9 - Transformation von T. reesei

*T. reesei* RH 3780d wurde mit den aus den Plasmiden pAB489-PhyM3, pAB490-PhyM2, pAB490-PhyM7, pAB489-PhyM9, pAB600-PhyM10, pAB-Phy2005 und pAB-Phy2006 isolierten linearisierten Expressionskassetten separat transformiert. Die zur Transformation und zur Handhabung von *T. reesei* verwendeten Techniken waren die gemäß Penttilä et al. (1987, Gene 61: 155-164). Die Transformanten wurden selektiert und zweimal durch Einzelsporenisolierung gereinigt. Von allen Transformanten wurden diejenigen mit der höchsten Sekretionsleistung ausgewählt und in Beispiel 10 zur Herstellung von Enzymmaterial weiterverwendet. Die verwendeten Transformanten sind in der folgenden Tabelle 2 aufgelistet.

**Tabelle 2: Auflistung der Transformanten für weitere Versuche in den Beispielen 10-13.**

| Genotyp | Transformante |
|---|---|
| Wt (pKDa4) | RH 31071 |
| pKDa2 (V200Y; M1) | RH 31068 |
| PhyM2 | RH 31575 |
| PhyM3 | RH 31545 |
| PhyM7 | RH 31507 |
| PhyM9 | RH 31686 |
| PhyM10 | RH 31898 |
| Phy2005 | RH 31676 |
| Phy2006 | RH 31677 |

### Beispiel 10 - Produktion von Enzym mit den einzelnen Mutanten

### A) Herstellung von Enzvmlösunaen durch Fermentation in Schüttelkolben

Transformanten, die die Expressionskassetten aus den Beispielen 2 bis 8 tragen, bzw. die Transformanten mit dem Plasmid pKDa2 (M1) und pKDa4 (Wildtyp Aminosäuresequenz *E*. *coli*-Phytase nach Dassa) aus DE 10 2004 050 410, wurden in Schüttelkolben mit pH-Steuerung auf einer DASGIP-Anlage auf Cellulase-induzierendem Medium folgender Zusammensetzung gezüchtet: Lactose 10,5% (w/v), DSG 5,25% (w/v), (NH₄)₂SO₄ 0,63% (w/v), Leitungswasser Rest, Einstellung des pH-Werts vor der Sterilisation auf 4,5. Nach Inokulation wurde in den Schüttelkolben eine pH-Rampe innerhalb von 5 h auf pH 3,3 gefahren. Die nach 6-tägiger Züchtung bei gesteuertem pH-Wert von pH 3,3 erhaltenen Kulturfiltrate wurden für die Bestimmung der Phytaseaktivität und für die Analyse der Thermostabilität mittels Differential-Scanning-Kalorimetrie (DSC) (Beispiel 11) verwendet.

### B) Herstellung von Enzymgranulaten durch Fermentation in 30-I-Bioreaktoren und Trocknung in einem ProCell5

Die Transformanten aus Beispiel 10 A) wurden in dem Medium aus Beispiel 10 A) in 30-1-Bioreaktoren bei einem pH-Wert von pH 3,2 ± 0,2, 200 bis 400 upm und 0,5 vvm Belüftungsrate gezüchtet. Am Ende der 6-tägigen Fermentation wurde die Biomasse durch Filtration abgetrennt und der klare Kulturüberstand mittels Ultrafiltration (30 kDa cut-off Membran) um den Faktor 6 eingeengt. Das Konzentrat wurde steril filtriert und dann für die nachfolgende Herstellung von Enzymgranulaten verwendet.

Die Granulate wurden aus den UF-Konzentraten durch Trocknung in einem Sprühgranulator vom Typ ProCell5, Firma Glatt Systemtechnik GmbH, Dresden, Deutschland, hergestellt. Dazu wurde der pH-Wert der UF-Konzentrate auf pH 5,2 eingestellt und vor der Trocknung 50% (w/w) Magermilchpulver mit 30% Laktosegehalt und 9,2% (w/w) Ca-Propionat, beide Zugaben bezogen auf den Gesamtproteingehalt des UF-Konzentrates, zugesetzt. Das Gemisch wurde durch eine 1,2 mm Ø Zweistoffdüse mit einem von 1,2 auf 2,2 bar ansteigenden Luftdruck bei 80°-90°C Zulufttemperatur getrocknet. Die Aktivitätsausbeuten bei der Trocknung lagen im Bereich 89 bis 99%. Die so hergestellten Granulate wurden für den Versuch in Beispiel 12 verwendet.

### Beispiel 11 - Bestimmung der Temperaturstabilität mittels DSC

Die Proben aus Beispiel 10A) wurden mittels einer PD-10-Säule (Pharmacia) in 100 mM Natriumacetat Puffer, pH 5,2 umgepuffert. Dazu wurden 1,5 ml Probe aufgetragen und mit 3,5 ml Puffer nach Anleitung des Herstellers eluiert. Alle DSC-Versuche wurden auf einem VP-DSC-Gerät (MicroCal Inc., Northampton, MA, USA) durchgeführt und die Daten wurden auf einen PC übertragen um dort mit Hilfe der Software Origin v7.0383 (OriginLab Corp. Northampton, MA, USA) ausgewertet zu werden.

Die Proben wurden vor der Messung 20 min lang in einem ThermoVac2 (MicroCal Inc., Northampton, MA; USA) entgast und auf 25°C in einem Wasserbad temperiert.

Die Messungen erfolgten mit folgenden Parametern:
A) Konstante Geräteparameter:
   Zellvolumen: 0,51231 ml
   Referenz Heizwiderstand: 1009,1 ohms
   Zellheizwiderstand: 1002,7 ohms
   Adiabatische Rate: 0,88181
   Delta T read: 3,676
B) VP-DSC scan Parameter waren wie folgt:
   Scanrate (aufwärts und abwärts): 1°C min⁻¹
   Temperatur vor dem Scanzyklus: 25°C
   Äquilibrierzeit vor dem Zyklus: 15 min
   Äquilibrierzeit nach dem Zyklus: 0 min
   Filter: 10 s
   Feedback: keines
   Starttemperatur: 25°C
   Endtemperatur: 105°C
   Zelldruck: 26,1 bis 28,7 psi

Die nachstehende Tabelle 3 zeigt die Schmelztemperatur Tm (in Grad Celsius) und die Verschiebung der Schmelztemperatur ΔT (in Grad Celsius) der mutanten *E. coli*-Phytasen, die nach den oben stehenden Beispielen hergestellt wurden, im Vergleich zur Wildtyp-*E. coli* Phytase (Dassa et al., 1990) hergestellt mit dem gleichen Wirtssystem wie die mutanten *E. coli* Phytasevarianten und einer *E. coli*-Phytase mit einer nicht-erfindungsgemäßen Mutation (M1), ebenfalls hergestellt mit dem gleichen Wirtssystem wie die erfindungsgemäßen mutanten *E. coli* Phytase-Varianten.

**Tab. 3: Schmelztemperatur Tm [°C] und Verschiebung der Schmelztemperatur ΔT [°C] der E. coli Phytasemutanten im Vergleich zum dem Wild-Typ Protein, alle hergestellt in T. reesei nach Beispiel 10A.**

| | **Phytase** | |
|---|---|---|
| **Probe/Genotyp** | **Tm [°C]** | **ΔT [°C]** |
| Wildtyp | 65,64 | --- |
| M1 | 65,01 | -0,63 |
| M2 | 66,75 | +1,11 |
| M3 | 66,04 | +0,40 |
| M7 | 66,21 | +0,57 |
| M9 | 66,27 | +0,63 |
| Phy2005 | 66,06 | +0,42 |
| Phy2006 | 66,06 | +0,42 |

Die Ergebnisse zeigen, dass bei allen Mutanten eine Erhöhung der Schmelztemperatur des Proteins eingetreten ist, was sich in einer positiven Verschiebung der Schmelzpunktstemperatur zeigt. Die Erhöhung der Schmelzpunkttemperatur ist gleichbedeutend mit einer Erhöhung der Temperaturstabilität. Die höchste Verbesserung der Thermostabilität zeigte dabei die Doppelmutation M2 mit ΔT = +1,11°C. Im Gegensatz dazu zeigte die in DE 10 2004 050 410 beschriebene Mutation zur Verbesserung der Sekretionshöhe (V200Y = M1) eine Verschlechterung der Thermostabilität (ΔT = -0,63°C).

Auch die Verlängerungen am N- oder N- und C-terminalen Ende zeigten Verbesserungen der Thermostabilität. Diese liegt in der Größenordnung wie die der Punktmutation bei M3.

### Beispiel 12 - Messung der Thermostabilität im Pelletierverfahren

Enzymgranulate hergestellt gemäß Beispiel 10B) wurden mit Weizenmehl, Typ 550, vorgemischt, um 300 g einer enzymhaltigen Vormischung zu erhalten. Diese Vormischung wurde am Biotechnologischen Institut des Research Institutes for Food and Molecular Biotechnology, Kolding, Dänemark, mit 15 kg Tierfutter vermischt um eine optimale Verdünnung für die Einmischung in 285 kg Tierfutter und eine gut bestimmbare Enzymaktivität in dem pelletierten Material zu gewährleisten. Die eingesetzte Menge an Enzymgranulat führte zu einer Phytaseaktivität von ca. 3 bis 6 Enzymeinheiten pro Gramm Tierfutter in dem Gemisch vor dem Testprozess. Die 300 kg Tierfutter die in der Pilot-Pelletieranlage behandelt wurden, haben die Zusammensetzung von Tabelle 4.

**Tabelle 4: Zusammensetzung des zu pelletierenden Tierfutters**

| Bestandteil | [%] |
|---|---|
| Weizen | Ad 75 |
| Hipro Soja 48 | 20 |
| Sojaöl | 4,75 |
| Vitamin/Mineralien, Beta Avitren 90 | 0,25 |
| Enzymvormischung* | 300 g |

| | |
|---|---|
| * Enthält Phytase mit Genotyp wt, PhyM1, PhyM2, PhyM3, PhyM7, PhyM9, Phy2005, oder Phy2006 | |

### Pelletierbedingungen:

Horizontalmischer mit 700 I Volumen, 48 upm. Die Menge, die gemischt wird, liegt im Bereich 80 bis 300 kg je h.

An den Mischer angeschlossen ist eine Horizontalförderschnecke zum Entleeren des Mischers, deren Fördergeschwindigkeit an den nachfolgenden Schritt angepasst ist.

Anschließend an die Mischung im Horizontalmischer erfolgt eine thermische Behandlung in einem Kahl Kaskadenmischer (Konditionierer) mit 130 cm Länge, 30 cm Durchmesser, 155 upm und 37 Kammern. Durchsatz 300 kg je h. Futtermittel und Enzyme haben ca. 30 s Kontakt mit Nassdampf und werden dabei auf eine Temperatur von 70° bis 95°C erwärmt. Der Dampf, der durch einen Dan-Stoker-Hochdruckkessel bereitgestellt wird, strömt mit 2 bar Überdruck durch ein Druckregulationsventil in den Kaskadenmischer. Das Ventil regelt die Menge Dampf, die in den Kaskadenmischer einströmt und dort zur Erwärmung des Futtermittels (einschl. des Enzyms) führt.

Das konditionierte Material wird durch eine Simon Heesen Presse pelletiert und anschließend in einer mit 1500 m³ h⁻¹ durchströmten perforierten Box durch Luft gekühlt. Die Pelletierpresse läuft mit 500 upm bei einer Leistungsaufnahme von 7,5 kW und erzeugt dabei Pellets von 3 x 20 mm.

Die Behandlung im Kaskadenmischer bewirkte eine thermische Belastung des im pelletierten Material enthaltenen Enzyms. Anschließend an die Herstellung der Pellets wurde die Wiederfindung der Phytaseaktivität im pelletierten Tierfutter bestimmt. In der nachstehenden Tabelle 5 ist die Wiederfindung der Phytaseaktivität im pelletierten Tierfutter in Abhängigkeit von der Temperatur bei der Behandlung angegeben.

**Tab. 5: Wiederfindung der Phytaseaktivität im Tierfutter nach der Konditionierung bei 70° bis 95°C und anschließender Pelletierung. Alle Enzyme wurden rekombinant mit T. reesei exprimiert.**

| | Phytase-Aktivitätswiederfindung [%] | | | | | | | |
|---|---|---|---|---|---|---|---|---|
| T[°C] | wt | M1 | M2 | M3 | M7 | M9 | Phy-2005 | Phy2006 |
| 70 | 93,0 | 64,0 | 103,1 | 101,6 | 109,5 | 108,4 | 97,6 | 93,6 |
| 75 | 90,3 | 47,8 | 104,9 | 90,7 | 88,2 | 105,0 | 85,4 | 90,1 |
| 80 | 46,6 | 27,8 | 73,0 | 43,7 | 55,5 | 55,3 | 32,9 | 58,6 |
| 85 | 7,8 | 5,9 | 37,9 | 9,2 | 16,8 | 11,0 | 7,6 | 27,5 |
| 90 | 1,3 | 0,0 | 9,7 | 2,8 | 5,4 | 3,0 | 0,6 | 11,8 |
| 95 | 0,5 | 0,0 | 4,9 | 0,0 | 1,4 | 0,0 | 1,0 | 0,5 |

Die Ergebnisse zeigen, dass die Veränderung der Thermostabilität, die bei den Messungen in Flüssigkeit mittels DSC gefunden wurde, auch in den "trockenen" Pelletierversuchen wiedergefunden wurde. Die Verbesserung der Thermostabilität gegenüber dem Wildtyp-Enzym ist dabei nicht bei allen Mutanten über den gesamten gemessenen Temperaturbereich konstant. M2 zeigte im DSC-Versuch die höchste Temperaturstabilität. Auch im Pelletierversuch wurde die beste Stabilisierung durch Mutation in einer isolierten Region durch die Doppelmutation M2 erzielt, die eine ionische Brücke in Helix D der *E. coli* Phytase einfügt. Die nicht erfinderische Mutante M1 zeigt wie im DSC Versuch eine Verschlechterung der Thermostabilität im Vergleich zum Wildtyp-Enzym exprimiert in *Trichoderma reesei*. Die ebenfalls für sich alleine nicht erfindungsgemäße Mutation M3 zeigt ähnliche Thermostabilitätseigenschaften wie das Wildtyp-Enyzm. Die Variante Phy2005, die nur die N-terminale Verlängerung durch den korrespondierenden Teil der sauren Phosphatase aus *A. niger* var. *awamori* trägt, ist nicht so stabil bei höheren Temperaturen wie Phy2006, die sowohl die N- wie auch die C-terminale Verlängerung enthält. Dies deutet darauf hin, dass diese Verlängerungen sehr wohl zu einer Zusammenlagerung von *E. coli* Phytase Molekülen analog zu der Dimerisierung der sauren Phosphatase unter normalen Bedingungen beitragen können und damit die Thermostabilität verbessern.

### Beispiel 13 - Messung der proteolytischen Stabilität in einem in-vitro-Verfahren

Zur Bestimmung der proteolytischen in-vitro-Stabilität wurden Enzymproben aus Beispiel 10A) verwendet, d.h. Proben der Transformanten M1 (der nicht erfindungsgemäßen Mutation), M2, M3, M7, Phy2005 und Phy2006 hergestellt mit den Transformaten aus Tabelle 2.

20 ml einer Pepsin-Lösung (Merck 7190), die 20 Proteaseeinheiten pro ml in Glycin-HCl-Puffer (pH 2,5) enthält, wobei die Aktivität nach Angabe des Herstellers auf Hämoglobin, pH 1,6, 25°C bezogen ist, wurde bei 40°C temperiert.

10 ml einer Phytase-Lösung (1:100 verdünnt) wurde zugegeben und die Lösung mit Puffer auf 50 ml aufgefüllt und anschließend bei 40°C, pH 2,5 für 30 min inkubiert. Die Reaktion wurde durch Eintauchen in ein Eis/Wasser-Bad gestoppt und der pH-Wert wurde sofort auf pH 5 mit Natronlauge angehoben und die Lösung dabei auf 100 ml verdünnt. Die Phytaseaktivität wurde dann nach Beispiel 1 gemessen.

10 ml der mit Pepsin behandelten Lösung wurde auf pH 7 eingestellt. 30 ml einer Pankreatin-Lösung (Merck 7130), die 30 Proteaseeinheiten (Casein, pH 8,0, 35°C), in 0,05 M Tris-HCl-Puffer (pH 7,0) enthält, wurde zugegeben, die Lösung mit Tris-HCl-Puffer, pH 7, auf 50 ml verdünnt und die Lösung in einem Wasserbad bei 40°C für 30 min inkubiert. Die Reaktion wurde durch Eintauchen in ein Eis/Wasser-Bad gestoppt und der pH-Wert wurde sofort auf pH 5 mit HCl erniedrigt und die Lösung dabei auf 100 ml verdünnt. Die Phytaseaktivität wurde dann nach Beispiel 1 gemessen. Die Ergebnisse sind in Tabelle 6 dargestellt.

**Tab. 6: Aktivitätswiederfindung nach Behandlung der Enzymmutanten mit Pepsin und anschließend Pankreatin**

| Genotyp | Restaktivität nach Pepsin Behandlung [%] | Restaktivität nach Pepsin- und Pankreatinbehandlung [%] |
|---|---|---|
| PhyM1 | 84 | 71 |
| PhyM2 | 111 | 85 |
| PhyM3 | 112 | 80 |
| PhyM7 | 101 | 75 |
| Phy2005 | 110 | 99 |
| Phy2006 | 109 | 78 |

Die Ergebnisse zeigen für alle Mutanten eine sehr hohe proteolytische Stabilität in Gegenwart von Pepsin und Pankreatin, verglichen mit der Vergleichsmutante PhyM1, unter in-vitro-Bedingungen, wie sie im Magen von Monogastriern, z. B. Schweinen, vorkommen. Die hier gezeigte hohe proteolytische Stabilität verdeutlicht, dass diese Enzymmutanten für einen Einsatz im Tierfutterbereich besonders geeignet sind.

## Patentansprüche

1. Rekombinantes DNA-Molekül, das nach Expression in einer prokaryotischen oder eukaryotischen Wirtszelle ein Polypeptid mit Phytaseaktivität codiert, wobei das rekombinante DNA-Molekül eine DNA-Sequenz, ausgewählt aus
a) DNA-Sequenzen, die ein Polypeptid mit Phytaseaktivität codieren, das durch Variation der reifen Wildtyp-*E*. coli-Phytasesequenz erhalten worden ist, wobei die Variation die Mutation Lysin → Asparaginsäure an Position 74 (K74D) umfasst, und
b) DNA-Sequenzen, die aufgrund der Degeneriertheit des genetischen Codes mit den Sequenzen gemäß a) verwandt sind,
umfasst, wobei das rekombinante DNA-Molekül bei Expression in einer geeigneten Wirtszelle mit einer erhöhten Temperatur- und Proteasestabilität der Enzymaktivität des so codierten Proteins einhergeht

2. Rekombinantes DNA-Molekül nach Anspruch 1, **dadurch gekennzeichnet, dass** die DNA-Sequenz zusätzlich die Variation V200P des codierten Polypeptids umfasst.

3. Rekombinantes DNA-Molekül nach Anspruch 1, **dadurch gekennzeichnet, dass** die DNA-Sequenz zusätzlich die Variation L145I und L198I des codierten Polypeptids umfasst.

4. Rekombinantes DNA-Molekül nach Anspruch 1, **dadurch gekennzeichnet, dass** es als N-terminale Anfügung des codierten Polypeptids die Sequenz FSYGAAIPQS TQEKQFSQEF RDGYSILKHY GGNGPYSERV SYGIARDPPTS umfasst und/oder als C-terminale Anfügung des codierten Polypeptids die Sequenz LSFWWNYNTT TELNYRSSPI ACQEGDAMD umfasst.

5. Rekombinantes DNA-Molekül nach Anspruch 1, **dadurch gekennzeichnet, dass** es eine Sequenz ausgewählt aus den Sequenzen SEQ ID NO:13 oder SEQ ID NO:17 umfasst.

6. Polypeptid, das Phytaseaktivität besitzt und von einem rekombinanten DNA-Molekül nach einem der Ansprüche 1 bis 5 codiert wird oder durch Expression einer damit transformierten Wirtszelle erhalten wird.

7. Polypeptid nach Anspruch 6, **dadurch gekennzeichnet, dass** es eine Sequenz ausgewählt aus SEQ ID NO:14 oder SEQ ID NO:18 umfasst.

8. DNA-Konstrukt mit der Fähigkeit, nach dem Einführen in eine geeignete Wirtszelle die Expression eines mutierten Phytasegens in einem Wirt zu steuern, **dadurch gekennzeichnet, dass** es eine DNA-Sequenz nach einem der Ansprüche 1 bis 5 und einen Terminator enthält.

9. DNA-Konstrukt nach Anspruch 8, **dadurch gekennzeichnet, dass** es 5'- und 3'-flankierende Sequenzen, einen Promotor und/oder Signal- und Markersequenzen enthält.

10. DNA-Konstrukt nach Anspruch 9, **dadurch gekennzeichnet, dass** es sich bei dem Promotor um den Cellobiohydrolase-1-, den Cellobiohydrolase-II-, den Amylase-, den Glucoamylase-, den Xylanase- oder den Enolase-Promotor handelt.

11. DNA-Konstrukt nach Anspruch 9 oder 10, **dadurch gekennzeichnet, dass** es sich bei der Signalsequenz und/oder den 5'-und 3'-flankierenden Sequenzen um Phytase-Signalsequenzen aus *Aspergillus niger* handelt.

12. DNA-Konstrukt nach Anspruch 11, **dadurch gekennzeichnet, dass** es sich bei den Phytase-Signalsequenzen aus *Aspergillus niger* um modifizierte Phytase-Signalsequenzen aus *Aspergillus niger* handelt.

13. Vektor mit der Fähigkeit, eine Wirtszelle zu transformieren, **dadurch gekennzeichnet , dass** der Vektor ein Konstrukt nach Anspruch 8 bis 12 enthält.

14. Vektor nach Anspruch 13, **dadurch gekennzeichnet, dass** er ausgewählt ist aus dem Plasmid pET-PhyM7, hinterlegt unter der Hinterlegungsnummer DSM 18716, oder dem Plasmid pUC-PhyM10, hinterlegt unter der Hinterlegungsnummer DSM 18719.

15. Transformierte Wirtszelle, ausgewählt aus Pilz, Hefe, Bakterien und Säugerzellen, enthaltend ein rekombinantes DNA-Molekül nach einem der Ansprüche 1 bis 5 und ausgestattet mit der Fähigkeit, ein Polypeptid mit Phytaseaktivität zu exprimieren.

16. Transformierte Wirtszelle nach Anspruch 15, **dadurch gekennzeichnet, dass** sie aus der Gattung Aspergillus, Rhizopus, Trichoderma, Neurospora, Mucor oder Penicillium ausgewählt ist.

17. Verfahren zur Produktion von Phytase, **dadurch gekennzeichnet, dass** eine transformierte Wirtszelle nach Anspruch 15 oder 16 unter Bedingungen gezüchtet wird, die der Bildung von Phytase förderlich sind und die so produzierte Phytase isoliert wird.

18. Zusammensetzung, umfassend ein Polypeptid nach Anspruch 6 oder 7, gegebenenfalls zusammen mit weiteren Hilfs- und/oder Wirkstoffen

19. Zusammensetzung nach Anspruch 18, **dadurch gekennzeichnet, dass** die Zusammensetzung eine Nahrungsmittel- oder Futtermittelzusammensetzung oder ein Backmittel ist.

20. Verwendung eines Polypeptids nach Anspruch 18 oder 19 zur Herstellung eines Präparats zur Verbesserung der Phosphatverwertung aus der Nahrung bei Tieren und Menschen oder zur Verbesserung der rheologischen Eigenschaften von Teigen zur Herstellung von Backwaren.

21. Verwendung einer Variation der reifen Wildtyp-*E. coli-*Phytasesequenz, umfassend die Mutation Lysin → Asparaginsäure an Position 74 (K74D), zur Herstellung eines rekombinanten DNA-Moleküls, das nach Expression in einer prokaryotischen oder eukaryotischen Wirtszelle ein Polypeptid mit Phytaseaktivität codiert, das eine erhöhte Temperatur- und Proteasestabilität besitzt.

22. Verwendung nach Anspruch 21, **dadurch gekennzeichnet, dass** die Verwendung zusammen mit der Variation L145I und L198I erfolgt.

23. Verwendung nach Anspruch 22, **dadurch gekennzeichnet, dass** die Verwendung zusammen mit der Variation V200P erfolgt.

24. Verwendung nach Anspruch 22, **dadurch gekennzeichnet, dass** die Verwendung zusammen mit der Variation der N-terminalen Anfügung der Sequenz FSYGAAIPQS TQEKQFSQEF RDGYSILKHY GGNGPYSERV SYGIARDPPTS und/oder der Variation der C-terminalen Anfügung der Sequenz LSFWWNYNTT TELNYRSSPI ACQEGDAMD erfolgt.

## Claims

1. Recombinant DNA molecule encoding a polypeptide having phytase activity after the expression in a prokaryotic or eukaryotic host cell, whereby the recombinant DNA molecule comprises a DNA sequence selected from
a) DNA sequences encoding a polypeptide that has phytase activity and is obtained by variation of the mature wild-type E.coli phytase sequence, the variation comprising the mutation lysine → aspartic acid in position 74 (K74D), and
b) DNA sequences which are related to the sequences listed under a) because of the degeneration of the genetic code,
whereby the recombinant DNA molecule, when expressed in a suitable host cell, has an increased temperature and protease stability of the enzyme activity of the protein coded in said manner.

2. Recombinant DNA molecule according to claim 1, **characterized in that** the DNA sequence additionally comprises at least the variation V200P of the coded polypeptide.

3. Recombinant DNA molecule according to claim 1, **characterized in that** the DNA sequence additionally comprises the variation L145I and L198I of the coded polypeptide.

4. Recombinant DNA molecule according to claim 1, **characterized in that** a N-terminal addition of the coded polypeptide comprises the sequence FSYGAAIPQS TQEKQFSQEF RDGYSILKHY GGNGPYSERV SYGIARDPPTS and/or a C-terminal addition of the coded polypeptide comprises the sequence LSFWWNYNTT TELNYRSSPI ACQEGDAMD.

5. Recombinant DNA molecule according to claim 1, **characterized in that** it comprises a sequence chosen from the sequences SEQ ID NO: 13 or SEQ ID NO: 17.

6. Polypeptide which has a phytase activity and which is coded by a recombinant DNA molecule according to any of claims 1 to 5 or which is obtained by the expression of a host cell transformed by the same.

7. Polypeptide according to claim 6, **characterized in that** it comprises a sequence, chosen from SEQ ID NO: 14 or SEQ ID NO: 18.

8. DNA construct with the capacity to control the expression of a mutated phytase gene in a host after the introduction into a suitable host cell, **characterized in that** it contains a DNA sequence according to any of claims 1 to 5 and a terminator.

9. DNA construct according to claim 8, **characterized in that** it contains 5' and 3' flanking sequences, a promoter and/or signal and marker sequences.

10. DNA construct according to claim 9, **characterized in that** the promoter is either the cellobiohydrolase I, cellobiohydrolase II, amylase, glucoamylase, xalanase or enolase promoter.

11. DNA construct according to claim 9 or 10, **characterized in that** the signal sequence and/or the 5' and 3' flanking sequences are phytase signal sequences of *Aspergillus niger.*

12. DNA construct according to claim 11, **characterized in that** the phytase signal sequences of *Aspergillus niger* are modified phytase signal sequences of *Aspergillus ni*ger.

13. Vector with the capacity to transform a host cell, **characterized in that** the vector contains a construct according to claims 8 to 12.

14. Vector according to claim 13, **characterized in that** it has been selected from the plasmid pET-PhyM7, deposited under the accession number DSM 18716 or the plasmid pUC-PhyM10, deposited under the accession number DSM 18719.

15. Transformed host cell, selected from fungi, yeast, bacteria and mammal cells, containing a recombinant DNA molecule according to any of claims 1 to 5 and having the capacity to express a polypeptide with phytase activity.

16. Transformed host cell according to claim 15, **characterized in that** it is selected from the genus Aspergillus, Rhizopus, Trichoderma, Neurospora or Penicillium.

17. Process for the production of phytase, **characterized in that** a transformed host cell according to claim 15 or 16 is cultured under conditions, which are advantageous for the production of phytase, and the hereby produced phytase is isolated.

18. Composition comprising a polypeptide according to claim 6 or 7 optionally together with further auxiliary and/or active ingredients.

19. Composition according to claim 18, **characterized in that** the composition is a composition of food or animal feed, or a baking ingredient.

20. Use of a polypeptide according to claim 18 or 19 for the production of a preparation for the improvement of the phosphat utilisation from food and animal feed in animals and humans, or for the improvement of the rheological characteristics of doughs for the production of bakery products.

21. Use of a variation of the mature wild-type E.coli phytase sequence, the variation comprising the mutation lysine → aspartic acid in position 74 (K74D) for the production of a recombinant DNA molecule which, after the expression in a prokaryotic or eukaryotic host cell, codes for a polypeptide with phytase activity which has an increased temperature and protease stability.

22. Use according to claim 21, **characterized in that** the use is made together with the variation L145I and L198I.

23. Use according to claim 22, **characterized in that** the use is made together with the variation V200P.

24. Use according to claim 22, **characterized in that** the use is made together with the variation of the N-terminal addition of the sequence FSYGAAIPQS TQEKQFSQEF RDGYSILKHY GGNGPYSERV SYGIARDPPTS and/or the variation of the C-terminal addition of the sequence LSFWWNYNTT TELNYRSSPI ACQEGDAMD.

## Revendications

1. Molécule d'ADN recombinante qui, après une expression dans une cellule hôte procaryote ou eucaryote, code un polypeptide ayant une activité de phytase, la molécule d'ADN recombinante comprenant une séquence d'ADN choisie parmi
a) des séquences d'ADN qui codent un polypeptide ayant une activité de phytase qui a été obtenue par variation de la séquence de phytase mature d'*E*. *coli* de type sauvage, la variation comprenant la mutation lysine → acide asparaginique en position 74 (K74D), et
b) des séquences d'ADN qui sont apparentées en raison de la dégénérescence du code génétique aux séquences selon le point a),
la molécule d'ADN recombinante s'accompagnant, lors de l'expression dans une cellule hôte appropriée d'une stabilité de température et de protéase élevée, de l'activité enzymatique de la protéine ainsi codée.

2. Molécule d'ADN recombinante selon la revendication 1, **caractérisée en ce que** la séquence d'ADN comprend en outre la variation V200P du polypeptide codé.

3. Molécule d'ADN recombinante selon la revendication 1, **caractérisée en ce que** la séquence d'ADN comprend en outre la variation L145I et L198I du polypeptide codé.

4. Molécule d'ADN recombinante selon la revendication 1, **caractérisée en ce qu'**elle comprend, comme addition N-terminale du polypeptide codé, la séquence FSYGAAIPQS TQEKQFSQEF RDGYSILKHY GGNGPYSERV SYGIARDPPTS et/ou comme addition C-terminale du polypeptide codé, la séquence LSFWWNYNTT TELNYRSSPI ACQEGDAMD.

5. Molécule d'ADN recombinante selon la revendication 1, **caractérisée en ce qu'**elle comprend une séquence choisie parmi les séquences SEQ ID N° : 13 ou SEQ ID N° : 17.

6. Polypeptide qui possède l'activité de phytase et est codé par une molécule d'ADN recombinante selon l'une des revendications 1 à 5 ou est obtenu par l'expression d'une cellule hôte ainsi transformée.

7. Polypeptide selon la revendication 6, **caractérisé en ce qu'**il comprend une séquence choisie parmi SEQ ID N° : 14 ou SEQ ID N° : 18.

8. Construction d'ADN ayant la capacité de commander, après l'introduction dans une cellule hôte appropriée, l'expression d'un gène de phytase muté dans un hôte, **caractérisée en ce qu'**elle contient une séquence d'ADN selon l'une des revendications 1 à 5, et un terminateur.

9. Construction d'ADN selon la revendication 8, **caractérisée en ce qu'**elle contient des séquences flanquantes en 5' et 3', un promoteur et/ou des séquences de signal et des séquences marqueurs.

10. Construction d'ADN selon la revendication 9, **caractérisée en ce que** le promoteur est le promoteur de la cellobiohydrolase I, de la cellobiohydrolase II, de l'amylase, de la glucoamylase, de la xylanase ou de l'énolase.

11. Construction d'ADN selon la revendication 9 ou 10, **caractérisée en ce que** la séquence de signal et/ou les séquences flanquantes en 5' et 3' sont des séquences de signal de phytase *d'Aspergillus niger.*

12. Construction d'ADN selon la revendication 11, **caractérisée en ce que** les séquences de signal de la phytase *d'Aspergillus niger* sont des séquences de signal de la phytase modifiées *d'Aspergillus niger.*

13. Vecteur ayant la capacité de transformer une cellule hôte, **caractérisé en ce que** le vecteur contient une construction selon l'une des revendications 8 à 12.

14. Vecteur selon la revendication 13, **caractérisé en ce qu'**il est choisi parmi le plasmide pET-PhyM7 enregistré sous le numéro d'enregistrement DSM 18716, ou le plasmide pUC-PhyM10 enregistré sous le numéro d'enregistrement DSM 18719.

15. Cellule hôte transformée, choisie parmi un champignon, des levures, des bactéries et des cellules de mammifère, contenant une molécule d'ADN recombinante selon l'une des revendications 1 à 5 et dotée de la capacité d'exprimer un polypeptide ayant une activité de phytase.

16. Cellule hôte transformée selon la revendication 15, **caractérisée en ce qu'**elle est choisie parmi les genres Aspergillus, Rhizopus, Trichoderma, Neurospora, Mucor ou Phenicillium.

17. Procédé de production de phytase, **caractérisé en ce qu'**une cellule hôte transformée selon la revendication 15 ou 16 est cultivée dans des conditions qui sont utiles à la formation de phytase et la phytase ainsi obtenue est isolée.

18. Composition comprenant un polypeptide selon la revendication 6 ou 7, éventuellement conjointement à d'autres adjuvants et/ou substances actives.

19. Composition selon la revendication 18, **caractérisée en ce que** la composition est un produit alimentaire ou une composition d'aliments pour animaux ou un adjuvant de panification.

20. Utilisation d'un polypeptide selon la revendication 18 ou 19, pour la production d'une préparation pour améliorer l'assimilation du phosphate de la nourriture chez des animaux et chez l'être humain ou pour améliorer les propriétés rhéologiques des pâtes pour la production des produits de boulangerie.

21. Utilisation d'une variation de la séquence de phytase mature d'*E*. *coli* de type sauvage comprenant la mutation lysine → acide asparaginique en position 74 (K74D), pour la production d'une molécule d'ADN recombinante qui, après l'expression dans une cellule hôte procaryote ou eucaryote code un polypeptide ayant une activité de phytase qui possède une stabilité de température et de protéase élevée.

22. Utilisation selon la revendication 21, **caractérisée en ce que** l'utilisation s'effectue conjointement avec la variation L145I et L198I.

23. Utilisation selon la revendication 22, **caractérisée en ce que** l'utilisation s'effectue conjointement à la variation V200P.

24. Utilisation selon la revendication 22, **caractérisée en ce que** l'utilisation s'effectue conjointement à la variation de l'addition N-terminale de la séquence FSYGAAIPQS TQEKQFSQEF RDGYSILKHY GGNGPYSERV SYGIARDPPTS et/ou à la variation de l'addition C-terminale de la séquence LSFWWNYNTT TELNYRSSPI ACQEGDAMD.
